# EUROPEAN PATENT SPECIFICATION

(11) **EP 3 813 841 B1**
(45) Date of publication and mention of the grant of the patent: **13.08.2025**
(21) Application number: 19824953.4
(22) Date of filing: 28.06.2019
(51) Int. Cl.: A61K 31/663, C07F 9/12, C07F 9/40, A61P 25/00, A61P 31/12

(54) **NEW MOLECULAR TWEEZERS AGAINST NEUROLOGICAL DISORDERS AND VIRAL INFECTIONS**
NEUE MOLEKULARE PINZETTEN GEGEN NEUROLOGISCHE STÖRUNGEN UND VIRALE INFEKTIONEN
NOUVELLES PINCES MOLÉCULAIRES CONTRE DES TROUBLES NEUROLOGIQUES ET DES INFECTIONS VIRALES

(30) Priority: 29.06.2018 US 201862692479 P
(43) Date of publication of application: 05.05.2021
(73) Proprietor: THE REGENTS OF THE UNIVERSITY OF CALIFORNIA, Oakland, CA 94607-5200 (US); Universität Duisburg-Essen, 45141 Essen (DE); Universität Ulm, 89081 Ulm (DE)
(72) Inventor: BITAN, Gal, Oakland, California 94607-5200 (US); BOUQUIO, Danielle, Oakland, California 94607-5200 (US); MALIK, Ravinder, Oakland, California 94607-5200 (US); SCHRADER, Thomas, 40822 Mettmann (DE); MÜNCH, Jan, 89231 Neu-Ulm (DE); HEID, Christian, 45739 Oer-Erkenschwick (DE); SOWISLOK, Andrea, 46240 Bottrop (DE); RÖCKER, Annika, 89075 Ulm (DE)
(74) Representative: Michalski Hüttermann & Partner Patentanwälte mbB
(86) International application number: PCT/US2019/039943
(87) International publication number: WO 2020/006489

(56) References cited:
- US-A1- 2012 108 548
- US-A1- 2012 108 548
- DUTT SOM: "Molecular Tweezers with Additional Binding Sites against Protein Aggregation", DISSERTATION, 1 January 2012 (2012-01-01), pages 1 - 227, XP055881518, Retrieved from the Internet <URL:https://d-nb.info/102928850X/34> [retrieved on 20220120]
- HEID CHRISTIAN NAT: "Molekulare Pinzetten zur Proteinoberflächenerkennung", DISSERTATION, 1 February 2018 (2018-02-01), pages 1 - 371, XP055881517, Retrieved from the Internet <URL:https://duepublico2.uni-due.de/servlets/MCRFileNodeServlet/duepublico_derivate_00070750/Diss_Heid.pdf> [retrieved on 20220120]
- DUTT SOM ET AL: "Linker Effects on Amino Acid and Peptide Recognition by Molecular Tweezers : Recognition by Molecular Tweezers", EUROPEAN JOURNAL OF ORGANIC CHEMISTRY, vol. 2013, no. 34, 1 December 2013 (2013-12-01), DE, pages 7705 - 7714, XP055881514, ISSN: 1434-193X, DOI: 10.1002/ejoc.201301211
- RÖCKER ANNIKA E ET AL: "The molecular tweezer CLR01 inhibits Ebola and Zika virus infection", ANTIVIRAL RESEARCH, ELSEVIER BV, NL, vol. 152, 8 February 2018 (2018-02-08), pages 26 - 35, XP085359679, ISSN: 0166-3542, DOI: 10.1016/J.ANTIVIRAL.2018.02.003
- SPRICK A: "Annex 1", DEZERNAT MEDIENBEARBEITUNG DISSERTATIONSSTELLE, 16 August 2022 (2022-08-16), pages 1 - 1, XP093020907
- FOKKENS, M ET AL.: "A Molecular Tweezer for Lysine and Arginine", JOURNAL OF THE AMERICAN CHEMICAL SOCIETY, vol. 127, no. 41, 19 October 2005 (2005-10-19), pages 14415 - 14421, XP002585647, DOI: 10.1021/JA052806A
- SCHRADER, T ET AL.: "Molecular tweezers for lysine and arginine - powerful inhibitors of pathologic protein aggregation", CHEMICAL COMMUNICATIONS, vol. 52, no. 76, 1 October 2016 (2016-10-01), pages 11318 - 11334, XP055667013

## Description

### CROSS-REFERENCE TO RELATED APPLICATIONS

This application claims priority to and benefit of USSN 62/692,479, filed on June 29, 2018.

### STATEMENT OF GOVERNMENTAL SUPPORT

This invention was made with government support under Grant Number AG050721, awarded by the National Institutes of Health. The government has certain rights in the invention.

### BACKGROUND

Molecular tweezers are belt-shaped, rigid host molecules, whose alternating arrangement of benzene and norbornadiene rings forms a well-defined cavity. A central hydroquinone unit is used to attach functional groups, such as carboxylates, sulfates and phosphates, conferring water solubility. Inside their characteristic cavity, which has a negative electrostatic potential surface, these host molecules include a very small variety of cationic guests, which are all bound by a unique mechanism: the cationic part is threaded through the cavity and locks into an ion pair with the appropriately placed host anion. This is supplemented by hydrophobic interactions between the portion of the guest threaded through the cavity, typically an alkyl chain, and the hydrocarbon skeleton of the host tweezers. Thus, the tweezers accommodate only the sidechains of lysine and arginine (Fokkens et al. (2005) J. Am. Chem. Soc. 127 (41): 14415-14421). This high specificity for positively charged amino acid residues together with their moderate affinity and fast on- and off-rates renders the tweezers a privileged compound class for powerful intervention with pathologic protein aggregation (Schrader et al. (2016) Chem. Commun. (Camb), 52(76): 11318-11334).

Additionally, molecular tweezers have been found to disrupt lipid-raft-rich membranes, such as those found in the envelope of many viruses, but not the membranes of normal mammalian cells (Lump et al. (2015) eLife, 4: e05397). This independent activity makes the molecular tweezers promising anti-viral compounds that could be used as microbicides or therapeutics against many infectious diseases caused by enveloped viruses.

Molecular tweezers are known inter alia from the US 2012/0108548.

### SUMMARY

In various embodiments new molecular-tweezer compounds, new synthetic routes and new methods for the preparation of molecular-tweezer compounds are provided. Additionally, new evidence for improved pharmacokinetic characteristics of certain molecular-tweezer derivatives, including improved oral bioavailability and blood-brain barrier penetration, and new evidence of improved anti-viral activity of new moleculartweezers derivatives against enveloped viruses, including, but not limited to Zika virus and HIV is provided.

The references to methods of treatment in the subsequent paragraphs of this description are to be interpreted as references to the compounds, pharmaceutical compositions and medicaments of the present invention for use in a method for treatment of the human body by therapy (or for diagnosis where appropriate).

The invention is set out in the appended set of claims. Various embodiments contemplated herein may include, but need not be limited to, one or more of the following:
Embodiment 1: A compound of formula I or a pharmaceutically acceptable salt or prodrug thereof: 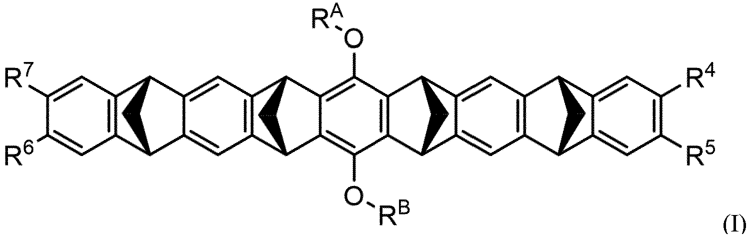 wherein:
   R^{A} is 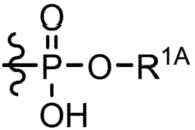 and;
   R^{B} is 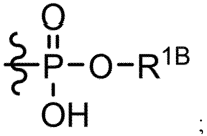 or
   R^{A} is 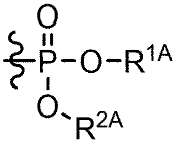 and
   R^{B} is 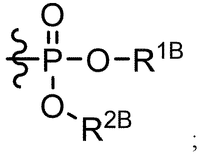 and
      R^{1A}, R^{1B}, R^{2A}, R^{2B} are selected from unsubstitued or substituted alkyl, alkenyl, or alkynyl, whereby the substituents are selected from halogen, hydroxyl, carbonyl, thiocarbonyl, alkoxy, phosphoryl, phosphate, phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, aromatic or heteroaromatic moieties which themselves can be substituted;
      R⁴ and R⁵ are independently selected from H, halo, alkyl, alkenyl, alkynyl, amino, amide, carboxyl, ester, or nitro; or R⁴ and R⁵, together with the atoms that separate them, complete a cycloalkyl, aryl, or heteroaryl;
      R⁶ and R⁷ are independently selected from H, halo, alkyl, alkenyl, alkynyl, amino, amide, carboxyl, ester, or nitro; or R⁶ and R⁷, together with the atoms that separate them, complete a cycloalkyl, aryl, or heteroaryl.
Comparative Embodiment 2: The compound of embodiment 1, whereby R^{1A} and R^{1B} are the same.:
Embodiment 3: The compound of embodiment 1 or 2, having the structure: 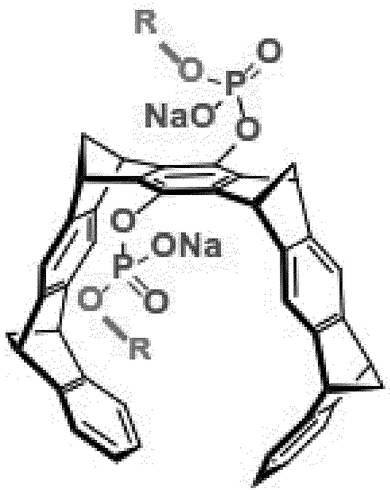 wherein R is CH₃, CH₃CH₂, (CH₃)₂CH, CH₂C≡CH, CH₃CH₂CH₂CH₂, CH₂CH₂C≡CH, CH₃CH₂CH-CH₃, CH₃(CH₂)₆CH₂, CH₃(CH₂)₁₄CH₂ or CH₃(CH₂)₁₆CH_{2.}
Comparative Embodiment 4: The compound of any one of the embodiments 1-3, wherein R^{B} is H or -P(O)(OH)₂.
Comparative Embodiment 5: The compound of any one of embodiments 1-4, wherein R^{A} is alkyl.
Comparative Embodiment 6: The compound of any one of embodiments 1-4, wherein R^{A} is 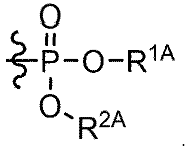
Comparative Embodiment 7: The compound of any one of embodiments 1-6, wherein R^{1A} is alkynyl, such as but-3-ynyl.
Comparative Embodiment 8: The compound of any one of embodiments 1-7, wherein R^{2A} is H or alkyl, such as methyl.
Comparative Embodiment 9: The compound of any one of embodiments 1-8, wherein R^{B} is 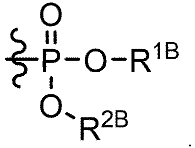
Comparative Embodiment 10: The compound of any one of embodiments 1-9, wherein R^{1B} is alkynyl, such as but-3-ynyl.
Comparative Embodiment 11: The compound of any one of embodiments 1-10, wherein R^{1B} is alkyl, such as methyl.
Comparative Embodiment 12: The compound of any one of embodiments 1-11, wherein R^{2B} is H or alkyl, such as methyl.
Comparative Embodiment 13: The compound of any one of embodiments 1-12, wherein R^{1A}, R^{2A}, R^{1B}, and R^{2B} are independently selected from alkyl, alkenyl, or alkynyl.
Comparative Embodiment 14: The compound of any one of embodiments 1-13, wherein R^{1A} and R^{1B} are independently selected from alkyl, alkenyl, or alkynyl; and R^{2A} and R^{2B} are H.
Comparative Embodiment 15: The compound of any one of embodiments 1-14, wherein R^{1A} is alkyl, alkenyl, or alkynyl; and R^{1B}, R^{2A}, and R^{2B} are H.
Comparative Embodiment 16: The compound of any one of embodiments 1-15, wherein R^{A} is 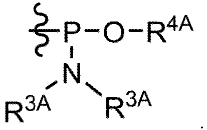
Comparative Embodiment 17: The compound of any one of embodiments 1-16, wherein each R^{3A} is alkyl, such as isopropyl.
Comparative Embodiment 18: The compound of any one of embodiments 1-17, wherein each R^{4A} is alkyl, such as 2-cyanoethyl.
Comparative Embodiment 19: The compound of any one of embodiments 1-18, wherein R^{B} is 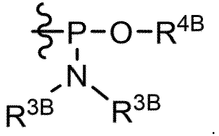
Comparative Embodiment 20: The compound of any one of embodiments 1-19, wherein each R^{3B} is AN independently selected alkyl, such as isopropyl.
Comparative Embodiment 21: The compound of any one of embodiments 1-20, wherein each R^{4A} is an independently selected alkyl, such as 2-cyanoethyl.
Comparative Embodiment 22: The compound of any one of embodiments 1-21, wherein:
   R^{A} is 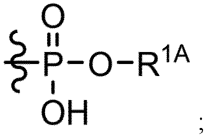
   R^{B} is 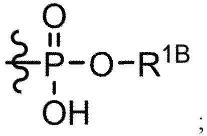 and
   R^{1A} and R^{1B} are selected from alkyl, alkenyl, or alkynyl, such as ethyl, isopropyl, or octyl.
Comparative Embodiment 23: The compound of any one of embodiments 1-22, wherein:
   R^{A} is 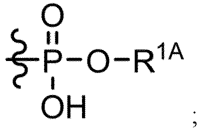
   R^{B} is 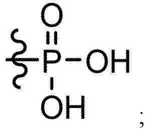 and
   R^{1A} is alkyl, alkenyl, or alkynyl, such as ethyl, isopropyl, or octyl.
Comparative Embodiment 24: The compound of any one of embodiments 1-23, wherein:
   R^{A} is alkyl, such as methyl, ethyl, trifluoromethyl, or trifluoroethyl; and
   R^{B} is 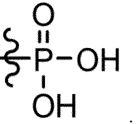
Comparative Embodiment 25: The compound of any one of the preceding embodiments, wherein:
   R^{A} is 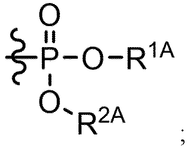
   R^{B} is 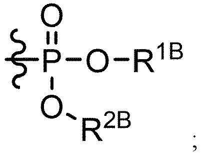 and
   R^{1A}, R^{1B}, R^{2A} and R^{2B} are selected from alkyl, alkenyl, or alkynyl, such as ethyl, isopropyl, or octyl.
Comparative Embodiment 26: The compound of any one of embodiments 1-25, wherein R⁴ and R⁵ are independently selected from H, halo, alkyl, alkenyl, alkynyl, amino, amide, carboxyl, ester, or nitro.
Comparative Embodiment 27: The compound of embodiment 26, wherein R⁴ and R⁵ are H.
Comparative Embodiment 28: The compound of embodiment 26, wherein at least one of R⁴ and R⁵ is halo, alkyl, alkenyl, alkynyl, amino, amide, carboxyl, ester, or nitro.
Comparative Embodiment 29: The compound of any one of embodiments 1-25, wherein R⁴ and R⁵, together with the atoms that separate them, complete a cycloalkyl, aryl, or heteroaryl.
Comparative Embodiment 30: The compound of any one of embodiments 1-29, wherein R⁶ and R⁷ are independently selected from H, halo, alkyl, alkenyl, alkynyl, amino, amide, carboxyl, ester, or nitro.
Comparative Embodiment 31: The compound of embodiment 30, wherein R⁶ and R⁷ are H.
Comparative Embodiment 32: The compound of embodiment 30, wherein at least one of R⁶ and R⁷ is halo, alkyl, alkenyl, alkynyl, amino, amide, carboxyl, ester, or nitro.
Comparative Embodiment 33: The compound of any one of embodiments 1-29, wherein R⁶ and R⁷, together with the atoms that separate them, complete a cycloalkyl, aryl, or heteroaryl.
Comparative Embodiment 34: The compound of any one of embodiments 1-33, wherein the compound is one of Compounds **2-19,** B, D-K, or M.
Embodiment 35: A pharmaceutical composition comprising:
   a compound of any one of embodiments 1-34; and
   a pharmaceutically acceptable carrier.
Comparative Embodiment 36: The pharmaceutical composition of embodiment 35, wherein said composition is formulated for administration via a route selected from the group consisting of intraperitoneal administration, topical administration, oral administration, inhalation administration, transdermal administration, subdermal depot administration, sub-dural administration, and rectal administration.
Comparative Embodiment 37: The pharmaceutical composition of any one of embodiments 35-36, wherein said composition comprises a unit dosage formulation.
Embodiment 38: Composition of any one of embodiments 1-34 for use in disrupting protein aggregation, comprising contacting a protein or a protein aggregate with a compound *(e.g.,* a molecular tweezers) of any one of embodiments 1-34.
Comparative Embodiment 39: The use of embodiment 38, wherein said use comprises inhibiting protein aggregation in a mammal, and said use comprises administering to said mammal an effective amount of said compound.
Comparative Embodiment 40: The use of embodiment 39, wherein said use comprises a use of mitigating one or more symptoms of a disease in a mammal characterized by amyloidosis, wherein said effective amount is an amount sufficient to partially or fully inhibit aggregation of an amyloidogenic protein.
Comparative Embodiment 41: The use of any one of embodiments 38-40, wherein said use slows or stops protein aggregation.
Comparative Embodiment 42: The use of any one of embodiments 38-40, wherein said use induces disaggregation of an aggregated protein.
Comparative Embodiment 43: The use of any one of embodiments 40-42, wherein said disease is a disease selected from the diseases listed in Table 1.
Comparative Embodiment 44: The use of any one of embodiments 40-42, wherein said amyloidogenic protein is an amyloidogenic protein selected from the amyloidogenic proteins listed in Table 1.
Comparative Embodiment 45: The use of embodiment 44, wherein said amyloidogenic protein comprises a protein selected from the group consisting of Aβ, Aβ fragment, Aβ variant (mutant), tau, Gelsolin, ADan, ABri, Islet amyloid polypeptide (amylin), α-synuclein, Huntingtin, Atrophin 1, Ataxin 1-3, β₂-microglobulin, , β₂-microglobulin, Fibrinogen α-chain, Serum amyloid A, Cystatin C, Fibrinogen α-chain, Gelsolin, Transthyretin, Cystatin C, and the neurotoxic prion protein (PrP) fragment¹⁰⁶⁻¹²⁶.
Comparative Embodiment 46: The use of embodiment 44, wherein said amyloidogenic protein comprises a Aβ, an Aβ fragment, or α-synuclein.
Comparative Embodiment 47: The use of any one of embodiments 40-46, wherein said mammal is a human diagnosed as having or at risk for a pathology listed in Table 1.
Comparative Embodiment 48: The use of any one of embodiments 39-47, wherein said mammal is a non-human mammal.
Comparative Embodiment 49: The use of any one of embodiments 39-47, wherein said mammal is a human.
Embodiment 50: Composition of any one of embodiments 1-34 for use in the treatment of a subject having a spinal cord injury or traumatic brain injury, said method comprising administering to said subject a molecular tweezers of any one of embodiments 1-34 in an amount sufficient to reduce aggregation and/or cytotoxicity of said amyloidogenic protein.
Comparative Embodiment 51: The use of embodiment 50, wherein said molecular tweezers is administered in an amount sufficient to ameliorate one or more symptoms of said spinal cord injury or traumatic brain injury.
Comparative Embodiment 52: The use of embodiment 51, wherein said amelioration comprises one or more responses selected from the group consisting of improved neuronal survival, improved neuronal regeneration, improvement/recovery of motor function, improvement/recovery of fine motor coordination, improvement/recovery from muscle spasticity, improvement/recovery from paresis or paralysis of one or both sides, reduction in severity and/or number of seizure disorders, improvement/recovery of balance, improvement/recovery of gait, improvement/recovery of cognitive function (e.g., improvement/recovery from short- and long-term memory deficits, improvement/recovery of impaired concentration, improvement/recovery from slowness of thinking and limited attention span), improvement/recovery of perception, improvement/recovery of communication, improvement/recovery of reading and writing skills, improvement/recovery of planning, improvement/recovery of sequencing, improvement/recovery of judgment, improvement/recovery of sensory function (e.g., improvement/recovery of hearing, improvement/recovery of sight, improvement/recovery of smell, improvement/recovery of taste).
Comparative Embodiment 53: The use of embodiment 51, wherein said amelioration comprises amelioration of one or more deficits selected from the group consisting of impairment of sensation, impairment of motor function, dysfunction of the bowel, dysfunction of the bladder, sexual dysfunction, impairment of fertility, inability to effectively regulate blood pressure, impairment of thermoregulation, impairment of sweating, chronic pain, and impairment of involuntary functions (e.g., breathing).
Comparative Embodiment 54: The use of any one of embodiments 50-53, wherein said subject has a spinal cord injury.
Comparative Embodiment 55: The use of any one of embodiments 50-53, wherein said subject has a traumatic brain injury.
Comparative Embodiment 56: The use of embodiment 55, wherein said traumatic brain injury is caused by an event selected from the group consisting of a falls, a vehicle-related collision, a gunshot wound, domestic violence, child abuse, a sports injury, an explosive blasts, and a combat injuries.
Comparative Embodiment 57: The use of embodiment 55, wherein said traumatic brain injury is caused by an ischemic event.
Comparative Embodiment 58: The use of any one of embodiments 50-57, wherein said protein is a synuclein protein.
Comparative Embodiment 59: The use of any one of embodiments 50-57, wherein said amyloidogenic protein is a Tau protein.
Comparative Embodiment 60: The use of any one of embodiments 50-57, wherein said amyloidogenic protein is an Aβ peptide.
Comparative Embodiment 61: The use of any one of embodiments 50-60, wherein said administration is parenteral.
Comparative Embodiment 62: The use of embodiment 61, wherein said administration is intraspinal.
Comparative Embodiment 63: The use of embodiment 61, wherein said administration is intrathecal or epidural.
Comparative Embodiment 64: The use of embodiment 61, wherein said administration is subdural.
Comparative Embodiment 65: The use of embodiment 61, wherein said administration is subcutaneous.
Comparative Embodiment 66: The use of embodiment 61, wherein said administration is intravenous.
Comparative Embodiment 67: The use of embodiment 61, wherein said administration is through a subcutaneously implanted device.
Comparative Embodiment 68: The use of embodiment 61, wherein said administration is through a cannula.
Comparative Embodiment 69: The use of any one of embodiments 50-68, wherein said molecular tweezers is administered to said subject within one week of said injury.
Comparative Embodiment 70: The use of embodiment 69, wherein said molecular tweezers is administered to said subject within 72 hours of said injury.
Comparative Embodiment 71: The use of embodiment 69, wherein said molecular tweezers is administered to said subject within 24 hours of said injury.
Embodiment 72: Composition of any one of embodiments 1-34 for use useof inhibiting the growth, and/or the proliferation, and/or the infectivity, of an enveloped virus, said use comprising contacting said virus with an effective amount of a compound of any one of embodiments 1-34.
Comparative Embodiment 73: The use of embodiment 72, wherein said use comprises administering said compound to a mammal infected with said virus or at risk of infection with said virus.
Comparative Embodiment 74: The use of embodiment 73, wherein said mammal is a mammal infected with said virus.
Comparative Embodiment 75: The use of any one of embodiments 72-74, wherein said mammal is a non-human mammal.
Comparative Embodiment 76: The use of any one of embodiments 72-74, wherein said mammal is a human.
Comparative Embodiment 77: The use of any one of embodiments 72-76, wherein said virus is a member of a family selected from the group consisting of Herpesviridae, Poxviridae, Hepadnaviridae, Coronaviridae, Flaviviridae, Togaviridae, Retroviridae, Orthomyxoviridae, Arenaviridae, Bunyaviridae, Filoviridae, Paramyxoviridae, and Rhabdoviridae.
Comparative Embodiment 78: The use of any one of embodiments 72-77, wherein said virus is selected from the group consisting of Herpes simplex, type 1, Herpes simplex, type 2, Varicella-zoster virus, Epstein-Barr virus, Human cytomegalovirus, Human herpesvirus, type 8, Smallpox, Hepatitis B virus, Severe acute respiratory syndrome virus, Hepatitis C virus, yellow fever virus, dengue virus, West Nile virus, TBE virus, Zika virus, Rubella virus, Human immunodeficiency virus (HIV), Influenza virus, Lassa virus, Crimean-Congo hemorrhagic fever virus, Hantaan virus, Ebola virus, Marburg virus, Measles virus, Mumps virus, Parainfluenza virus, Respiratory syncytial virus, Rabies virus, and Hepatitis D virus (HDV).
Comparative Embodiment 79: The use of any one of embodiments 72-77, wherein said virus is Zika virus or HIV-1.
Comparative Embodiment 80: The use of any one of embodiments 72-79, wherein said use ameliorates one of more symptoms of a pathology caused by said virus and/or slows or prevents infection of said mammal by said virus.
Comparative Embodiment 81: A use of treating a lipofuscin-related disorder in a mammal, said use comprising:
administering to said mammal in need thereof an effective amount of a compound (molecular tweezers) of any one of embodiments 1-34.
Comparative Embodiment 82: The use of embodiment 81, wherein said molecular tweezers is capable of inhibiting lipofuscin aggregation.
Comparative Embodiment 83: The use of any one of embodiments 81-82, wherein said effective amount is an amount effective to delay the onset of, or to slow the progression, or stop, or to reverse lipofuscin accumulation/aggregation associated with said lipofuscin-related disorder.
Comparative Embodiment 84: The use of any one of embodiments 81-83, wherein said effective amount is an amount effective to ameliorate one or more symptoms of the lipofuscin-related disorder.
Comparative Embodiment 85: The use of any one of embodiments 81-83, wherein said effective amount is an amount effective to delay or stop the onset of, or to slow, or to stop, or to reverse progression of the lipofuscin-related disorder.
Comparative Embodiment 86: The use of any one of embodiments 85, where said lipofuscin-related disorder is selected from the group consisting of a lipofuscin-related disorder associated with an eye disease, a neuronal ceroid lipofuscinosis (e.g. Batten disease), acromegaly, amyotrophic lateral sclerosis, denervation atrophy, lipid myopathy, chronic obstructive pulmonary disease, centronuclear myopathy, and melanosis coli.
Comparative Embodiment 87: The use of any one of embodiments 81-86, wherein said lipofuscin-related disorder is a lipofuscin-related disorder associated with an eye disease.
Comparative Embodiment 88: The use of embodiment 87, wherein said eye disease comprises a disease selected from the group consisting of age-related macular degeneration, Stargardt disease, vitelliform macular degeneration (Best's macular dystrophy), retinal pigment epitheliopathy associated with choroidal melanoma, and severe ocular trauma.
Comparative Embodiment 89: The use of embodiment 88, wherein said lipofuscin-related disorder comprises macular degeneration.
Comparative Embodiment 90: The use of embodiment 89, wherein said amelioration of one or more symptoms comprises an amelioration of one or more symptoms selected from the group consisting of drusen or waste deposits on the surface of the retina, changes in color (pigment) of the macula, blurred or fuzzy vision, the illusion that straight lines are wavy; the illusion that some objects are smaller than they really are, the appearance of a gray, dark or empty area in the center of the visual field, and fading of color vision.
Comparative Embodiment 91: The use of embodiment 89, wherein said administration is effective to delay the onset of, or to slow, stop, or reverse one or more processes selected from the group consisting of formation of drusen or waste deposits on the surface of the retina, changes in color (pigment) of the macula, blurring of fuzziness of vision; the illusion that straight lines are wavy; the illusion that some objects are smaller than they really are, the appearance of a gray, dark or empty area in the center of the visual field, and fading of color vision.
Comparative Embodiment 92: The use of embodiment 86, wherein said eye disease comprises Stargardt disease.
Comparative Embodiment 93: The use of embodiment 92, wherein said amelioration of one or more symptoms comprises an amelioration of one or more symptoms selected from the group consisting of blurry or distorted vision, inability to see in low lighting, difficulty recognizing familiar faces, and loss of color vision.
Comparative Embodiment 94: The use of embodiment 92, wherein said administration is effective to slow, stop, or reverse one or more processes selected from the group consisting of blurry or distortion of vision, inability to see in low lighting, difficulty recognizing familiar faces, and loss of color vision.
Comparative Embodiment 95: The use of any one of embodiments 81-85, wherein said lipofuscin-related disorder is a neuronal ceroid lipofuscinosis.
Comparative Embodiment 96: The use of embodiment 95, wherein said lipofuscin-related disorder comprises a lipofuscinosis selected from the group consisting of infantile NCL (Santavuori-Haltia disease), late Infantile NCL (Jansky-Bielschowsky disease, Juvenile NCL (CLN1, Batten disease), Adult NCL (Kufs disease), Finnish Late Infantile NCL, Variant Late Infantile NCL, CLN7 NCL, CLN8 NCL (Northern Epilepsy, progressive epilepsy with mental retardation (EPMR)), Turkish Late Infantile Variant NCL, Batten disease, and CLN10 NCL (Congenital, Cathepsin D Deficiency).
Comparative Embodiment 97: The use of embodiment 96, wherein said lipofuscinosis comprises Batten Disease.
Comparative Embodiment 98: The use of any one of embodiments 95-97, wherein said amelioration of one or more symptoms comprises an amelioration of one or more symptoms selected from the group consisting of cognitive dysfunction, movement/locomotor dysfunction, and vision loss.
Comparative Embodiment 99: The use of any one of embodiments 95-97, wherein said administration is effective to slow, stop, or reverse one or more processes selected from the group consisting of progression of cognitive dysfunction, progression of movement/locomotor dysfunction, and progression of vision loss.
Comparative Embodiment 100: The use of any one of embodiments 81-85, wherein said lipofuscin-related disorder is selected from the group consisting of acromegaly, amyotrophic lateral sclerosis, denervation atrophy, lipid myopathy, chronic obstructive pulmonary disease, centronuclear myopathy, and melanosis coli.
Comparative Embodiment 101: The use of any one of embodiments 81-100, wherein said administration is via a route selected from the group consisting of oral delivery, isophoretic delivery, transdermal delivery, parenteral delivery, aerosol administration, administration via inhalation, intravenous administration, ocular administration, depot delivery (including subcutaneous/subdermal depot delivery), vaginal administration, and rectal administration.
Comparative Embodiment 102: The use of any one of embodiments 81-100, wherein said administration comprises ocular administration.
Comparative Embodiment 103: The use of embodiment 102, wherein said ocular administration comprises administration via a route selected from the group consisting of topical ocular administration, topical ocular administration in combination with systemic and/or oral administration, IVT injection, periocular administration, subconjunctival injection, subtenon injection, intravitreal injection, subretinal administration and retrobulbar injection.
Comparative Embodiment 104: The use of any one of embodiments 81-100, wherein said administration is parenteral.
Comparative Embodiment 105: The use of embodiment 104, wherein said administration comprises administration to the central nervous system.
Comparative Embodiment 106: The use of embodiment 105, wherein said administration is selected from a route selected from the group consisting of intraspinal administration, intrathecal or epidural administration, subdural administration, and administration is through a cannula.
Comparative Embodiment 107: The use of embodiment 104, wherein said administration is selected from a route selected from the group consisting of subcutaneous administration, intravenous administration, and administration through a subcutaneously implanted device.
Comparative Embodiment 108: The use of any one of embodiments 81-107, wherein said mammal is a human.
Comparative Embodiment 109: The use of any one of embodiments 81-107, wherein said mammal is non-human mammal.
Comparative Embodiment 110: A use of treating a lysosomal storage disease in a mammal, said use comprising:
administering to said mammal an effective amount of a compound *(e.g.,* a molecular tweezers) of any one of embodiments 1-34.
Comparative Embodiment 111: The use of embodiment 110, wherein said effective amount is an amount effective to slow the progression, or stop, or reverse protein accumulation/aggregation associated with said lysosomal storage disease.
Comparative Embodiment 112: The use of any one of embodiments 110-111, wherein said effective amount is an amount effective to ameliorate one or more symptoms of the pathology associated with said lysosomal storage disease.
Comparative Embodiment 113: The use of any one of embodiments 110-111, wherein said effective amount is an amount effective to delay the onset, or to slow, or to stop, or to reverse progression of a pathology associated with said lysosomal storage disease.
Comparative Embodiment 114: The use of any one of embodiments 110-113, wherein said administration is before appearance of symptoms in said mammal.
Comparative Embodiment 115: The use of embodiment 114, wherein said mammal is identified as having the lysosomal storage disease by the presence of a genetic marker for said lysosomal storage disease.
Comparative Embodiment 116: The use of any one of embodiments 110-113, wherein said administration is within 3 months of birth, or within 6 months of birth, or within 1 year of birth, or within 3 years of birth.
Comparative Embodiment 117: The use of any one of embodiments 110-116, wherein said lysosomal storage disease comprises a lysosomal storage disease characterized by neurological impairment and/or neurodegenerative processes.
Comparative Embodiment 118: The use of embodiment 117, wherein said lysosomal storage disease comprises a pathology selected from the group consisting of a mucopolysaccharidosis (MPS), aspartylglucosaminuria, GM1-gangliosidosis, Krabbe (globoid cell leukodystrophy or galactosylceramide lipidosis), Metachromatic leukodystrophy, Sandhoff disease, mucolipidosis type II (I-cell disease), mucolipidosis type IIIA (pseudo-Hurler polydystrophy), Niemann-Pick disease type C2 and C1, Danon disease, free sialic acid storage disorder, mucolipidosis type IV, and multiple sulfatase deficiency (MSD).
Comparative Embodiment 119: The use of embodiment 118, wherein said lysosomal storage disease comprises a mucopolysaccharidosis selected from the group consisting of Sanfilippo syndrome (MPS III), Hurler syndrome (MPS IH), Hurler-Scheie syndrome (MPS I-H/S), Scheie syndrome (MPS IS), Hunter syndrome (MPS II), Morquio syndrome (MP IV), Maroteaux-Lamy syndrome (MPS VI), Sly syndrome (MPS VII), and MPS IX.
Comparative Embodiment 120: The use of embodiment 119, wherein said mucopolysaccharidosis comprises Sanfilippo syndrome (MPS III).
Comparative Embodiment 121: The use of any one of embodiments 119-120, wherein said amelioration of one or more symptoms of Sanfilippo syndrome (MPS III) comprises an amelioration of one or more symptoms selected from the group consisting of cognitive deficiencies, claw hand, visceromegaly, sleep disorders, loss of motor function, loss of communication abilities, and seizures.
Comparative Embodiment 122: The use of any one of embodiments 119-120, wherein said use comprises slowing, stopping, or reversing the progression of one or more symptoms of Sanfilippo syndrome (MPS III) selected from the group consisting of cognitive deficiencies, claw hand, visceromegaly, sleep disorders, loss of motor function, loss of communication abilities, and seizures.
Comparative Embodiment 123: The use of embodiment 118, wherein said lysosomal storage disease comprises aspartylglucosaminuria.
Comparative Embodiment 124: The use of embodiment 123, wherein amelioration of one or more symptoms or aspartylglucosaminuria comprises an amelioration of one or more symptoms selected from the group consisting of delay or loss of speech, cognitive impairment, seizures, locomotor impairment, osteoporosis, and joint hypermobility.
Comparative Embodiment 125: The use of embodiment 123, wherein said use comprises slowing, stopping, or reversing the progression of one or more symptoms of aspartylglucosaminuria selected from the group consisting of increasing loss of speech, increasing cognitive impairment, increasing frequency and/or severity of seizures, increasing locomotor impairment, increasing osteoporosis, and increasing joint hypermobility.
Comparative Embodiment 126: The use of embodiment 118, wherein said lysosomal storage disease comprises GM1-gangliosidosis.
Comparative Embodiment 127: The use of embodiment 126, wherein amelioration of one or more symptoms of GM1-gangliosidosis comprises an amelioration of one or more symptoms selected from the group consisting of cognitive impairment, locomotor impairment, hepatosplenomegaly, skeletal abnormalities, seizures, clouding of the cornea, and loss of vision.
Comparative Embodiment 128: The use of embodiment 126, wherein said use comprises slowing, stopping, or reversing the progression of one or more symptoms of GM1-gangliosidosis selected from the group consisting of increasing cognitive impairment, progressive locomotor impairment, progressive hepatosplenomegaly, increasing skeletal abnormalities, increasing frequency and/or severity of seizures, increasing clouding of the cornea, and increasing loss of vision.
Comparative Embodiment 129: The use of embodiment 118, wherein said lysosomal storage disease comprises Krabbe disease (globoid cell leukodystrophy or galactosylceramide lipidosis).
Comparative Embodiment 130: The use of embodiment 129, wherein amelioration of one or more symptoms of Krabbe disease comprises an amelioration of one or more symptoms selected from the group consisting of irritability, fevers, limb stiffness, seizures, feeding difficulties, vomiting, and cognitive impairment, locomotor impairment, muscle weakness, spasticity, deafness, optic atrophy, optic nerve enlargement, blindness, paralysis, and difficulty when swallowing.
Comparative Embodiment 131: The use of embodiment 129, wherein said use comprises slowing, stopping, or reversing the progression of one or more symptoms of Krabbe disease selected from the group consisting of increasing irritability, fevers, limb stiffness, increasing frequency and/or severity of seizures, feeding difficulties, vomiting, cognitive impairment, locomotor impairment, muscle weakness, spasticity, deafness, optic atrophy, optic nerve enlargement, blindness, paralysis, and difficulty when swallowing.
Comparative Embodiment 132: The use of embodiment 118, wherein said lysosomal storage disease comprises metachromatic leukodystrophy.
Comparative Embodiment 133: The use of embodiment 132, wherein amelioration of one or more symptoms of leukodystrophy comprises an amelioration of one or more symptoms selected from the group consisting of leukodystrophy throughout CNS and/or peripheral nervous system, cognitive impairment, loss of sensation in the extremities (peripheral neuropathy), incontinence, seizures, paralysis, an inability to speak, blindness, and hearing loss.
Comparative Embodiment 134: The use of embodiment 132, wherein said use comprises slowing, stopping, or reversing the progression of one or more symptoms of leukodystrophy selected from the group consisting of increasing leukodystrophy throughout CNS and/or peripheral nervous system, cognitive impairment, loss of sensation in the extremities (peripheral neuropathy), incontinence, seizures, paralysis, an inability to speak, blindness, and hearing loss.
Comparative Embodiment 135: The use of embodiment 118, wherein said lysosomal storage disease comprises Sandhoff disease.
Comparative Embodiment 136: The use of embodiment 135, wherein amelioration of one or more symptoms of Sandhoff disease comprises cognitive impairment, loss of locomotor function, seizures, hearing loss vision loss, organomegaly, bone abnormalities, and paralysis.
Comparative Embodiment 137: The use of embodiment 135, wherein said use comprises slowing, stopping, or reversing the progression of one or more symptoms of Sandhoff disease selected from the group consisting of cognitive impairment, loss of locomotor function, seizures, hearing loss vision loss, organomegaly, bone abnormalities, and paralysis.
Comparative Embodiment 138: The use of embodiment 118, wherein said lysosomal storage disease comprises mucolipidosis type II (I-cell disease).
Comparative Embodiment 139: The use of embodiment 138, wherein amelioration of one or more symptoms of mucolipidosis type II comprises an amelioration of one or more symptoms selected from the group consisting of weak muscle tone (hypotonia), growth impairment, bone abnormalities, hyphosis, club feet, impaired mobility, heart valve abnormalities, prolonged or recurrent respiratory and/or ear infections, and hearing loss.
Comparative Embodiment 140: The use of embodiment 138, wherein said use comprises slowing, stopping, or reversing the progression of one or more symptoms of mucolipidosis type II selected from the group consisting of weak muscle tone (hypotonia), growth impairment, bone abnormalities, hyphosis, club feet, impaired mobility, heart valve abnormalities, prolonged or recurrent respiratory and/or ear infections, and hearing loss.
Comparative Embodiment 141: The use of embodiment 118, wherein said lysosomal storage disease comprises mucolipidosis type IIIA (pseudo-Hurler polydystrophy).
Comparative Embodiment 142: The use of embodiment 141, wherein amelioration of one or more symptoms comprises an amelioration of one or more symptoms of mucolipidosis type IIIA selected from the group consisting of joint stiffness, scoliosis, skeletal deformities of the hands (*e.g*., claw-hands), growth delays, deterioration of the hip joints, clouding of the corneas of the eyes, mild mental retardation, easy fatigability, carpal tunnel syndrome, and heart disease.
Comparative Embodiment 143: The use of embodiment 141, wherein said use comprises slowing, stopping, or reversing the progression of one or more symptoms of mucolipidosis type IIIA selected from the group consisting of joint stiffness, scoliosis, skeletal deformities of the hands (*e.g*., claw-hands), growth delays, deterioration of the hip joints, clouding of the corneas of the eyes, mild mental retardation, easy fatigability, carpal tunnel syndrome, and heart disease.
Comparative Embodiment 144: The use of embodiment 118, wherein said lysosomal storage disease comprises Niemann-Pick disease type C2 and C1.
Comparative Embodiment 145: The use of embodiment 144, wherein amelioration of one or more symptoms comprises an amelioration of one or more symptoms of Niemann-Pick disease selected from the group consisting of splenomegaly, hepatomegaly, hepatosplenomegaly, jaundice, cognitive impairment, cerebellar ataxia (unsteady walking with uncoordinated limb movements), dysarthria (slurred speech), dysphagia (difficulty in swallowing), tremor, epilepsy (both partial and generalized), vertical supranuclear palsy (upgaze palsy, downgaze palsy, saccadic palsy or paralysis), sleep inversion, gelastic cataplexy (sudden loss of muscle tone or drop attacks), dystonia (abnormal movements or postures caused by contraction of agonist and antagonist muscles across joints), spasticity (velocity dependent increase in muscle tone), hypotonia, ptosis (drooping of the upper eyelid), psychosis, progressive dementia, progressive hearing loss, bipolar disorder, major and psychotic depression, loss of volitional movement, and severe dementia.
Comparative Embodiment 146: The use of embodiment 144, wherein said use comprises slowing, stopping, or reversing the progression of one or more symptoms of Niemann-Pick disease selected from the group consisting of splenomegaly, hepatomegaly, hepatosplenomegaly, jaundice, cognitive impairment, cerebellar ataxia (unsteady walking with uncoordinated limb movements), dysarthria (slurred speech), dysphagia (difficulty in swallowing), tremor, epilepsy (both partial and generalized), vertical supranuclear palsy (upgaze palsy, downgaze palsy, saccadic palsy or paralysis), sleep inversion, gelastic cataplexy (sudden loss of muscle tone or drop attacks), dystonia (abnormal movements or postures caused by contraction of agonist and antagonist muscles across joints), spasticity (velocity dependent increase in muscle tone), hypotonia, ptosis (drooping of the upper eyelid), psychosis, progressive dementia, progressive hearing loss, bipolar disorder, major and psychotic depression, loss of volitional movement, and severe dementia.
Comparative Embodiment 147: The use of embodiment 118, wherein said lysosomal storage disease comprises Danon disease.
Comparative Embodiment 148: The use of embodiment 147, wherein amelioration of one or more symptoms of Danon disease comprises an amelioration of one or more symptoms selected from the group consisting of cardiomyopathy, skeletal muscle myopathy, and cognitive impairment.
Comparative Embodiment 149: The use of embodiment 147, wherein said use comprises slowing, stopping, or reversing the progression of one or more symptoms of Danon disease selected from the group consisting of cardiomyopathy, skeletal muscle myopathy, and cognitive impairment.
Comparative Embodiment 150: The use of embodiment 118, wherein said lysosomal storage disease comprises free sialic acid storage disorder.
Comparative Embodiment 151: The use of embodiment 150, wherein amelioration of one or more symptoms of free sialic acid storage disorder comprises an amelioration of one or more symptoms selected from the group consisting of cognitive impairment, developmental delay, weak muscle tone (hypotonia), failure to gain weight and grow at the expected rate (failure to thrive), bone malformations, an enlarged liver and spleen (hepatosplenomegaly), and an enlarged heart (cardiomegaly).
Comparative Embodiment 152: The use of embodiment 150, wherein said use comprises slowing, stopping, or reversing the progression of one or more symptoms of free sialic acid storage disorder selected from the group consisting of cognitive impairment, developmental delay, weak muscle tone (hypotonia), failure to gain weight and grow at the expected rate (failure to thrive), bone malformations, an enlarged liver and spleen (hepatosplenomegaly), and an enlarged heart (cardiomegaly).
Comparative Embodiment 153: The use of embodiment 118, wherein said lysosomal storage disease comprises mucolipidosis type IV.
Comparative Embodiment 154: The use of embodiment 153, wherein amelioration of one or more symptoms of mucolipidosis type IV comprises an amelioration of one or more symptoms selected from the group consisting of delayed development, vision impairment, psychomotor delay, cognitive impairment, limited or absent speech, difficulty chewing and swallowing, weak muscle tone (hypotonia), abnormal muscle stiffness (spasticity), locomotor impairment, clouding of the cornea, and impaired production of stomach acid (achlorhydria).
Comparative Embodiment 155: The use of embodiment 153, wherein said use comprises slowing, stopping, or reversing the progression of one or more symptoms of mucolipidosis type IV selected from the group consisting of delayed development, vision impairment, psychomotor delay, cognitive impairment, limited or absent speech, difficulty chewing and swallowing, weak muscle tone (hypotonia), abnormal muscle stiffness (spasticity), locomotor impairment, clouding of the cornea, and impaired production of stomach acid (achlorhydria).
Comparative Embodiment 156: The use of embodiment 118, wherein said lysosomal storage disease comprises multiple sulfatase deficiency (MSD).
Comparative Embodiment 157: The use of embodiment 156, wherein amelioration of one or more symptoms of multiple sulfatase deficiency comprises an amelioration of one or more symptoms selected from the group consisting of leukodystrophy, scoliosis, hepatosplenomegaly, psychomotor regression), and ichthyosis.
Comparative Embodiment 158: The use of embodiment 156, wherein said use comprises slowing, stopping, or reversing the progression of one or more symptoms of multiple sulfatase deficiency selected from the group consisting of leukodystrophy, scoliosis, hepatosplenomegaly, psychomotor regression), and ichthyosis.
Comparative Embodiment 159: The use of any one of embodiments 117-158, wherein said amelioration of one or more symptoms comprises a reduction of neuroinflammation.
Comparative Embodiment 160: The use of embodiment 159, wherein said reduction of neuro-inflammation comprises a reduction in one or more markers of neuroinflammation, wherein said marker(s) of neuroinflammation are selected from the group consisting of Iba1 (marker microglial activation) , GFAP (marker for astrocytic response), TNF-alpha, interleukins, and TGF-beta.
Comparative Embodiment 161: The use of any one of embodiments 117-160, wherein said amelioration of one or more symptoms comprises a reduction in neuronal loss (neurodegeneration).
Comparative Embodiment 162: The use of any one of embodiments 117-158, wherein use is effective to slow, or to stop, or to reverse progression of neuroinflammation in said mammal.
Comparative Embodiment 163: The use of embodiment 162, wherein said use is effective to slow, or to stop, or to reverse progression of neuro-inflammation as characterized by a reduction in one or more markers of neuroinflammation, wherein said marker(s) of neuroinflammation are selected from the group consisting of Iba1 (marker microglial activation) , GFAP (marker for astrocytic response), TNF-alpha, interleukins, and TGF-beta.
Comparative Embodiment 164: The use of any one of embodiments 117-158, and 162-163, wherein said use is effective to slow, or to stop, or to reverse neuronal loss (neurodegeneration) in said mammal.
Comparative Embodiment 165: The use of any one of embodiments 110-164, wherein said administration is via a route selected from the group consisting of oral delivery, isophoretic delivery, transdermal delivery, parenteral delivery, aerosol administration, administration via inhalation, intravenous administration, ocular administration, depot delivery, vaginal administration, and rectal administration.
Comparative Embodiment 166: The use of any one of embodiments 110-164, wherein said administration is parenteral.
Comparative Embodiment 167: The use of embodiment 166, wherein said administration is selected from a route selected from the group consisting of intraspinal administration, intrathecal or epidural administration, subdural administration, subcutaneous administration, intravenous administration, administration through a subcutaneously implanted device, and administration is through a cannula.
Comparative Embodiment 168: The use of any one of embodiments 110-167, wherein said mammal is a human.
Comparative Embodiment 169: The use of any one of embodiments 110-167, wherein said mammal is non-human mammal.

### DEFINITIONS

The methods and techniques of the present disclosure are generally performed, unless otherwise indicated, according to conventional methods well known in the art and as described in various general and more specific references that are cited and discussed throughout this specification. See, *e.g*. "Principles of Neural Science", McGraw-Hill Medical, New York, N.Y. (2000); Motulsky, "Intuitive Biostatistics", Oxford University Press, Inc. (1995); Lodish et al., "Molecular Cell Biology, 4th ed.", W. H. Freeman & Co., New York (2000); Griffiths et al., "Introduction to Genetic Analysis, 7th ed.", W. H. Freeman & Co., N.Y. (1999); and Gilbert et al., "Developmental Biology, 6th ed.", Sinauer Associates, Inc., Sunderland, MA (2000).

The terms "disrupting protein aggregation" or "inhibiting protein aggregation" refer to the slowing, or stopping, or preventing of aggregation of a protein *(e.g.,* Aβ, α-synuclein, *etc*.), or causing already aggregated protein(s) to disaggregate *(e.g.,* as characterized by a reduction in size, or opacity, or mass of aggregated protein plaques).

Chemistry terms used herein, unless otherwise defined herein, are used according to conventional usage in the art, as exemplified by "The McGraw-Hill Dictionary of Chemical Terms", Parker S., Ed., McGraw-Hill, San Francisco, C.A. (1985).

The term "agent" is used herein to denote a chemical compound (such as an organic or inorganic compound, a mixture of chemical compounds), a biological macromolecule (such as a nucleic acid, an antibody, including parts thereof as well as humanized, chimeric and human antibodies and monoclonal antibodies, a protein or portion thereof, *e.g.,* a peptide, a lipid, a carbohydrate), or an extract made from biological materials such as bacteria, plants, fungi, or animal (particularly mammalian) cells or tissues. Agents include, for example, agents whose structure is known, and those whose structure is not known.

Molecular tweezers, are host molecules with open cavities capable of binding guest molecules (*see, e.g.,* Hardouin-Lerouge et al. (2011) Chem. Soc. Rev. 40: 30-43). The open cavity of the molecular tweezers may bind guests using non-covalent bonding which can include one or more of hydrogen bonding, metal coordination, hydrophobic forces, van der Waals forces, π-π interactions, and/or electrostatic effects. These complexes can be viewed as a subset of macrocyclic molecular receptors and their typical structure is characterized by two "arms" that bind the guest molecule between them and are only connected at one end leading to a certain flexibility of these molecules (induced fit model).

The term "lipofuscin-related disorder" refers to a pathology in which lipofuscin aggregates and/or where the amount of lipofuscin aggregations is greater than in a normal healthy mammal of the same gender, age, and species. Illustrative lipofuscin-related disorders include but are not limited to lipofuscin-related disorders associated with an eye disease (*e.g*., age-related macular degeneration, vitelliform macular degeneration (Best's macular dystrophy), retinal pigment epitheliopathy associated with choroidal melanoma, severe ocular trauma; and the like), a neuronal ceroid lipofuscinosis (*e.g*. Batten disease), acromegaly, amyotrophic lateral sclerosis, denervation atrophy, lipid myopathy, chronic obstructive pulmonary disease, centronuclear myopathy, melanosis coli, and the like. In certain embodiments "lipofuscin-related-disorders" expressly exclude Alzheimer's disease, and/or Parkinson's Diseases, and/or amyotrophic lateral sclerosis (ALS). In certain embodiments lipofuscin-related disorders comprises pathologies in which lipofuscin has a pathogenic role.

The terms "subject," "individual," and "patient" may be used interchangeably and refer to humans, the as well as non-human mammals (*e.g*., non-human primates, canines, equines, felines, porcines, bovines, ungulates, lagomorphs, and the like). In various embodiments, the subject can be a human (*e.g*., adult male, adult female, adolescent male, adolescent female, male child, female child) under the care of a physician or other health worker in a hospital, as an outpatient, or other clinical context. In certain embodiments, the subject may not be under the care or prescription of a physician or other health worker.

"Treating" a condition or a patient refers to taking steps to obtain beneficial or desired results, including clinical results. As used herein, and as well understood in the art, "treatment" is an approach for obtaining beneficial or desired results, including clinical results. Beneficial or desired clinical results can include, but are not limited to, alleviation or amelioration of one or more symptoms or conditions, diminishment of extent of disease, stabilized (*i.e.* not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

The term "preventing" is art-recognized, and when used in relation to a condition, such as a local recurrence (*e.g*., pain), a disease such as cancer, a syndrome complex such as heart failure or any other medical condition, is well understood in the art, and includes administration of a composition which reduces the frequency of, or delays the onset of, symptoms of a medical condition in a subject relative to a subject which does not receive the composition. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, *e.g.,* by a statistically and/or clinically significant amount.

"Administering" or "administration of" a substance, a compound or an agent to a subject can be carried out using one of a variety of uses known to those skilled in the art. For example, a compound or an agent can be administered, intravenously, arterially, intradermally, intramuscularly, intraperitoneally, subcutaneously, ocularly, sublingually, orally (by ingestion), intranasally (by inhalation), intraspinally, intracerebrally, and transdermally (by absorption, *e.g*., through a skin duct). A compound or agent can also appropriately be introduced by rechargeable or biodegradable polymeric devices or other devices, *e.g.*, patches and pumps, or formulations, which provide for the extended, slow or controlled release of the compound or agent. Administering can also be performed, for example, once, a plurality of times, and/or over one or more extended periods. Administering, as used herein includes "causing to be administered". Thus, for example, prescribing a pharmaceutical for a subject/patient where the patient actually self-administers the pharmaceutical is considered "administering" by the prescribing agent/agency.

Appropriate uses of administering a substance, a compound or an agent to a subject will also depend, for example, on the age and/or the physical condition of the subject and the chemical and biological properties of the compound or agent *(e.g.,* solubility, digestibility, bioavailability, stability and toxicity). In some embodiments, a compound or an agent is administered orally, *e.g.,* to a subject by ingestion. In some embodiments, the orally administered compound or agent is in an extended release or slow release formulation, or administered using a device for such slow or extended release.

As used herein, the phrase "conjoint administration" refers to any form of administration of two or more different therapeutic agents such that the second agent is administered while the previously administered therapeutic agent is still effective in the body *(e.g.,* the two agents are simultaneously effective in the patient, which may include synergistic effects of the two agents). For example, the different therapeutic compounds can be administered either in the same formulation or in separate formulations, either concomitantly or sequentially. Thus, an individual who receives such treatment can benefit from a combined effect of different therapeutic agents.

A "therapeutically effective amount" or a "therapeutically effective dose" of a drug or agent is an amount of a drug or an agent that, when administered to a subject will have the intended therapeutic effect. The full therapeutic effect does not necessarily occur by administration of one dose, and may occur only after administration of a series of doses. Thus, a therapeutically effective amount may be administered in one or more administrations. The precise effective amount needed for a subject will depend upon, for example, the subject's size, health and age, and the nature and extent of the condition being treated, such as cancer or MDS. The skilled worker can readily determine the effective amount for a given situation by routine experimentation.

The term "acetal" is art-recognized and may be represented by the general formula 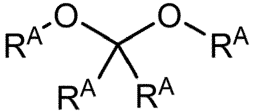 wherein each R^{A} independently represents hydrogen or a hydrocarbyl, such as alkyl, or any occurrence of R^{A} taken together with another and the intervening atom(s) complete a carbocycle or heterocycle having from 4 to 8 atoms in the ring structure.

The term "acyl" is art-recognized and refers to a group represented by the general formula hydrocarbylC(O)-, preferably alkylC(O)-.

The term "acylamino" is art-recognized and refers to an amino group substituted with an acyl group and may be represented, for example, by the formula hydrocarbylC(O)NH-.

The term "acyloxy" is art-recognized and refers to a group represented by the general formula hydrocarbylC(O)O-, preferably alkylC(O)O-.

The term "alkoxy" refers to an alkyl group, preferably a lower alkyl group, having an oxygen attached thereto. Representative alkoxy groups include methoxy, trifluoromethoxy, ethoxy, propoxy, tert-butoxy and the like.

The term "alkoxyalkyl" refers to an alkyl group substituted with an alkoxy group and may be represented by the general formula alkyl-O-alkyl.

The term "alkenyl", as used herein, refers to an aliphatic group containing at least one double bond and is intended to include both "unsubstituted alkenyls" and "substituted alkenyls", the latter of which refers to alkenyl moieties having substituents replacing a hydrogen on one or more carbons of the alkenyl group. Such substituents may occur on one or more carbons that are included or not included in one or more double bonds. Moreover, such substituents include all those contemplated for alkyl groups, as discussed below, except where stability is prohibitive. For example, substitution of alkenyl groups by one or more alkyl, carbocyclyl, aryl, heterocyclyl, or heteroaryl groups is contemplated.

An "alkyl" group or "alkane" is a straight chained or branched non-aromatic hydrocarbon which is completely saturated. Typically, a straight chained or branched alkyl group has from 1 to about 20 carbon atoms, preferably from 1 to about 10 unless otherwise defined. Examples of straight chained and branched alkyl groups include methyl, ethyl, n-propyl, iso-propyl, n-butyl, sec-butyl, tert-butyl, pentyl, hexyl, pentyl and octyl. A C₁-C₆ straight chained or branched alkyl group is also referred to as a "lower alkyl" group.

Moreover, the term "alkyl" (or "lower alkyl") as used throughout the specification, examples, and claims is intended to include both "unsubstituted alkyls" and "substituted alkyls", the latter of which refers to alkyl moieties having substituents replacing a hydrogen on one or more carbons of the hydrocarbon backbone. Such substituents, if not otherwise specified, can include, for example, a halogen *(e.g.,* fluoro), a hydroxyl, a carbonyl (such as a carboxyl, an alkoxycarbonyl, a formyl, or an acyl), a thiocarbonyl (such as a thioester, a thioacetate, or a thioformate), an alkoxy, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. In preferred embodiments, the substituents on substituted alkyls are selected from C₁₋₆ alkyl, C₃₋₆ cycloalkyl, halogen, carbonyl, cyano, or hydroxyl. In more preferred embodiments, the substituents on substituted alkyls are selected from fluoro, carbonyl, cyano, or hydroxyl. It will be understood by those skilled in the art that the moieties substituted on the hydrocarbon chain can themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), -CF₃, - CN and the like. Exemplary substituted alkyls are described below. Cycloalkyls can be further substituted with alkyls, alkenyls, alkoxys, alkylthios, aminoalkyls, carbonylsubstituted alkyls, -CF₃, -CN, and the like.

The term "C_{x-y}" when used in conjunction with a chemical moiety, such as, acyl, acyloxy, alkyl, alkenyl, alkynyl, or alkoxy is meant to include groups that contain from x to y carbons in the chain. For example, the term "C_{x-y} alkyl" refers to substituted or unsubstituted saturated hydrocarbon groups, including straight-chain alkyl and branchedchain alkyl groups that contain from x to y carbons in the chain, including haloalkyl groups. Preferred haloalkyl groups include trifluoromethyl, difluoromethyl, 2,2,2-trifluoroethyl, and pentafluoroethyl. C₀ alkyl indicates a hydrogen where the group is in a terminal position, a bond if internal. The terms "C_{2-y} alkenyl" and "C_{2-y} alkynyl" refer to substituted or unsubstituted unsaturated aliphatic groups analogous in length and possible substitution to the alkyls described above, but that contain at least one double or triple bond respectively.

The term "alkylamino", as used herein, refers to an amino group substituted with at least one alkyl group.

The term "alkylthio", as used herein, refers to a thiol group substituted with an alkyl group and may be represented by the general formula alkylS-.

The term "alkynyl", as used herein, refers to an aliphatic group containing at least one triple bond and is intended to include both "unsubstituted alkynyls" and "substituted alkynyls", the latter of which refers to alkynyl moieties having substituents replacing a hydrogen on one or more carbons of the alkynyl group. Such substituents may occur on one or more carbons that are included or not included in one or more triple bonds. Moreover, such substituents include all those contemplated for alkyl groups, as discussed above, except where stability is prohibitive. For example, substitution of alkynyl groups by one or more alkyl, carbocyclyl, aryl, heterocyclyl, or heteroaryl groups is contemplated.

The term "amide", as used herein, refers to a group 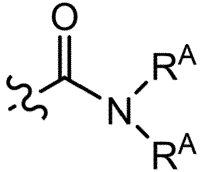 wherein each R^{A} independently represent a hydrogen or hydrocarbyl group, or two R^{A} are taken together with the N atom to which they are attached complete a heterocycle having from 4 to 8 atoms in the ring structure.

The terms "amine" and "amino" are art-recognized and refer to both unsubstituted and substituted amines and salts thereof, *e.g.,* a moiety that can be represented by 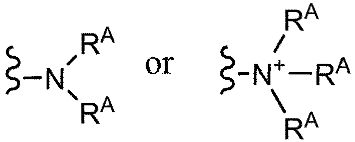 wherein each R^{A} independently represents a hydrogen or a hydrocarbyl group, or two R^{A} are taken together with the N atom to which they are attached complete a heterocycle having from 4 to 8 atoms in the ring structure.

The term "aminoalkyl", as used herein, refers to an alkyl group substituted with an amino group.

The term "aralkyl", as used herein, refers to an alkyl group substituted with an aryl group.

The term "aryl" as used herein include substituted or unsubstituted singlering aromatic groups in which each atom of the ring is carbon. Preferably the ring is a 6- or 10-membered ring, more preferably a 6-membered ring. The term "aryl" also includes polycyclic ring systems having two or more cyclic rings in which two or more carbons are common to two adjoining rings wherein at least one of the rings is aromatic, *e.g*., the other cyclic rings can be cycloalkyls, cycloalkenyls, cycloalkynyls, aryls, heteroaryls, and/or heterocyclyls. Aryl groups include benzene, naphthalene, phenanthrene, phenol, aniline, and the like.

The term "boron" as used herein with respect to a substituent on an organic compound, is art-recognized and refers to a group -B(R^{A})₂, wherein each R^{A} independently represents hydrogen or a hydrocarbyl, such as alkyl, or any occurrence of R^{A} taken together with another and the intervening atom(s) complete a heterocycle having from 4 to 8 atoms in the ring structure.

The term "boronic ester" or "boronate ester" as used herein is art-recognized and refers to a group -B(OR^{A})₂, wherein each R^{A} independently represents hydrogen or a hydrocarbyl, such as alkyl, or any occurrence of R^{A} taken together with another and the intervening atom(s) complete a heterocycle having from 4 to 8 atoms in the ring structure.

The term "carbamate" is art-recognized and refers to a group 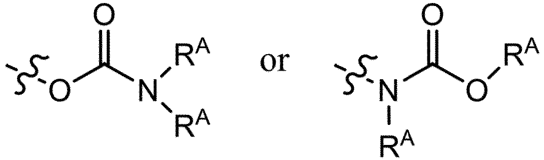 wherein each R^{A} independently represent hydrogen or a hydrocarbyl group, such as an alkyl group, or both R^{A} taken together with the intervening atom(s) complete a heterocycle having from 4 to 8 atoms in the ring structure.

The terms "carbocycle", and "carbocyclic", as used herein, refers to a saturated or unsaturated ring in which each atom of the ring is carbon. The term carbocycle includes both aromatic carbocycles and non-aromatic carbocycles. Non-aromatic carbocycles include both cycloalkane rings, in which all carbon atoms are saturated, and cycloalkene rings, which contain at least one double bond. "Carbocycle" includes 5-7 membered monocyclic and 8-12 membered bicyclic rings. Each ring of a bicyclic carbocycle may be selected from saturated, unsaturated and aromatic rings. Carbocycle includes bicyclic molecules in which one, two or three or more atoms are shared between the two rings. The term "fused carbocycle" refers to a bicyclic carbocycle in which each of the rings shares two adjacent atoms with the other ring. Each ring of a fused carbocycle may be selected from saturated, unsaturated and aromatic rings. In an exemplary embodiment, an aromatic ring, *e.g.,* phenyl, may be fused to a saturated or unsaturated ring, *e.g.,* cyclohexane, cyclopentane, or cyclohexene. Any combination of saturated, unsaturated and aromatic bicyclic rings, as valence permits, is included in the definition of carbocyclic. Exemplary "carbocycles" include cyclopentane, cyclohexane, bicyclo[2.2.1]heptane, 1,5-cyclooctadiene, 1,2,3,4-tetrahydronaphthalene, bicyclo[4.2.0]oct-3-ene, naphthalene and adamantane. Exemplary fused carbocycles include decalin, naphthalene, 1,2,3,4-tetrahydronaphthalene, bicyclo[4.2.0]octane, 4,5,6,7-tetrahydro-1H-indene and bicyclo[4.1.0]hept-3-ene. "Carbocycles" may be substituted at any one or more positions capable of bearing a hydrogen atom.

A "cycloalkyl" group is a cyclic hydrocarbon which is completely saturated. "Cycloalkyl" includes monocyclic and bicyclic rings. Typically, a monocyclic cycloalkyl group has from 3 to about 10 carbon atoms, more typically 3 to 8 carbon atoms unless otherwise defined. The second ring of a bicyclic cycloalkyl may be selected from saturated, unsaturated and aromatic rings. Cycloalkyl includes bicyclic molecules in which one, two or three or more atoms are shared between the two rings. The term "fused cycloalkyl" refers to a bicyclic cycloalkyl in which each of the rings shares two adjacent atoms with the other ring. The second ring of a fused bicyclic cycloalkyl may be selected from saturated, unsaturated and aromatic rings. A "cycloalkenyl" group is a cyclic hydrocarbon containing one or more double bonds.

The term "carbocyclylalkyl", as used herein, refers to an alkyl group substituted with a carbocycle group.

The term "carbonate" is art-recognized and refers to a group -OCO₂-R^{A}, wherein R^{A} represents a hydrocarbyl group.

The term "carboxy", as used herein, refers to a group represented by the formula -CO₂H.

The term "diazo", as used herein, refers to a group represented by the formula =N=N.

The term "disulfide" is art-recognized and refers to a group -S-S-R^{A}, wherein R^{A} represents a hydrocarbyl group.

The term "enol ester", as used herein, refers to a group -C(O)O-C(R^{A})=C(R^{A})₂ wherein R^{A} represents a hydrocarbyl group.

The term "ester", as used herein, refers to a group -C(O)OR^{A} wherein R^{A} represents a hydrocarbyl group.

The term "ether", as used herein, refers to a hydrocarbyl group linked through an oxygen to another hydrocarbyl group. Accordingly, an ether substituent of a hydrocarbyl group may be hydrocarbyl-O-. Ethers may be either symmetrical or unsymmetrical. Examples of ethers include, but are not limited to, heterocycle-O-heterocycle and aryl-O-heterocycle. Ethers include "alkoxyalkyl" groups, which may be represented by the general formula alkyl-O-alkyl.

The terms "halo" and "halogen" as used herein means halogen and includes chloro, fluoro, bromo, and iodo.

The terms "hetaralkyl" and "heteroaralkyl", as used herein, refers to an alkyl group substituted with a hetaryl group.

The term "heteroalkyl", as used herein, refers to a saturated or unsaturated chain of carbon atoms and at least one heteroatom, wherein no two heteroatoms are adjacent.

The terms "heteroaryl" and "hetaryl" include substituted or unsubstituted aromatic single ring structures, preferably 5- to 7-membered rings, more preferably 5- to 6-membered rings, whose ring structures include at least one heteroatom, preferably one to four heteroatoms, more preferably one or two heteroatoms. The terms "heteroaryl" and "hetaryl" also include polycyclic ring systems having two or more cyclic rings in which two or more carbons are common to two adjoining rings wherein at least one of the rings is heteroaromatic, *e.g.*, the other cyclic rings can be cycloalkyls, cycloalkenyls, cycloalkynyls, aryls, heteroaryls, and/or heterocyclyls. Heteroaryl groups include, for example, pyrrole, furan, thiophene, imidazole, oxazole, thiazole, pyrazole, pyridine, pyrazine, pyridazine, and pyrimidine, and the like.

The term "heteroatom" as used herein means an atom of any element other than carbon or hydrogen. Preferred heteroatoms are nitrogen, oxygen, and sulfur.

The terms "heterocyclyl", "heterocycle", and "heterocyclic" refer to substituted or unsubstituted non-aromatic ring structures, preferably 3- to 10-membered rings, more preferably 3- to 7-membered rings, whose ring structures include at least one heteroatom, preferably one to four heteroatoms, more preferably one or two heteroatoms. The terms "heterocyclyl" and "heterocyclic" also include polycyclic ring systems having two or more cyclic rings in which two or more carbons are common to two adjoining rings wherein at least one of the rings is heterocyclic, *e.g.*, the other cyclic rings can be cycloalkyls, cycloalkenyls, cycloalkynyls, aryls, heteroaryls, and/or heterocyclyls. Heterocyclyl groups include, for example, piperidine, piperazine, pyrrolidine, tetrahydropyran, tetrahydrofuran, morpholine, lactones, lactams, and the like.

The term "heterocyclylalkyl", as used herein, refers to an alkyl group substituted with a heterocycle group.

The term "hydrocarbyl", as used herein, refers to a group that is bonded through a carbon atom that does not have a =O or =S substituent, and typically has at least one carbon-hydrogen bond and a primarily carbon backbone, but may optionally include heteroatoms. Thus, groups like methyl, ethoxyethyl, 2-pyridyl, and trifluoromethyl are considered to be hydrocarbyl for the purposes of this application, but substituents such as acetyl (which has a =O substituent on the linking carbon) and ethoxy (which is linked through oxygen, not carbon) are not. Hydrocarbyl groups include, but are not limited to aryl, heteroaryl, carbocycle, heterocyclyl, alkyl, alkenyl, alkynyl, and combinations thereof.

The term "hydroxyalkyl", as used herein, refers to an alkyl group substituted with a hydroxy group.

The term "lower" when used in conjunction with a chemical moiety, such as, acyl, acyloxy, alkyl, alkenyl, alkynyl, or alkoxy is meant to include groups where there are ten or fewer non-hydrogen atoms in the substituent, preferably six or fewer. A "lower alkyl", for example, refers to an alkyl group that contains ten or fewer carbon atoms, preferably six or fewer. In certain embodiments, acyl, acyloxy, alkyl, alkenyl, alkynyl, or alkoxy substituents defined herein are respectively lower acyl, lower acyloxy, lower alkyl, lower alkenyl, lower alkynyl, or lower alkoxy, whether they appear alone or in combination with other substituents, such as in the recitations hydroxyalkyl and aralkyl (in which case, for example, the atoms within the aryl group are not counted when counting the carbon atoms in the alkyl substituent).

The term "orthoester" as used herein is art-recognized and refers to a group - C(OR^{A})₃, wherein each R^{A} independently represents hydrogen or a hydrocarbyl, such as alkyl, or any occurrence of R^{A} taken together with another and the intervening atom(s) complete a heterocycle having from 4 to 8 atoms in the ring structure.

The term "phosphoester", as used herein, refers to a group -P(O₂)OH.

The term "phosphodiester", as used herein, refers to a group -P(O₂)OR^{A} wherein R^{A} represents a hydrocarbyl group.

The terms "polycyclyl", "polycycle", and "polycyclic" refer to two or more rings (e.g., cycloalkyls, cycloalkenyls, cycloalkynyls, aryls, heteroaryls, and/or heterocyclyls) in which two or more atoms are common to two adjoining rings, *e.g.,* the rings are "fused rings". Each of the rings of the polycycle can be substituted or unsubstituted. In certain embodiments, each ring of the polycycle contains from 3 to 10 atoms in the ring, preferably from 5 to 7.

The term "selenide", as used herein, is equivalent to an ether, wherein the oxygen is replaced with a selenium.

The term "selenoxide" is art-recognized and refers to the group -Se(O)-R^{A}, wherein R^{A} represents a hydrocarbyl.

The term "siloxane" is art-recognized and refers to a group with an Si-O-Si linkage, such as the group -Si(R^{A})₂-O-Si-(R^{A})₃, wherein each R^{A} independently represents hydrogen or hydrocarbyl, such as alkyl, or both R^{A} taken together with the intervening atom(s) complete a heterocycle having from 4 to 8 atoms in the ring structure.

The term "silyl" refers to a silicon moiety with three hydrocarbyl moieties attached thereto.

The term "substituted" refers to moieties having substituents replacing a hydrogen on one or more carbons of the backbone. It will be understood that "substitution" or "substituted with" includes the implicit proviso that such substitution is in accordance with permitted valence of the substituted atom and the substituent, and that the substitution results in a stable compound, *e.g*., which does not spontaneously undergo transformation such as by rearrangement, cyclization, elimination, *etc.* As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic substituents of organic compounds. The permissible substituents can be one or more and the same or different for appropriate organic compounds. For purposes of this invention, the heteroatoms such as nitrogen may have hydrogen substituents and/or any permissible substituents of organic compounds described herein which satisfy the valences of the heteroatoms. Substituents can include any substituents described herein, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxy, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. In preferred embodiments, the substituents on substituted alkyls are selected from C₁₋₆ alkyl, C₃₋₆ cycloalkyl, halogen, carbonyl, cyano, or hydroxyl. In more preferred embodiments, the substituents on substituted alkyls are selected from fluoro, carbonyl, cyano, or hydroxyl. It will be understood by those skilled in the art that substituents can themselves be substituted, if appropriate. Unless specifically stated as "unsubstituted," references to chemical moieties herein are understood to include substituted variants. For example, reference to an "aryl" group or moiety implicitly includes both substituted and unsubstituted variants.

The term "sulfate" is art-recognized and refers to the group -OSO₃H, or a pharmaceutically acceptable salt thereof.

The term "sulfonamide" is art-recognized and refers to the group represented by the general formulae 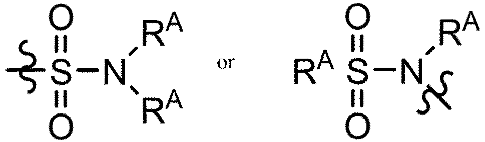 wherein each R^{A} independently represents hydrogen or hydrocarbyl, such as alkyl, or both R^{A} taken together with the intervening atom(s) complete a heterocycle having from 4 to 8 atoms in the ring structure.

The term "sulfoxide" is art-recognized and refers to the group -S(O)-R^{A}, wherein R^{A} represents a hydrocarbyl.

The term "sulfonate" is art-recognized and refers to the group SO₃H, or a pharmaceutically acceptable salt thereof.

The term "sulfone" is art-recognized and refers to the group -S(O)₂-R^{A}, wherein R^{A} represents a hydrocarbyl.

The term "thioalkyl", as used herein, refers to an alkyl group substituted with a thiol group.

The term "thioester", as used herein, refers to a group -C(O)SR^{A} or -SC(O)R^{A} wherein R^{A} represents a hydrocarbyl.

The term "thioether", as used herein, is equivalent to an ether, wherein the oxygen is replaced with a sulfur.

The term "urea" is art-recognized and may be represented by the general formula 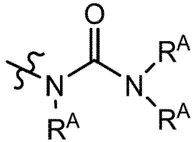 wherein each R^{A} independently represents hydrogen or a hydrocarbyl, such as alkyl, or any occurrence of R^{A} taken together with another and the intervening atom(s) complete a heterocycle having from 4 to 8 atoms in the ring structure.

"Protecting group" refers to a group of atoms that, when attached to a reactive functional group in a molecule, mask, reduce or prevent the reactivity of the functional group. Typically, a protecting group may be selectively removed as desired during the course of a synthesis. Examples of protecting groups can be found in Greene and Wuts, Protective Groups in Organic Chemistry, 3rd Ed., 1999, John Wiley & Sons, NY and Harrison et al., Compendium of Synthetic Organic Uses, Vols. 1-8, 1971-1996, John Wiley & Sons, NY. Representative nitrogen protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilyl-ethanesulfonyl ("TES"), trityl and substituted trityl groups, allyloxycarbonyl, 9-fluorenylmethyloxycarbonyl ("FMOC"), nitroveratryloxycarbonyl ("NVOC") and the like. Representative hydroxyl protecting groups include, but are not limited to, those where the hydroxyl group is either acylated (esterified) or alkylated such as benzyl and trityl ethers, as well as alkyl ethers, tetrahydropyranyl ethers, trialkylsilyl ethers *(e.g.,* TMS or TIPS groups), glycol ethers, such as ethylene glycol and propylene glycol derivatives and allyl ethers.

The term "modulate" as used herein includes the inhibition or suppression of a function or activity (such as cell proliferation) as well as the enhancement of a function or activity.

The phrase "pharmaceutically acceptable" is art-recognized. In certain embodiments, the term includes compositions, excipients, adjuvants, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable salt" or "salt" is used herein to refer to an acid addition salt or a basic addition salt which is suitable for or compatible with the treatment of patients.

The term "pharmaceutically acceptable acid addition salt" as used herein means any non-toxic organic or inorganic salt of any base compounds represented by Formula I. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, citric, ascorbic, maleic, benzoic, phenylacetic, cinnamic and salicylic acids, as well as sulfonic acids such as p-toluene sulfonic and methanesulfonic acids. Either the mono or di-acid salts can be formed, and such salts may exist in either a hydrated, solvated or substantially anhydrous form. In general, the acid addition salts of compounds of Formula I are more soluble in water and various hydrophilic organic solvents, and generally demonstrate higher melting points in comparison to their free base forms. The selection of the appropriate salt will be known to one skilled in the art. Other non-pharmaceutically acceptable salts, *e.g*., oxalates, may be used, for example, in the isolation of compounds of Formula I for laboratory use, or for subsequent conversion to a pharmaceutically acceptable acid addition salt.

The term "pharmaceutically acceptable basic addition salt" as used herein means any non-toxic organic or inorganic base addition salt of any acid compounds represented by Formula I or any of their intermediates. Illustrative inorganic bases which form suitable salts include lithium, sodium, potassium, calcium, magnesium, or barium hydroxide. Illustrative organic bases which form suitable salts include aliphatic, alicyclic, or aromatic organic amines such as methylamine, trimethylamine and picoline or ammonia. The selection of the appropriate salt will be known to a person skilled in the art.

Many of the compounds useful in the uses and compositions of this disclosure have at least one stereogenic center in their structure. This stereogenic center may be present in a R or a S configuration, said R and S notation is used in correspondence with the rules described in Pure Appl. Chem. (1976), 45, 11-30. The disclosure contemplates all stereoisomeric forms such as enantiomeric and diastereoisomeric forms of the compounds, salts, prodrugs or mixtures thereof (including all possible mixtures of stereoisomers). See, *e.g.,* WO 01/062726. Furthermore, certain compounds which contain alkenyl groups may exist as Z (zusammen) or E (entgegen) isomers. In each instance, the disclosure includes both mixtures and separate individual isomers.

Some of the compounds may also exist in tautomeric forms. Such forms, although not explicitly indicated in the formulae described herein, are intended to be included within the scope of the present disclosure.

"Prodrug" or "pharmaceutically acceptable prodrug" refers to a compound that is metabolized, for example hydrolyzed or oxidized, in the host after administration to form the compound of the present disclosure (*e.g*., compounds of formula I). Typical examples of prodrugs include compounds that have biologically labile or cleavable (protecting) groups on a functional moiety of the active compound. Prodrugs include compounds that can be oxidized, reduced, aminated, deaminated, hydroxylated, dehydroxylated, hydrolyzed, dehydrolyzed, alkylated, dealkylated, acylated, deacylated, phosphorylated, or dephosphorylated to produce the active compound. Examples of prodrugs using ester or phosphoramidate as biologically labile or cleavable (protecting) groups are disclosed in U.S. Patents 6,875,751, 7,585,851, and 7,964,580e. The prodrugs of this disclosure are metabolized to produce a compound of Formula I. The present disclosure includes within its scope, prodrugs of the compounds described herein. Conventional procedures for the selection and preparation of suitable prodrugs are described, for example, in "Design of Prodrugs" Ed. H. Bundgaard, Elsevier, 1985.

The phrase "pharmaceutically acceptable carrier" as used herein means a pharmaceutically acceptable material, composition or vehicle, such as a liquid or solid filter, diluent, excipient, solvent or encapsulating material useful for formulating a drug for medicinal or therapeutic use.

The term "Log of solubility", "LogS" or "logS" as used herein is used in the art to quantify the aqueous solubility of a compound. The aqueous solubility of a compound significantly affects its absorption and distribution characteristics. A low solubility often goes along with a poor absorption. LogS value is a unit stripped logarithm (base 10) of the solubility measured in mol/liter.

### BRIEF DESCRIPTION OF THE DRAWINGS

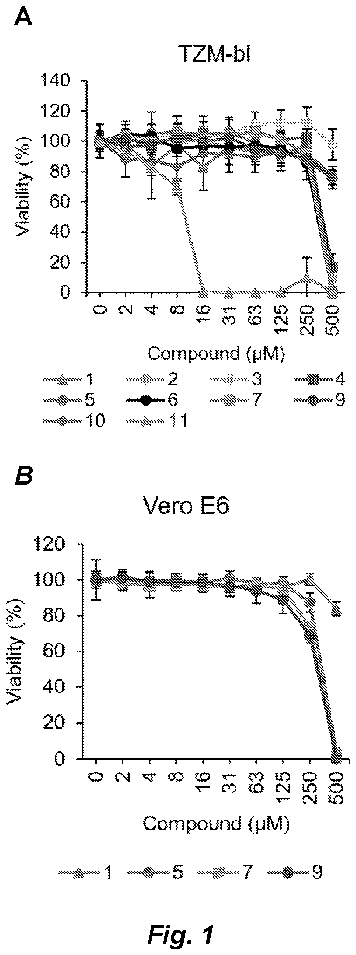

Figure 1, panels A and B, illustrates the results of a cell viability assay with the new molecular tweezers. Indicated concentrations of compounds were added to the cells and incubated for 2 days before CELLTITER-GLO^{®} assay (Promega) was performed on TZM-bl cells (HIV-1 target cells) (panel A), or on Vero E6 (ZIKV target cells) (panel B). The experiment was performed in triplicates, raw data were normalized to untreated cells. Data represent mean ± SD (n=3).

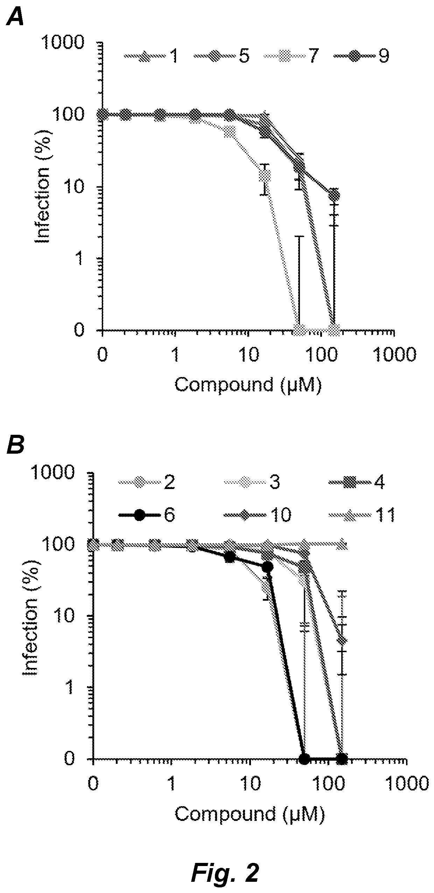

Figure 2, panels A and B, illustrates the results of a ZIKV infectivity (MTT) assay with the new molecular tweezers on Vero E6 cells. ZIKV MR766 (MOI 2) was incubated with indicated concentrations of compounds for 10 min at 37 °C before the mixtures were added to Vero E6 cells. After 3-4 days, the ZIKV induced cytopathic effect was quantified using the MTT assay. Panel A: For compounds **1, 5, 7,** and **9** the assay was performed 3 times in triplicates, data represent means ± SD (n = 9). Panel B: For compounds **2, 3, 4, 6, 10,** and **11** the assay has only been performed once in triplicates, data represent means ± SD (n = 3).

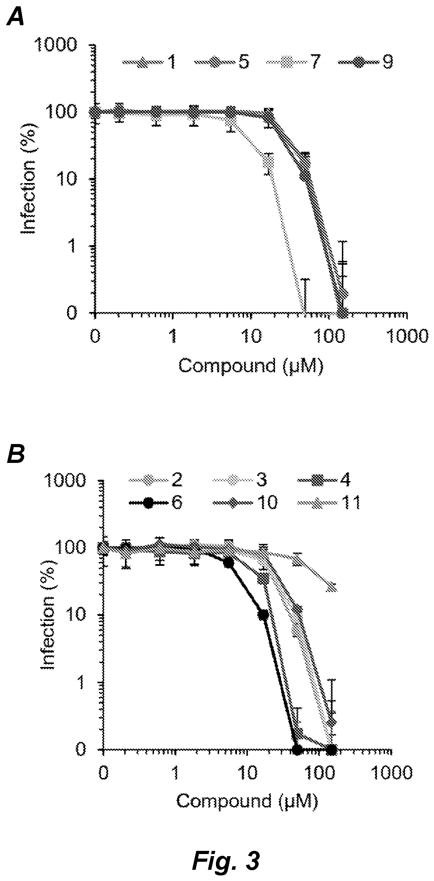

Figure 3, panels A and B, illustrates the results of an HIV-1 infectivity assay with new tweezers on TZM-bl cells. CR5-tropic HIV-1 strain (20 ng/ml p24 antigen) was incubated for 10 min at 37 °C with indicated concentrations of the different compounds before these mixtures were used to infect TZM-bl cells. Three days later, infection rates were determined by measuring β-galactosidase activity. Panel A: For compounds **1, 5, 7** and **9** the assay was performed 3 times in triplicates, data represent means ± SD (n=9). Panel B: For compounds **2, 3, 4, 6, 10,** and **11** the assay has only been performed once in triplicates, data represent means ± SD (n=3).

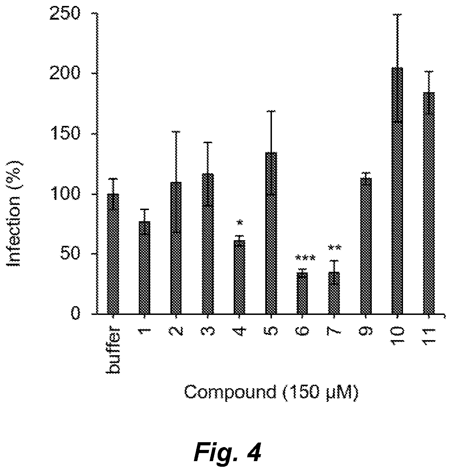

Figure 4 illustrates Anti-HIV-1 activity of the compounds at 150 µM in the presence of 2.5 % human serum. CCR5-tropic HIV-1 NL4-3 strain (20 ng/ml p24 antigen) was incubated with 150 µM of the different compounds and 2.5% of human serum for 10 min at 37°C. Then, the mixtures were added to TZM-bl cells. Three days later, infection rates were determined by measuring β-galactosidase activity. Data indicates mean of triplicate infections ±SD. Unpaired t-tests were used to compare the buffer control to the 150 µM condition of the different compounds (* denotes p < 0.01; ** p < 0.001; *** p < 0.0001).

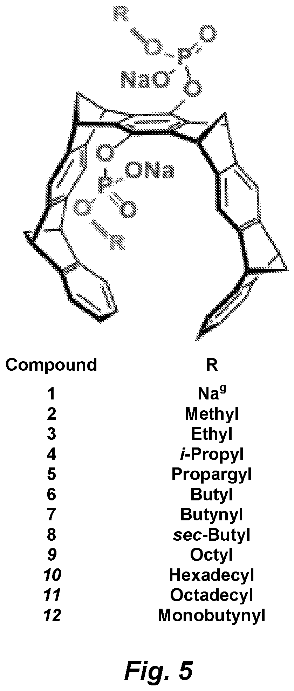

Figure 5 illustrates the structure of compounds 1-12.

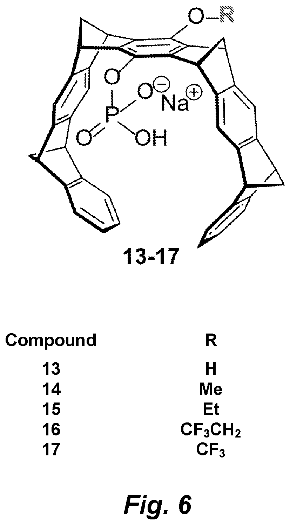

Figure 6 illustrates the structure of compounds 13-17.

### DETAILED DESCRIPTION

In various embodiments new molecular-tweezer compounds, new synthetic and new methods for the preparation of molecular-tweezer compounds are provided. Additionally, new evidence for improved pharmacokinetic characteristics of certain molecular-tweezer derivatives, including improved oral bioavailability and blood-brain barrier penetration, and new evidence of improved anti-viral activity of new moleculartweezers derivatives against enveloped viruses, including, but not limited to Zika virus and HIV is provided.

The compounds described herein (e.g., molecular tweezers) are promising drug candidates for proteinopathies and for viral infection by enveloped viruses. Compared to a previous lead compound, CLR01 (AKA TW1), which is described in U.S. Patent No: 8,791,092), the new derivatives have improved PK characteristics and anti-viral activity.

Molecular tweezers have been reported to be efficient inhibitors of abnormal protein aggregation, which causes, or is part of the pathologic mechanism of proteinopathies, including common neurodegenerative diseases, such as Alzheimer's and Parkinson's, and rare, orphan diseases, such as multiple system atrophy, amyotrophic lateral sclerosis, and familial amyloidotic polyneuropathy. More recently, molecular tweezers also were discovered to be potent inhibitors of viral infection by enveloped viruses, such as HIV, Zika, and Ebola.

In certain embodiments the compounds described herein (*e.g*., molecular tweezers) are believed to be useful for inhibiting protein aggregation (or disaggregating aggregated proteins). In certain embodiments the compounds described herein *(e.g.,* molecular tweezers) are believed to be useful in the treatment of pathologies characterized by protein aggregation (*e.g*., amyloidopathies), and/or in the treatment of brain or spinal cord damage associated with acute trauma, stroke, and the like, and/or in the treatment of lysosomal storage diseases, and/or in the treatment of lipofuscin-related disorders, and in various viral infections.

### New Molecular Tweezers.

In various embodiments new compounds (molecular tweezers are provided) that are useful for inhibiting protein aggregation, for the treatment of various pathologies characterized by protein aggregation, and for inhibiting infectivity of viruses (*e.g*., enveloped viruses). In certain embodiments the compounds comprise a compound of Formula I, II, III, or IV or a pharmaceutically acceptable salt thereof: 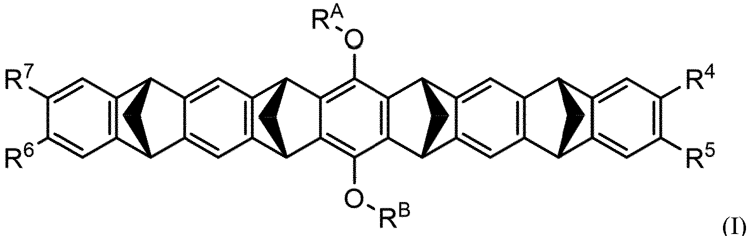 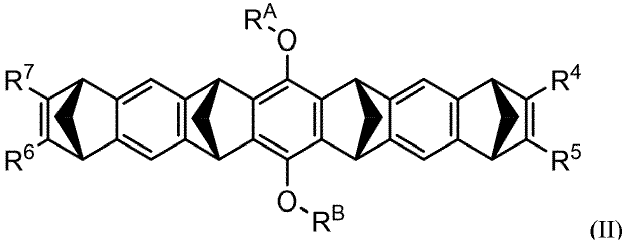 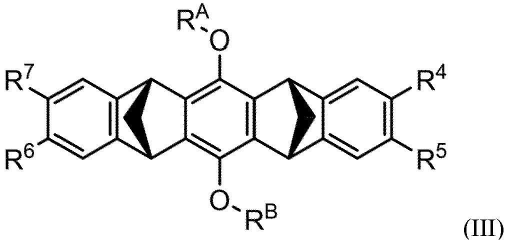 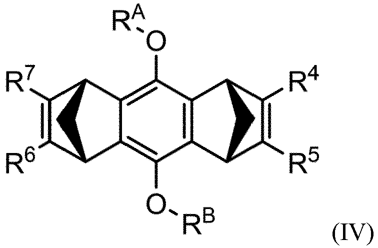 where R^{A} is selected from alkyl, 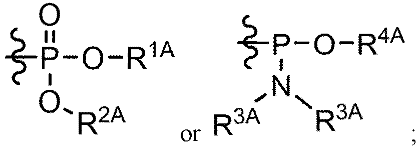 R^{B} is selected from H, acyloxy, 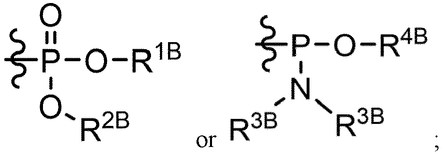 R⁴ and R⁵ are independently selected from H, halo, alkyl, alkenyl, alkynyl, amino, amide, carboxyl, ester, or nitro; or R⁴ and R⁵, together with the atoms that separate them, complete a cycloalkyl, aryl, or heteroaryl; R⁶ and R⁷ are independently selected from H, halo, alkyl, alkenyl, alkynyl, amino, amide, carboxyl, ester, or nitro; or R⁶ and R⁷, together with the atoms that separate them, complete a cycloalkyl, aryl, or heteroaryl; R^{1A}, R^{2A}, R^{1B}, and R^{2B} are independently selected from H, alkyl, alkenyl, or alkynyl; each instance of R^{3A} and R^{3B} is independently selected from alkyl, alkenyl, or alkynyl; and R^{4A} and R^{4B} are each independently selected from alkyl, alkenyl, or alkynyl.

In certain embodiments the compounds (molecular tweezers contemplated herein expressly exclude 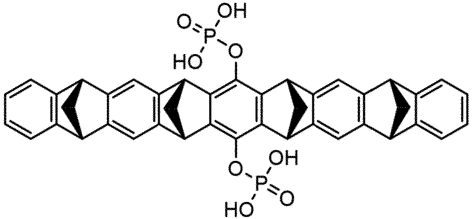 (TW1), 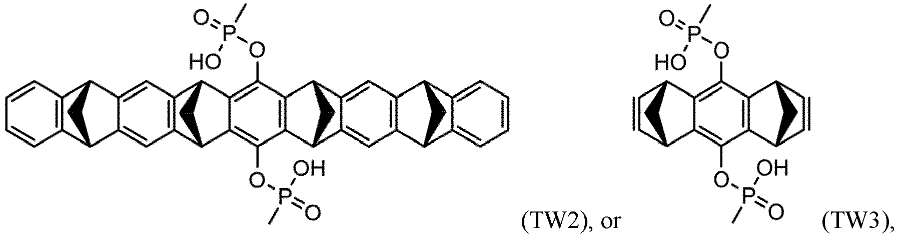 and/or any compound or generic formula described in U.S. Patent No: 8791,092, and/or PCT Publication No: WO 2010/102248. In certain embodiments R^{A} and R^{B} are different. In certain embodiments R^{B} is H or -P(O)(OH)₂. In certain embodiments R^{A} is alkyl. In certain embodiments R^{A} is 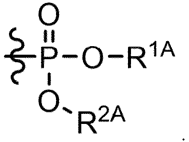 In certain of any of the foregoing embodiments, embodiments R^{1A} can be alkynyl (e.g., but-3-ynyl). In certain of any of the foregoing embodiments, R^{2A} is H or alkyl (e.g., methyl). In certain of any of the foregoing embodiments R^{B} is 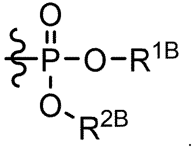 In certain of any of the foregoing embodiments R^{1B} is alkynyl, (*e.g*., but-3-ynyl). In certain of any of the foregoing embodiments R^{1B} is alkyl *(e.g.,* methyl). In certain of any of the foregoing embodiments R^{2B} is H or alkyl, (*e.g.,* methyl). In certain of any of the foregoing embodiments R^{1A}, R^{2A}, R^{1B}, and R^{2B} are independently selected from alkyl, alkenyl, or alkynyl. In certain embodiments of these embodiments, R^{1A}, R^{2A}, R^{1B}, and R^{2B} are the same. In certain of any of the foregoing embodiments R^{1A} and R^{1B} are independently selected from alkyl, alkenyl, or alkynyl, and R^{2A} and R^{2B} are H. In certain of any of the foregoing embodiments R^{1A} is alkyl, alkenyl, or alkynyl; and R^{1B}, R^{2A}, and R^{2B} are H. In certain of any of the foregoing embodiments R^{A} is 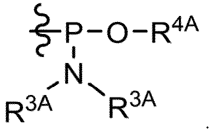 In certain of any of the foregoing embodiments each R^{3A} is alkyl, *(e.g.,* isopropyl). In certain of any of the foregoing embodiments each R^{4A} is alkyl, (*e.g*., 2-cyanoethyl). In certain of any of the foregoing embodiments R^{B} is 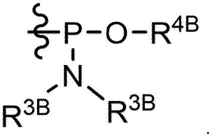 In certain of any of the foregoing embodiments R^{3B} is alkyl, (*e.g*., isopropyl). In certain of any of the foregoing embodiments each R^{4A} is an independently selected alkyl, (*e.g., 2-*cyanoethyl). In certain of any of the foregoing embodiments R^{A} is 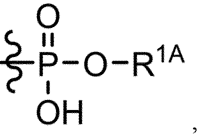 ^{RB} is 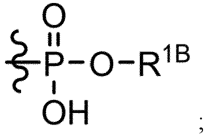 and R^{1A} and R^{1B} are selected from alkyl, alkenyl, or alkynyl, (*e.g*., ethyl, isopropyl, or octyl). In certain of any of the foregoing embodiments R^{A} is 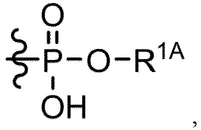 R^{B} is 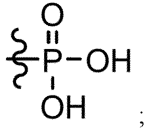 and R^{1A} is alkyl, alkenyl, or alkynyl, (*e.g*., ethyl, isopropyl, or octyl). In certain of any of the foregoing embodiments R^{A} is alkyl, (*e.g.,* methyl, ethyl, trifluoromethyl, or trifluoroethyl, and R^{B} is 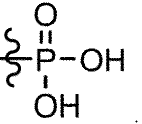 In certain of any of the foregoing embodiments R^{A} is 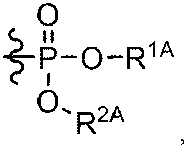 R^{B} is 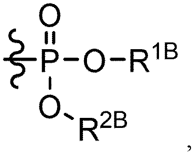 and R^{1A}, R^{1B}, R^{2A} and R^{2B} are selected from alkyl, alkenyl, or alkynyl, (*e.g*., ethyl, isopropyl, or octyl). In certain of any of the foregoing embodiments R⁴ and R⁵ are independently selected from H, halo, alkyl, alkenyl, alkynyl, amino, amide, carboxyl, ester, or nitro. In certain of these embodiments, R⁴ and R⁵ are H. In certain of these embodiments, at least one of R⁴ and R⁵ is halo, alkyl, alkenyl, alkynyl, amino, amide, carboxyl, ester, or nitro. In certain of any of the foregoing embodiments R⁴ and R⁵, together with the atoms that separate them, complete a cycloalkyl, aryl, or heteroaryl. In certain of any of the foregoing embodiments R⁶ and R⁷ are independently selected from H, halo, alkyl, alkenyl, alkynyl, amino, amide, carboxyl, ester, or nitro. In certain of these embodiments, R⁶ and R⁷ are H. In certain of these embodiments, at least one of R⁶ and R⁷ is halo, alkyl, alkenyl, alkynyl, amino, amide, carboxyl, ester, or nitro. In certain of any of the foregoing embodiments R⁶ and R⁷, together with the atoms that separate them, complete a cycloalkyl, aryl, or heteroaryl. In certain embodiments the compound is one of Compounds **2-19** (*see, e.g.,* Figures 5, and 6, **B, D-K,** or **M** (*see, e.g.,* Examples herein).

Methods of making the compounds (*e.g*., molecular tweezers) described herein (*e.g*. Compounds 2-19, D, D-K, M, and the like are provided in Example 1. Using the teachings provided herein, numerous other molecular tweezers will be available to one of skill in the art.

### Pharmaceutical formulations.

In various embodiments pharmaceutical formulations comprising any one or more of the compounds (*e.g*., molecular tweezers) described herein are provided. In certain embodiments the pharmaceutical formulation A pharmaceutical composition (preparation) can be administered to a subject by any of a number of routes of administration including, for example, orally (for example, drenches as in aqueous or non-aqueous solutions or suspensions, tablets, capsules (including sprinkle capsules and gelatin capsules), boluses, powders, granules, pastes for application to the tongue); absorption through the oral mucosa (*e.g*., sublingually); subcutaneously; transdermally (for example as a patch applied to the skin); and topically (for example, as a cream, ointment or spray applied to the skin). The compound may also be formulated for inhalation. In certain embodiments, a compound may be simply dissolved or suspended in sterile water. Details of appropriate routes of administration and compositions suitable for same can be found in, for example, U.S. Patent Nos: 8,791,082, 6,110,973, 5,763,493, 5,731,000, 5,541,231, 5,427,798, 5,358,970 and 4,172,896, as well as in patents cited therein.

The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient that can be combined with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association an active compound, such as a compound of the invention, with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules (including sprinkle capsules and gelatin capsules), cachets, pills, tablets, lozenges (using a flavored basis, usually sucrose and acacia or tragacanth), lyophile, powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. Compositions or compounds may also be administered as a bolus, electuary or paste.

To prepare solid dosage forms for oral administration (capsules (including sprinkle capsules and gelatin capsules), tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; (10) complexing agents, such as, modified and unmodified cyclodextrins; and (11) coloring agents. In the case of capsules (including sprinkle capsules and gelatin capsules), tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions, such as dragees, capsules (including sprinkle capsules and gelatin capsules), pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions that can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms useful for oral administration include pharmaceutically acceptable emulsions, lyophiles for reconstitution, microemulsions, solutions, suspensions, syrups and elixirs. In addition to the active ingredient, the liquid dosage forms may contain inert diluents commonly used in the art, such as, for example, water or other solvents, cyclodextrins and derivatives thereof, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Dosage forms for the topical or transdermal administration include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

The ointments, pastes, creams and gels may contain, in addition to an active compound, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an active compound, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the active compound in the proper medium. Absorption enhancers can also be used to increase the flux of the compound across the skin. The rate of such flux can be controlled by either providing a rate controlling membrane or dispersing the compound in a polymer matrix or gel.

The phrases "parenteral administration" and "administered parenterally" as used herein means modes of administration other than enteral and topical administration, usually by injection, and includes, without limitation, intravenous, intraocular (such as intravitreal), intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion. Pharmaceutical compositions suitable for parenteral administration comprise one or more active compounds in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle.

Injectable depot forms are made by forming microencapsulated matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

For use in the methods described herein, active compounds can be given *per se* or as a pharmaceutical composition containing, for example, 0.1 to 99.5% (more preferably, 0.5 to 90%) of active ingredient in combination with a pharmaceutically acceptable carrier.

Methods of introduction may also be provided by rechargeable or biodegradable devices. Various slow release polymeric devices have been developed and tested *in vivo* in recent years for the controlled delivery of drugs, including proteinaceous biopharmaceuticals. A variety of biocompatible polymers (including hydrogels), including both biodegradable and non-degradable polymers, can be used to form an implant for the sustained release of a compound at a particular target site.

Actual dosage levels of the active ingredients in the pharmaceutical compositions may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular compound or combination of compounds employed, or the ester, salt or amide thereof, the route of administration, the time of administration, the rate of excretion of the particular compound(s) being employed, the duration of the treatment, other drugs, compounds and/or materials used in combination with the particular compound(s) employed, the age, sex, weight, condition, general health and prior medical history of the patient being treated, and like factors well known in the medical arts.

A physician or veterinarian having ordinary skill in the art can readily determine and prescribe the therapeutically effective amount of the pharmaceutical composition required. For example, the physician or veterinarian could start doses of the pharmaceutical composition or compound at levels lower than that required in order to achieve the desired therapeutic effect and gradually increase the dosage until the desired effect is achieved. By "therapeutically effective amount" is meant the concentration of a compound that is sufficient to elicit the desired therapeutic effect. It is generally understood that the effective amount of the compound will vary according to the weight, sex, age, and medical history of the subject. Other factors which influence the effective amount may include, but are not limited to, the severity of the patient's condition, the disorder being treated, the stability of the compound, and, if desired, another type of therapeutic agent being administered with the compound of the invention. A larger total dose can be delivered by multiple administrations of the agent. Methods to determine efficacy and dosage are known to those skilled in the art (Isselbacher et al. (1996) Harrison's Principles of Internal Medicine 13 ed., 1814-1882).

In general, a suitable daily dose of an active compound used in the compositions and methods of the invention will be that amount of the compound that is the lowest dose effective to produce a therapeutic effect. Such an effective dose will generally depend upon the factors described above.

If desired, the effective daily dose of the active compound may be administered as one, two, three, four, five, six or more sub-doses administered separately at appropriate intervals throughout the day, optionally, in unit dosage forms. In certain embodiments of the present invention, the active compound may be administered two or three times daily. In preferred embodiments, the active compound will be administered once daily.

The patient receiving this treatment is any animal in need, including primates, in particular humans, and other mammals such as equines, cattle, swine, sheep, cats, and dogs; poultry; and pets in general.

In certain embodiments, compounds (*e.g*., molecular tweezers) described herein may be used alone or conjointly administered with another type of therapeutic agent.

The present disclosure includes the use of pharmaceutically acceptable salts of compounds (*e.g*., molecular tweezers) described herein in the compositions and methods of described herein. In certain embodiments, contemplated salts of the invention include, but are not limited to, alkyl, dialkyl, trialkyl or tetra-alkyl ammonium salts. In certain embodiments, contemplated salts of the invention include, but are not limited to, L-arginine, benenthamine, benzathine, betaine, calcium hydroxide, choline, deanol, diethanolamine, diethylamine, 2-(diethylamino)ethanol, ethanolamine, ethylenediamine, N-methylglucamine, hydrabamine, 1H-imidazole, lithium, L-lysine, magnesium, 4-(2-hydroxyethyl)morpholine, piperazine, potassium, 1-(2-hydroxyethyl)pyrrolidine, sodium, triethanolamine, tromethamine, and zinc salts. In certain embodiments, contemplated salts of the invention include, but are not limited to, Na, Ca, K, Mg, Zn or other metal salts. In certain embodiments, contemplated salts of the invention include, but are not limited to, 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, l-ascorbic acid, l-aspartic acid, benzenesulfonic acid, benzoic acid, (+)-camphoric acid, (+)-camphor-10-sulfonic acid, capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, d-glucoheptonic acid, d-gluconic acid, d-glucuronic acid, glutamic acid, glutaric acid, glycerophosphoric acid, glycolic acid, hippuric acid, hydrobromic acid, hydrochloric acid, isobutyric acid, lactic acid, lactobionic acid, lauric acid, maleic acid, l-malic acid, malonic acid, mandelic acid, methanesulfonic acid , naphthalene-1,5-disulfonic acid, naphthalene-2-sulfonic acid, nicotinic acid, nitric acid, oleic acid, oxalic acid, palmitic acid, pamoic acid, phosphoric acid, proprionic acid, l-pyroglutamic acid, salicylic acid, sebacic acid, stearic acid, succinic acid, sulfuric acid, l-tartaric acid, thiocyanic acid, p-toluenesulfonic acid, trifluoroacetic acid, and undecylenic acid salts.

In certain embodiments, the pharmaceutically acceptable acid addition salts can also exist as various solvates, such as with water, methanol, ethanol, dimethylformamide, and the like. Mixtures of such solvates can also be prepared. The source of such solvate can be from the solvent of crystallization, inherent in the solvent of preparation or crystallization, or adventitious to such solvent.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water-soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal-chelating agents, such as citric acid, ethylenediamine tetraacetic acid (EDTA), sorbitol, tartaric acid, phosphoric acid, and the like.

The foregoing pharmaceutical formulations are illustrative and not limiting. Using the teachings provided herein numerous other pharmaceutical formulations comprising one or more of the compounds (e.g., molecular tweezers) described herein will be readily available to one of skill in the art.

### Molecular tweezers for inhibition of protein aggregation, e.g., in the treatment of amyloid-related diseases.

In certain embodiments molecular tweezers are provided that inhibit protein aggregation. In certain embodiments the molecular tweezers inhibit the aggregation and/or fibril formation of amyloidogenic proteins and/or induces the disaggregation of such proteins.

Accordingly, in certain embodiments a method of disrupting protein aggregation is provided where the method comprise contacting a protein or a protein aggregate with one or more compound(s) (*e.g.*, a molecular tweezers) described herein. In certain embodiments the method comprise a inhibiting protein aggregation in a mammal, and the method comprises administering to the mammal an effective amount of the compound (molecular tweezers). In certain embodiments the method comprises a method of mitigating one or more symptoms of a disease in a mammal characterized by protein aggregation (*e.g*., amyloidosis), where the effective amount is an amount sufficient to partially or fully inhibit aggregation of a protein (*e.g.,* an amyloidogenic protein). In certain embodiments the method slows or stops protein aggregation. In certain embodiments the method induces disaggregation of an aggregated protein.

Thus, in various embodiments, molecular tweezers are provided that are useful in the prevention and treatment of disorders associated with amyloidosis, a pathogenic process of protein or peptide misfolding and aggregation. The amyloid deposits present in these diseases consist of particular peptides or proteins that are characteristic for each of these diseases but regardless of their sequence the amyloid fibrils have a characteristic β-sheet structure and generally share a common aggregation pathway.

In each disease, a specific protein or peptide misfolds and/or oligomerizes to form soluble aggregation intermediates, and adopts β-sheet structure en route to fibril formation ultimately forming insoluble amyloid fibers, plaques or inclusions. These insoluble forms of the aggregated protein or peptide form by the intermolecular association of β-strands into β-sheets. Recent evidence suggests that the soluble amyloid oligomers may be the principal cause of toxicity. Table 1 describes an illustrative, but non-limiting list of amyloid related disorders and the corresponding amyloidogenic proteins involved.

**Table 1. Illustrative amyloid related disorders and the corresponding amyloidogenic proteins involved.**

| **Disease** | **Amyloidogenic Proteins involved** | **References** |
|---|---|---|
| Alzheimer's disease, Mild | Aβ, tau | (1-4) |
| Cognitive Impairment (MCI) | | |
| Cerebral Amyloid Angiopathy (CAA) | | |
| Down's Syndrome | | (5) |
| Age-related macular degeneration | Aβ | (6) |
| Familial Alzheimer's disease | Multiple mutationcontaining variants of Aβ and tau | (7) |
| Finnish hereditary systemic amyloidosis | Gelsolin | (8) |
| Familial Danish Dementia | ADan | (9) |
| Familial British Dementia | ABri | |
| Type 2 diabetes | Islet amyloid polypeptide (amylin) | (10,11) |
| Parkinson's disease | α-synuclein | (12) |
| Dementia with Lewy bodies | | (13) |
| Frontotemporal dementia | Tau | (14) |
| Huntington's disease | Huntingtin | (15) |
| Dentatombral Pallidoluysian Atrophy | Atrophin 1 | (16,17) |
| spinocerebellar ataxia | Ataxin 1-3, TATA boxbinding protein | (18,19) |
| spinal and bulbar muscular atrophy /Kennedy's disease | Androgen receptor | (20) |
| Bovine Spongiform Encephalopathy | Prion (PrP) | (21-23) |
| Scrapie | | |
| Kuru | | |
| Gerstmann-Straussler-Scheinker disease | | |
| Fatal familial insomnia | | |
| Creutzfeldt- Jakob disease | | |
| Dialysis-related amyloidosis | β₂-microglobulin | (24) |
| Secondary systemic amyloidosis | | (25,26) |
| Systemic (reactive) AA amyloidosis | β₂-microglobulin, serum amyloid A | |
| Prostatic amyloidosis | β₂-microglobulin, transthyretin | (27,28) |
| Conjunctival amyloidosis | Unknown deposits | (29) |
| Primary systemic amyloidosis | Insoluble monoclonal immunoglobulin | (30,31) |
| Systemic AL amyloidosis | Immunoglobulin light chain | |
| Immunoglobulin heavy chainassociated amyloidosis | Immunoglobulin heavy chain | (32) |
| Nodular AL amyloidosis/Primary Sjoegren's Syndrome | Antinuclear antibodies | (33) |
| Myeloma-associated amyloidosis | Immunoglobulin | (34) |
| Nodular Glomerulosclerosis | | (35) |
| Chronic inflammatory disease | β₂-microglobulin, serum amyloid A, immunoglobulin | (25,26) |
| Hereditary non-neuropathic systemic amyloidosis | Lysozyme | (36) |
| Familial visceral amyloidosis | | (37) |
| Fibrinogen α-chain amyloidosis | Fibrinogen α-chain | (38) |
| Familial Mediterranean Fever | Serum amyloid A | (39) |
| Hereditary renal amyloidosis | Cystatin C | (40) |
| | Fibrinogen α-chain | (38) |
| | Gelsolin | (8) |
| Senile systemic amyloidosis | Transthyretin | (41-45) |
| Familial Amyloid Polyneuropathy/Corino de Andrade's disease | | |
| Familial Cardia amyloidosis | | |
| Familial oculo-leptomeningel amyloidosis | | |
| Insulin-related amyloidosis/inj ection-localized amyloidosis | Insulin | (46) |
| Medullary Carcinoma of the thyroid | Calcitonin | (47) |
| Isolated atrial amyloidosis | Atrial natriuretic factor | (48) |
| Hereditary Cerebral amyloid angiopathy | Cystatin C | (40) |
| Hereditary Cerebral Hemorrhage with Amyloidosis (Icelandic) | | |
| Familial amyotrophic lateral sclerosis | Superoxide dismutase 1 | (49) |
| 1. Holtzman (2001) J. Mol. Neurosci. 17: 147-155. | | |
| 2. Selkoe (2008) Behav. Brain. Res. 192: 106-113. | | |
| 3. Butterfield et al. (2007) Free Radic. Biol. Med. 43: 658-677. | | |
| 4. Thal et al. (2008) Acta Neuropathol. 115: 599-609. | | |
| 5. Lott et al. (2006) Curr. Alzheimer Res. 3: 521-528. | | |
| 6. Wang et al. (2008) J. Immunol. 181: 712-720. | | |
| 7. Krone et al. (2008) J. Mol. Biol. 381: 221-228. | | |
| 8. Fadika & Baumann (2002) Amyloid 9: 75-82. | | |
| 9. Rostagno et al. (2005) Cell Mol. Life Sci. 62: 1814-1825. | | |
| 10. Johnson et al. (1989) N. Engl. J. Med. 321: 513-518. | | |
| 11. Kahn et al. (1999) Diabetes, 48: 241-253. | | |
| 12. Baba et al. (1998) Am. J. Pathol. 152: 879-884. | | |
| 13. Dodel et al. (2008) J. Neurol. 255(Suppl 5): 39-47. | | |
| 14. Goux et al. (2004) J. Biol. Chem. 279: 26868-26875. | | |
| 15. Scherzinger et al. (1999) Proc. Natl. Acad. Sci. USA, 96: 4604-4609. | | |
| 16. Kanazawa (1999) Philos. Trans. R. Soc. Lond. B Biol. Sci. 354: 1069-1074. | | |
| 17. Yamada et al. (2006) Neuropathology, 26: 346-351. | | |
| 18. Manto (2005) Cerebellum 4: 2-6. | | |
| 19. Orr & Zoghbi (2001) Hum. Mol. Genet. 10: 2307-2311. | | |
| 20. Davies et al. (2008) J. Mol. Endocrinol. 41: 301-314. | | |
| 21. Collinge (1997) Hum. Mol. Genet. 6: 1699-1705. | | |
| 22. Prusiner (1993) Dev. Biol. Stand. 80: 31-44. | | |
| 23. Zou & Gambetti (2007) Cell Mol. Life Sci. 64: 3266-3270. | | |
| 24. Jahn et al. (2008) J. Biol. Chem. 283: 17279-17286. | | |
| 25. DiBartola & Benson (1989) J. Vet. Intern. Med. 3: 31-41. | | |
| 26. Obici et al. (2005) Biochim. Biophys. Acta: 1753: 11-22. | | |
| 27. Rocken et al. (1996) Pathol. Res. Pract. 192: 998-1006. | | |
| 28. Cross et al. (1992) J. Clin. Pathol. 45: 894-897. | | |
| 29. Demirci et al. (2006) Surv. Ophthalmol. 51: 419-433. | | |
| 30. Gertz & Rajkumar (2002) Curr. Treat. Options Oncol. 3: 261-271. | | |
| 31. Sanchorawala (2006) Clin. J. Am. Soc. Nephrol. 1: 1331-1341. | | |
| 32. Eulitz et al. (1990) Proc. Natl. Acad. Sci. USA, 87: 6542-6546. | | |
| 33. Fox (2005) Sjogren's syndrome. Lancet 366: 321-331. | | |
| 34. Shaheen et al. (2008) Adv. Anat. Pathol. 15: 196-210. | | |
| 35. Ronco et al. (2006) Clin. J. Am. Soc. Nephrol. 1: 1342-1350. | | |
| 36. Pepys et al. (1993) Nature, 362: 553-557. | | |
| 37. Gillmore et al. (1999) Nephrol. Dial. Transplant, 14: 2639-2644. | | |
| 38. Uemichi et al. (1994) J. Clin. Invest. 93: 731-736. | | |
| 39. Ozen (2003) Eur. J. Pediatr. 162: 449-454. | | |
| 40. Ghiso et al. (1986) Proc. Natl. Acad. Sci. USA, 83: 2974-2978. | | |
| 41. Petersen et al. (1997) Ann. Neurol. 41:307-313*.* | | |
| 42. Kelly (1996) Curr. Opin. Struct. Biol. 6: 11-17. | | |
| 43. Reixach et al. (2006) Biochem. Biophys. Res. Commun. 348: 889-897. | | |
| 44. Saraiva (1995) Hum. Mutat. 5: 191-196. | | |
| 45. Saraiva (2001) FEBS Lett. 498: 201-203. | | |
| 46. Swift (2002) Diabet. Med. 19: 881-882. | | |
| 47. Dammrich et al. (1984) Histochem. 81: 369-72. | | |
| 48. Torricelli et al. (2004) J. Mol. Endocrinol. 33: 335-41. | | |
| 49. Banci et al. (2008) PLoS ONE 3: e1677. | | |

It was a surprising discovery that the molecular tweezers described herein can inhibit the aggregation of amyloid proteins and thereby inhibit amyloidosis and consequently one or more of the symptoms associated with a pathology characterized by amyloidosis (*e.g*., pathologies listed in Table 1).

In particular, it was found that molecular tweezers, *e.g.*, as described herein, can inhibit Aβ folding, aggregation and toxicity, both *in vitro* and *in vivo,* making such compounds promising leads for development of drugs for treatment of AD (and other pathologies characterized by amyloidosis).

It was also demonstrated that molecular tweezers, *e.g*., as described herein, inhibits the aggregation and fibril formation of several other amyloidogenic proteins. Based on these results, it is believed that molecular tweezers can be useful for inhibiting assembly and toxicity of amyloid forming proteins other than Aβ. More generally, it is believed that the tweezers scaffold(s) described herein provide a useful general platform for development of drugs targeting such proteins for treatment of amyloid-related diseases, including, but not limited to those listed in Table 1.

Accordingly, in certain embodiments, molecular tweezers useful for inhibiting the assembly and/or toxicity of amyloid forming proteins are provided herein. Illustrative molecular tweezers are shown for example, in Figures 5 and 6. As explained above, in various embodiments, pharmaceutical formulations comprising one or more molecular tweezers species are contemplated. In addition, molecular tweezers fo use in the inhibition of amyloidosis and/or the treatment of pathologies characterized by amyloidosis are also within the scope of the present invention. Also described are the use of various molecular tweezers in the manufacture of a medicament to inhibit protein aggregation, (*e.g*., in certain embodiments, amyloidosis), and/or to treat a pathology characterized by the formation of aggregated protein deposits (*e.g.*, amyloid protein deposits).

### Molecular Tweezers for the Treatment of spinal cord and/or traumatic brain injury.

Spinal cord injury or traumatic brain injury may result in death or impairment of cells, *e.g.*, neurons and associated loss of function. Death or impairment of cells can negatively impact recovery. For example, neurodegeneration may reduce the potential for recovery of neuronal functions following spinal cord injury or traumatic brain injury. Cells impacted by spinal cord injury or traumatic brain injury may undergo immediate death or impairment or, alternatively, delayed death or impairment. Certain cells may be more likely to die following, *e.g*., spinal cord injury, than others.

Outcomes of spinal cord injury or traumatic brain injury include, but are not limited to, the impairment or death of cells, e.g. neurons (neurodegeneration). Aspects of neurodegeneration may include decreased neuronal survival, decreased axon sparing, and/or decreased axon growth. Neurodegeneration may limit functional recovery following spinal cord injury or traumatic brain injury or inhibit other post-injury neuronal activities, such as neuronal *(e.g.,* axonal) growth, neuronal *(e.g.* axonal) regeneration *(e.g.* axonal sprouting), or neuronal repair. Accordingly, a treatment of spinal cord injury or traumatic brain injury may be a treatment that, without limitation, decreases cellular impairment or cell death *(e.g.,* neurodegeneration), improves functional recovery, and/or improves post-injury neuronal activities.

The various treatments described herein may improve outcomes associated with any of one or more phenotypes or symptoms that may be associated with spinal cord injury or traumatic brain injury. Spinal cord injury or traumatic brain injury may result in a loss of function, such as mobility and/or feeling. SCI may result in, e.g. impairment of sensation, impairment of motor function, dysfunction of the bowel, dysfunction of the bladder, sexual dysfunction, impairment of fertility, inability to effectively regulate blood pressure, impairment of thermoregulation, impairment of sweating, chronic pain, or impairment of involuntary functions (e.g., breathing).

Treatments described herein may improve outcomes associated with any of one or more phenotypes or symptoms that may be associated with traumatic brain injury, including total or partial functional disability, psychosocial impairment, impairment of cognition, impairment of perception, impairment of motor abilities, impairment of physical functions, impairment of information processing, or impairment of speech, impairment of vision, impairment of hearing, sensory impairment; headaches; impairment of fine motor coordination; spasticity of muscles, paresis or paralysis of one or both sides, seizure disorders, impairment of balance; gait impairments, cognitive impairments (*e.g*., short- and long-term memory deficits, impaired concentration, slowness of thinking and limited attention span, as well as impairments of perception, communication, reading and writing skills, planning, sequencing, and judgment), and psychosocial-behavioral- emotional impairments (*e.g*., fatigue, mood swings, denial, self-centeredness, anxiety, depression, lowered self-esteem, sexual dysfunction, restlessness, lack of motivation, inability to selfmonitor, difficulty with emotional control, inability to cope, agitation, excessive laughing or crying, and difficulty relating to others), chronic or acute pain, and the like.

It was discovered that accumulation of amyloidogenic proteins (including, but not limited to amyloidogenic synucleins (*e.g*., α-synuclein) and/or non-amyloidogenic synuclein proteins may contribute to outcomes of spinal cord injury or traumatic brain injury, *e.g.,* to the death or impairment of cells, particularly the death or impairment of neurons. It has been demonstrated that accumulation of amyloidogenic proteins and synuclein proteins in cells following spinal cord injury, and/or aggregation of the accumulated proteins, may contribute to the death of cells (*see, e.g.,* U.S. Patent Pub. No: 2015/0202222). Synuclein proteins may contribute to neurotoxicity and neurodegeneration following spinal cord injury or traumatic brain injury. Without being bound to a particular theory, it is believed that accumulation and aggregation of synuclein proteins, such as α-synuclein, may result in aggregated intracellular inclusions or other intracellular structures, such as amyloid fibers. Alpha-synuclein (α-synuclein), Aβ, Tau, or other amyloidogenic proteins and non-amyloidogenic synuclein proteins known in the art may accumulate and/or aggregate independently following spinal cord injury or traumatic brain injury. Alternatively, any two or more of synuclein proteins and/or amyloidogenic proteins known in the art may accumulate and/or aggregate in an interdependent or correlated manner following spinal cord injury or traumatic brain injury. For instance, cells that accumulate synuclein proteins may also be more prone to accumulate ubiquitin-containing inclusions or to the aggregation of other amyloidogenic proteins, *e.g.,* Aβ or Tau.

Accordingly, compositions and methods for reducing accumulation and/or aggregation of amyloidogenic proteins (including amyloidogenic synuclein proteins) and non-amyloidogenic synuclein proteins following injury, *e.g*., spinal cord injury or traumatic brain injury are provided. In certain embodiments of the methods described herein, spinal cord injury and/or traumatic brain injury (including, but not limited to acute trauma, ischemia, and the like) is treated by administration of one or more molecular tweezers to the subject (*e.g.,* a human, a non-human mammal, a non-mammalian vertebrate, *etc*.) having the spinal cord and/or brain injury. In particular embodiments, the molecular tweezers comprises one or more of Compounds **2-19** (*see, e.g.,* Figures 5 and 6).

Treatment of spinal cord injury and/or traumatic brain injury using the methods described herein can improve survival, regeneration, or other outcomes in cells that are likely to die as a result of injury (*e.g*., neurons likely to die) and/or cells that are likely to survive injury (*e.g*., neurons that are likely to survive). Distinct cell types or groups of cells may respond to treatment with varying efficacy or varying responses. Treatment outcomes may also be observed at the systemic or organism level, including some aspects of functional recovery.

### Amyloidogenic Proteins and Synuclein Proteins

Expression, accumulation, oligomerization and/or aggregation of amyloidogenic proteins and/or synuclein proteins may negatively impact recovery following spinal cord injury or traumatic brain injury. Neurons may become impaired or die following spinal cord injury or traumatic brain injury, impairing cellular and functional recovery. Accumulation and/or aggregation of amyloidogenic proteins and/or synuclein proteins may contribute to cellular, *e.g*. neuronal, damage, impairment or death.

Examples of amyloidogenic proteins include, but are not limited to, α-synuclein, Tau, and Aβ. Examples of synuclein proteins are α-synuclein, β-synuclein, and γ synuclein. It is noted that not all synuclein proteins are amyloidogenic. Thus, for example, α-synuclein is known to be amyloidogenic, while β-synuclein and γ-synuclein are generally understood to be non-amyloidogenic. Sequences corresponding to each are known in the art. For example, human synuclein mRNA sequences include, *e.g*., Accession Numbers NM_000345.3 (α-synuclein), NM_001001502.1 (β-synuclein), and NM_003087 (γ-synuclein). Synucleins are also known in other species, *e.g*., Lamprey synuclein mRNA sequences include, *e.g*., Accession Number JN544525.1 (γ synuclein). Human Tau protein mRNA sequences include, *e.g.*, Accession Number NM_016835. Human Aβ precursor protein mRNA sequences include, *e.g.,* NM_000484.

In various embodiments, methods of inhibiting the accumulation or aggregation of amyloidogenic proteins (optionally including α-synuclein) and/or non-amyloidogenic synuclein proteins following spinal cord injury and/or traumatic brain injury are provided. In certain embodiments, such methods include treatment with a molecular tweezers capable of inhibiting accumulation or aggregation of one or more amyloidogenic proteins and/or synuclein proteins. Examples of treatments to inhibit accumulation or aggregation of one or more amyloidogenic proteins and/or synuclein proteins include treatment with an α-synuclein antisense nucleobase oligomer or treatment with the molecular tweezers (*e.g*., one or more of Compounds **2-19**). These treatments may improve outcomes of spinal cord surgery. For instance, treatment with a molecular tweezers or nucleobase oligomer improve neuronal survival, improve axon sparing, improve axon growth, improve neuronal regeneration, improve axon regeneration, or improve axon sprouting. Improved outcomes may occur in the most severely damaged cells, *e.g*., neurons that are likely to die. Improved outcomes may additionally or alternatively occur in other neurons, such as neurons that are likely to survive. Certain improvements may be observed at the organismal or systemic level. Improvements may occur evenly or unevenly across various cell types, individually identifiable cells, or groups of cells having particular characteristics.

### Molecular tweezers for use as antivirals.

It was discovered that the compounds (*e.g*., molecular tweezers) described herein are effective for inhibiting viruses, in certain embodiments enveloped viruses, *in vivo* or *ex vivo.* Accordingly, in certain embodiments a method of inhibiting the growth, and/or the proliferation, and/or the infectivity, of an enveloped virus, is provided where the method involves contacting the virus with an effective amount of a compound (molecular tweezers) as described herein. In certain embodiments the method comprises inhibiting the growth and/or proliferation of the virus. In certain embodiments the method comprises inhibiting the infectivity of the virus. In certain embodiments the method comprises reducing the titer of a viral infection in the mammal.

In certain embodiments the method comprises administering the compound (molecular tweezers) to a mammal infected with the virus. In certain embodiments the method comprises administering the compound (molecular tweezers) to a mammal at risk for infection by the virus. In certain embodiments the mammal is a non-human mammal. In certain embodiments the mammal is a human. In certain embodiments the virus comprises an enveloped virus. In certain embodiments the virus is a member of a family selected from the group consisting of Herpesviridae, Poxviridae, Hepadnaviridae, Coronaviridae, Flaviviridae, Togaviridae, Retroviridae, Orthomyxoviridae, Arenaviridae, Bunyaviridae, Filoviridae, Paramyxoviridae, and Rhabdoviridae (*see, e.g.,* Table 2, below). In certain embodiments the virus comprises a virus selected from the group consisting of Herpes simplex, type 1, Herpes simplex, type 2, Varicella-zoster virus, Epstein-Barr virus, Human cytomegalovirus, Human herpesvirus, type 8, Smallpox, Hepatitis B virus, Severe acute respiratory syndrome virus, Hepatitis C virus, yellow fever virus, dengue virus, West Nile virus, TBE virus Zika virus, Rubella virus, Human immunodeficiency virus (HIV), Influenza virus, Lassa virus, Crimean-Congo hemorrhagic fever virus, Hantaan virus, Ebola virus, Marburg virus, Measles virus, Mumps virus, Parainfluenza virus, Respiratory syncytial virus, Rabies virus, and Hepatitis D virus (HDV) (*see, e.g.,* Table 2, below). In certain embodiments the virus is Zika virus or HIV-1 (*see, e.g.,* Examples herein). In certain embodiments administering the compound(s) (*e.g*., molecular tweezers) in an amount sufficient to ameliorates one or more symptoms of a pathology caused by the virus and/or to slow or prevent infection of the mammal by the virus

Table 2. Illustrative enveloped viruses responsible for diseases in humans.

| **Family** | **Baltimore Group** | **Important species** |
|---|---|---|
| Herpesviridae | Group I (dsDNA) | Herpes simplex, type 1, Herpes simplex, type 2, Varicella-zoster virus, Epstein-Barr virus, Human cytomegalovirus, Human herpesvirus, type 8 |
| Poxviridae | Group I (dsDNA) | Smallpox |
| Hepadnaviridae | Group VII (dsDNA-RT) | Hepatitis B virus |
| Coronaviridae | Group IV (positive-sense ssRNA) | Severe acute respiratory syndrome virus |
| Flaviviridae | Group IV (positive-sense ssRNA) | Hepatitis C virus, yellow fever virus, dengue virus, West Nile virus, TBE virus Zika virus |
| Togaviridae | Group IV (positive-sense ssRNA) | Rubella virus |
| Retroviridae | Group VI (ssRNA-RT) | Human immunodeficiency virus (HIV) |
| Orthomyxoviridae | Group V (negative-sense ssRNA) | Influenza virus |
| Arenaviridae | Group V (negative-sense ssRNA) | Lassa virus |
| Bunyaviridae | Group V (negative-sense ssRNA) | Crimean-Congo hemorrhagic fever virus, Hantaan virus |
| Filoviridae | Group V (negative-sense ssRNA) | Ebola virus, Marburg virus |
| Paramyxoviridae | Group V (negative-sense ssRNA) | Measles virus, Mumps virus, Parainfluenza virus, Respiratory syncytial virus, |
| Rhabdoviridae | Group V (negative-sense ssRNA) | Rabies virus |
| Unassigned | Group V (negative-sense ssRNA) | Hepatitis D |

### Molecular tweezers for the treatment of lysosomal storage diseases.

In various embodiments the compound(s) described herein (*e.g*., molecular tweezers) (*see, e.g.,* compounds 2-19 in Figures 5 and 6) are believed to be useful for the treatment and/or prophylaxis of liposomal storage diseases.

Accordingly, in various embodiments one or more compounds (*e.g.*, molecular tweezers) described herein are administered to a subject (*e.g.,* to a mammal in need thereof) for the treatment or prophylaxis of a lysosomal storage disease (LSD). In certain embodiments the lysosomal storage disease comprises an LSD with neuropathological implications and consequent neurological impairment.

Lysosomal storage diseases (LSDs) are a group of about 70 inherited metabolic disorders that result from defects in lysosomal function. Lysosomes are intracellular compartments that contain enzymes that digest large molecules and pass the fragments on to other parts of the cell for recycling. This process requires several critical enzymes and if one or more of these enzymes is defective, e.g., because of a mutation, the large molecules accumulate within the cell, eventually killing it.

Lysosomal storage disorders are caused by lysosomal dysfunction usually as a consequence of deficiency of a single enzyme required for the metabolism of lipids, glycoproteins, or so-called mucopolysaccharides. Individually, LSDs occur with incidences of less than 1: 100,000; however, as a group, the incidence is about 1:5,000 - 1: 10,000 (see, *e.g.,* Meikle et al. (1999) JAMA, 281(3): 249-254). Most of these disorders are autosomal recessively inherited such as Niemann-Pick disease, type C, but a few are X-linked recessively inherited, such as Fabry disease and Hunter syndrome (MPS II).

Lysosomal disorders are usually triggered when a particular lysosome enzyme exists in too small an amount or is missing altogether. When this happens excess products destined for breakdown and recycling are stored in the cell.

Like other genetic disorders, individuals inherit lysosomal storage diseases from their parents. Although each disorder results from different gene mutations that translate into a deficiency in enzyme activity, they all share a common biochemical characteristic - all lysosomal disorders originate from an abnormal accumulation of substances inside the lysosome.

LSDs affect mostly children and they often die at a young and unpredictable age, many within a few months or years of birth. Many other children die of this disease following years of suffering from various symptoms of their particular disorder.

The LSDs are generally classified by the nature of the primary stored material involved, and can be broadly broken into the following: 1) Lipid storage disorders, mainly sphingolipidoses (including Gaucher's and Niemann-Pick diseases); 2) Gangliosidosis (including Tay-Sachs disease; 3) Leukodystrophies; 4) Mucopolysaccharidoses (including Hunter syndrome and Hurler disease); 5) glycoprotein storage disorders; and 6) mucolipidoses.

In certain embodiments lysosomal storage diseases include but are not limited to, Sphingolipidoses, Ceramidase (*e.g*., Farber disease, Krabbe disease), Galactosialidosis, gangliosidoses including Alpha-galactosidases (*e.g.,* Fabry disease (alpha-galactosidase A), Schindler disease (alpha-galactosidase B)), Beta-galactosidase (*e.g.,* GM1 gangliosidosis, GM2 gangliosidosis, Sandhoff disease, Tay-Sachs disease), Glucocerebrosidoses (*e.g*., Gaucher disease (Type I, Type II, Type III), Sphingomyelinase (*e.g.,* Lysosomal acid lipase deficiency, Niemann-Pick disease), Sulfatidosis (*e.g.,* Metachromatic leukodystrophy. Multiple sulfatase deficiency), Mucopolysaccharidoses (*e.g.,* Type I (MPS I (Hurler syndrome, MPS I S Scheie syndrome, MPS I H-S Hurler-Scheie syndrome), Type II (Hunter syndrome), Type III (Sanfilippo syndrome), Type IV (Morquio), Type VI (Maroteaux-Lamy syndrome), Type VII (Sly syndrome), Type IX (hyaluronidase deficiency)), mucolipidoses (*e.g*., Type I (sialidosis), Type II (I-cell disease), Type III (pseudo-Hurler polydystrophy/phosphotransferase deficiency), Type IV (mucolipidin 1 deficiency)), lipidoses (*e.g*., Niemann-Pick disease), Neuronal ceroid lipofuscinoses (*e.g*., Type 1 Santavuori-Haltia disease/ infantile NCL (CLN1 PPT1)), Type 2 Jansky-Bielschowsky disease / late infantile NCL (CLN2/LINCL TPP1), Type 3 Batten-Spielmeyer-Vogt disease / juvenile NCL (CLN3), Type 4 Kufs disease / adult NCL (CLN4), Type 5 Finnish Variant / late infantile (CLN5), Type 6 Late infantile variant (CLN6), Type 7 CLN7, Type 8 Northern epilepsy (CLN8), Type 8 Turkish late infantile (CLN8), Type 9 German/Serbian late infantile, Type 10 Congenital cathepsin D deficiency (CTSD)), Wolman disease, Oligosaccharidoses (*e.g*., Alpha-mannosidosis, Betamannosidosis, Aspartylglucosaminuria, Fucosidosis), lysosomal transport diseases (*e.g*., Cystinosis, Pycnodysostosis, Salla disease / sialic acid storage disease, Infantile free sialic acid storage disease), Glycogen storage diseases, *e.g*., Type II Pompe disease, Type IIb Danon disease), Cholesteryl ester storage disease, and the like.

Illustrative lysosomal storage diseases that can be treated using the molecular tweezers described herein include, but are not limited to a mucopolysaccharidosis (MPS), aspartylglucosaminuria, GM1-gangliosidosis, Krabbe (globoid cell leukodystrophy or galactosylceramide lipidosis), Metachromatic leukodystrophy, Sandhoff disease, mucolipidosis type II (I-cell disease), mucolipidosis type IIIA (pseudo-Hurler polydystrophy), Niemann-Pick disease type C2 and C1, Danon disease, free sialic acid storage disorder, mucolipidosis type IV, and multiple sulfatase deficiency (MSD).

In certain embodiments the lysosomal storage disease comprises a mucopolysaccharidosis selected from the group consisting of Sanfilippo syndrome (MPS III), Hurler syndrome (MPS IH), Hurler-Scheie syndrome (MPS I-H/S), Scheie syndrome (MPS IS), Hunter syndrome (MPS II), Morquio syndrome (MP IV), Maroteaux-Lamy syndrome (MPS VI), Sly syndrome (MPS VII), and MPS IX.

### Sanfilippo syndrome (MPS III)

Mucopolysaccharidosis type III (MPS III), is characterized by severe and rapid intellectual deterioration. Deficiencies in one of the four enzymes required for heparan sulfate (HS) degradation are responsible for each of the MPS III subtypes: heparan sulfamidase for MPS IIIA, alpha-N-acetylglucosaminidase for MPS IIIB, alphaglucosaminide N-acetyltransferase for MPS IIIC, and N-acetylglucosamine-6-sulfate sulfatase for MPS IIID. The clinical features of MPS III include, but are not limited to severe mental defects with relatively mild somatic features (moderately severe claw hand and visceromegaly, little or no corneal clouding or skeletal, *e.g*., vertebral, change). The first symptoms typically appear between the ages of 2 and 6 years, with behavioral disorders (hyperkinesia, aggressiveness) and intellectual deterioration, sleep disorders and very mild dysmorphism. The neurological involvement becomes more prominent around 10 years of age with loss of motor milestones and communication problems. Seizures often occur after the age of 10. A few cases of attenuated forms have also been reported. The prognosis is poor with death occurring in most cases of type IIIA at the end of the second decade. Longer survival times (30/40 years) have been reported for the B and D subtypes.

Diagnosis of MPS III is based on detection of increased levels of heparan sulfate (HS) in urine. Demonstration of one of the four enzyme deficiencies in cultivated leukocytes or fibroblasts allows determination of the type of MPS III. For types IIIA and IIID, the measurement of the activity of another sulfatase is compulsory for exclusion of multiple sulfatase deficiency (MSD or Austin disease).

In certain embodiments the prophylactic and/or therapeutic methods described herein involve ameliorating one or more of the above symptoms, *e.g*., one or more symptoms selected from the group consisting of cognitive deficiencies, claw hand, visceromegaly, sleep disorders, loss of motor function, loss of communication abilities, and seizures) and/or delaying the onset, slowing, stopping, or reversing the progression of one or more of these symptoms.

### Hurler syndrome (MPS I)

Hurler syndrome, also known as mucopolysaccharidosis type I (MPS I), Hurler's disease, also gargoylism, is a genetic disorder that results in the buildup of glycosaminoglycans (a.k.a. mucopolysaccharides) due to a deficiency of alpha-L iduronidase, an enzyme responsible for the degradation of mucopolysaccharides in lysosomes. Without this enzyme, a buildup of dermatan sulfate and heparan sulphate occurs in the body. Symptoms appear during childhood and early death can occur due to organ damage. MPS I is divided into three subtypes based on severity of symptoms. All three types result from an absence of, or insufficient levels of, the enzyme α-L-iduronidase. MPS I H or Hurler syndrome is the most severe of the MPS I subtypes. The other two types are MPS I S or Scheie syndrome and MPS I H-S or Hurler-Scheie syndrome.

Hurler syndrome is marked by progressive deterioration, hepatosplenomegaly, dwarfism, and unique facial features. A progressive mental retardation occurs, with death frequently occurring by the age of 10 years. Developmental delay is evident by the end of the first year, and patients usually stop developing between ages 2 and 4. This is followed by progressive mental decline and loss of physical skills. Language may be limited due to hearing loss and an enlarged tongue. In time, the clear layers of the cornea become clouded and retinas may begin to degenerate. Carpal tunnel syndrome (or similar compression of nerves elsewhere in the body) and restricted joint movement are common.

In certain embodiments the prophylactic and/or therapeutic methods described herein involve ameliorating one or more of the above symptoms

### Hunter syndrome (MPS II)

Mucopolysaccharidosis type II (MPS II), also known as Hunter syndrome, is a condition that affects many different parts of the body and occurs almost exclusively in males. It is a progressively debilitating disorder.

At birth, individuals with MPS II typically do not display any features of the condition. Between ages 2 and 4, they develop full lips, large rounded cheeks, a broad nose, and an enlarged tongue (macroglossia). The vocal cords also enlarge, which results in a deep, hoarse voice. Narrowing of the airway causes frequent upper respiratory infections and short pauses in breathing during sleep (sleep apnea). As the disorder progresses, individuals need medical assistance to keep their airway open.

Individuals with this disorder often have a large head (macrocephaly), a buildup of fluid in the brain (hydrocephalus), an enlarged liver and spleen (hepatosplenomegaly). Most people with this disorder develop hearing loss and have recurrent ear infections. Some individuals with MPS II develop problems with the retina and have reduced vision. Carpal tunnel syndrome commonly occurs in children with this disorder and is characterized by numbness, tingling, and weakness in the hand and fingers. Narrowing of the spinal canal (spinal stenosis) in the neck can compress and damage the spinal cord. The heart is also significantly affected by MPS II, and many individuals develop heart valve problems.

In certain embodiments the prophylactic and/or therapeutic methods described herein involve ameliorating one or more of the above symptoms

### Mucopolysaccharidosis type VII (MPS VII)

Mucopolysaccharidosis type VII (MPS VII), also known as Sly syndrome, is a progressive condition that affects most tissues and organs. The most severe cases of MPS VII are characterized by hydrops fetalis, a condition in which excess fluid builds up in the body before birth. Most babies with hydrops fetalis are stillborn or die soon after birth.

Other people with MPS VII typically begin to show signs and symptoms of the condition during early childhood. The features of MPS VII include a large head (macrocephaly), a buildup of fluid in the brain (hydrocephalus), distinctive-looking facial features that are described as "coarse," and a large tongue (macroglossia). Affected individuals also frequently develop an enlarged liver and spleen (hepatosplenomegaly), heart valve abnormalities, and a soft out-pouching around the belly-button (umbilical hernia) or lower abdomen (inguinal hernia). The airway may become narrow in some people with MPS VII, leading to frequent upper respiratory infections and short pauses in breathing during sleep (sleep apnea). The cornea becomes cloudy which can cause significant vision loss. People with MPS VII may also have recurrent ear infections and hearing loss. Affected individuals may have developmental delay and progressive intellectual disability, although intelligence is unaffected in some people with this condition.

In certain embodiments the prophylactic and/or therapeutic methods described herein involve ameliorating one or more of the above symptoms.

### Aspartylglucosaminuria.

Aspartylglucosaminuria is a condition that causes a progressive decline in mental functioning. Infants with aspartylglucosaminuria appear healthy at birth, and development is typically normal throughout early childhood. Symptoms of this condition typically present around the age of 2 or 3, and are often characterized by delayed speech. Mild intellectual disability then becomes apparent, and learning occurs at a slowed pace. Intellectual disability progressively worsens in adolescence. Most people with this disorder lose much of the speech they have learned, and affected adults usually have only a few words in their vocabulary. Adults with aspartylglucosaminuria may develop seizures or problems with movement. People with this condition may also have bones that become progressively weak and prone to fracture (osteoporosis), an unusually large range of joint movement (hypermobility), and loose skin.

In certain embodiments the prophylactic and/or therapeutic methods described herein involve ameliorating one or more of the above symptoms (e.g., one or more symptoms selected from the group consisting of delay or loss of speech, cognitive impairment, seizures, locomotor impairment, osteoporosis, and joint hypermobility), and/or delaying the onset, slowing, stopping, or reversing the progression of one or more of these symptoms.

### GM1-glngliosidosis

M1 gangliosidosis is an inherited disorder that progressively destroys nerve cells in the brain and spinal cord. The signs and symptoms of the most severe form of GM1 gangliosidosis, called type I or the infantile form, usually become apparent by the age of 6 months. Infants with this form of the disorder typically appear normal until their development slows and muscles used for movement weaken. Affected infants eventually lose the skills they had previously acquired (developmentally regress) and may develop an exaggerated startle reaction to loud noises. As the disease progresses, children with GM1 gangliosidosis type I can develop an enlarged liver and spleen (hepatosplenomegaly), skeletal abnormalities, seizures, profound intellectual disability, and clouding of the cornea. Loss of vision occurs as the retina gradually deteriorates. An eye abnormality called a cherry-red spot, which can be identified with an eye examination, is characteristic of this disorder. In some cases, affected individuals have an enlarged and weakened heart muscle (cardiomyopathy).

Type II GM1 gangliosidosis consists of intermediate forms of the condition, also known as the late infantile and juvenile forms. Children with GM1 gangliosidosis type II typically show normal early development, but they begin to develop signs and symptoms of the condition around 18 months of age (late infantile form) or 5 years (juvenile form). Individuals with GM1 gangliosidosis type II experience developmental regression but usually do not have cherry-red spots, distinctive facial features, or enlarged organs. Type II usually progresses more slowly than type I, but still causes a shortened life expectancy . People with the late infantile form typically survive into mid-childhood, while those with the juvenile form may live into early adulthood.

Type III of GM1 gangliosidosis is known as the adult or chronic form, and it represents the mildest end of the disease spectrum. The age at which symptoms first appear varies in GM1 gangliosidosis type III, although most affected individuals develop signs and symptoms in their teens. The characteristic features of this type include involuntary tensing of various muscles (dystonia) and abnormalities of the spinal bones (vertebrae).

In certain embodiments the prophylactic and/or therapeutic methods described herein involve ameliorating one or more of the above symptoms (e.g., one or more symptoms selected from the group consisting of cognitive impairment, locomotor impairment, hepatosplenomegaly, skeletal abnormalities, seizures, clouding of the cornea, and loss of vision), and/or delaying the onset, slowing, stopping, or reversing the progression of one or more of these symptoms.

### Krabbe disease (globoid cell leukodystrophy).

Krabbe disease (also called globoid cell leukodystrophy) is a severe neurological condition. It is part of a group of disorders known as leukodystrophies, that result from the loss of myelin (demyelination) in the nervous system. Krabbe disease is also characterized by abnormal cells in the brain called globoid cells, which are large cells that usually have more than one nucleus.

The most common form of Krabbe disease, called the infantile form, usually begins before the age of 1. Initial signs and symptoms typically include irritability, muscle weakness, feeding difficulties, episodes of fever without any sign of infection, stiff posture, and delayed mental and physical development. As the disease progresses, muscles continue to weaken, affecting the infant's ability to move, chew, swallow, and breathe. Affected infants also experience vision loss and seizures. Because of the severity of the condition, individuals with the infantile form of Krabbe disease rarely survive beyond the age of 2. Less commonly, Krabbe disease begins in childhood, adolescence, or adulthood (late-onset forms). Vision problems and walking difficulties are the most common initial symptoms in these forms of the disorder, however, signs and symptoms vary considerably among affected individuals.

In certain embodiments the prophylactic and/or therapeutic methods described herein involve ameliorating one or more of the above symptoms (*e.g.*, one or more symptoms selected from the group consisting of irritability, fevers, limb stiffness, seizures, feeding difficulties, vomiting, and cognitive impairment, locomotor impairment, muscle weakness, spasticity, deafness, optic atrophy, optic nerve enlargement, blindness, paralysis, and difficulty when swallowing), and/or delaying the onset, slowing, stopping, or reversing the progression of one or more of these symptoms.

### Metachromatic leukodystrophy

Metachromatic leukodystrophy is an inherited disorder characterized by the accumulation of fats called sulfatides in cells. This accumulation especially affects cells in the nervous system that produce myelin. Sulfatide accumulation in myelin-producing cells causes progressive destruction of white matter (leukodystrophy) throughout the nervous system, including in the brain and spinal cord (the central nervous system) and the nerves connecting the brain and spinal cord to muscles and sensory cells that detect sensations such as touch, pain, heat, and sound (the peripheral nervous system).

In people with metachromatic leukodystrophy, white matter damage causes progressive deterioration of intellectual functions and motor skills, such as the ability to walk. Affected individuals also develop loss of sensation in the extremities (peripheral neuropathy), incontinence, seizures, paralysis, an inability to speak, blindness, and hearing loss. Eventually they lose awareness of their surroundings and become unresponsive. While neurological problems are the primary feature of metachromatic leukodystrophy, effects of sulfatide accumulation on other organs and tissues have been reported, most often involving the gallbladder.

In certain embodiments the prophylactic and/or therapeutic methods described herein involve ameliorating one or more of the above symptoms (*e.g*., one or more symptoms selected from the group consisting of leukodystrophy throughout CNS and/or peripheral nervous system, cognitive impairment, loss of sensation in the extremities (peripheral neuropathy), incontinence, seizures, paralysis, an inability to speak, blindness, and hearing loss), and/or delaying the onset, slowing, stopping, or reversing the progression of one or more of these symptoms.

### Sandhoff disease

Sandhoff disease is an inherited disorder that progressively destroys nerve cells (neurons) in the brain and spinal cord. The most common and severe form of Sandhoff disease becomes apparent in infancy. Infants with this disorder typically appear normal until the age of about 3 to 6 months, when their development slows and muscles used for movement weaken. Affected infants lose motor skills such as turning over, sitting, and crawling. As the disease progresses, children with Sandhoff disease experience seizures, vision and hearing loss, intellectual disability, and paralysis. An eye abnormality called a cherry-red spot, which can be identified with an eye examination, is characteristic of this disorder. Some affected children also display organomegaly and/or bone abnormalities.

In certain embodiments the prophylactic and/or therapeutic methods described herein involve ameliorating one or more of the above symptoms (*e.g.*, one or more symptoms selected from the group consisting of cognitive impairment, loss of locomotor function, seizures, hearing loss vision loss, organomegaly, bone abnormalities, and paralysis), and/or delaying the onset, slowing, stopping, or reversing the progression of one or more of these symptoms.

### Mucolipidosis type II (I-cell disease)

Mucolipidosis II alpha/beta (also known as I-cell disease) is a progressively debilitating disorder that affects many parts of the body. Most affected individuals do not survive past early childhood.

At birth, children with mucolipidosis II alpha/beta are small and typically have weak muscle tone (hypotonia) and a weak cry. Affected individuals grow slowly after birth and usually stop growing during the second year of life. Development is delayed, particularly the development of speech and motor skills such as sitting and standing.

Children with mucolipidosis II alpha/beta typically have several bone abnormalities, many of which are present at birth. Affected individuals may have an abnormally rounded upper back (kyphosis), feet that are abnormally rotated (clubfeet), dislocated hips, unusually shaped long bones, and short hands and fingers. People with this condition can also have joint deformities (contractures) that significantly affect mobility. Most children with mucolipidosis II alpha/beta do not develop the ability to walk independently. Affected individuals can have dysostosis multiplex. Other features of mucolipidosis II alpha/beta include, but are not limited to heart valve abnormalities, narrowing of the airway which can contribute to prolonged or recurrent respiratory infections, and recurrent ear infections, which can lead to hearing loss.

In certain embodiments the prophylactic and/or therapeutic methods described herein involve ameliorating one or more of the above symptoms (*e.g.*, one or more symptoms selected from the group consisting of cognitive impairment, loss of locomotor function, seizures, hearing loss vision loss, organomegaly, bone abnormalities, and paralysis), and/or delaying the onset, slowing, stopping, or reversing the progression of one or more of these symptoms.

### Mucolipidosis type IIIA (pseudo-Hurler polydystrophy)

Pseudo-Hurler polydystrophy (mucolipidosis type III) is a genetic metabolic disorder characterized by a defective enzyme known as UPD-N-acetylglucosamine-1-phosphotransferase. This defective enzyme ultimately results in the accumulation of certain complex carbohydrates (mucopolysaccharides) and fatty substances (mucolipids) in various tissues of the body. The symptoms of this disorder are similar, but less severe than those of I-cell disease (mucolipidosis type II) and may include, but are not limited to, progressive joint stiffness, curvature of the spine (scoliosis), and/or skeletal deformities of the hands (*e.g*., claw-hands). Growth delays accompanied by deterioration of the hip joints typically develop in children with pseudo-Hurler polydystrophy. Additional symptoms may include clouding of the corneas of the eyes, mild to moderate coarseness of facial features, mild mental retardation, easy fatigability, and/or heart disease.

In most cases, children with pseudo-Hurler polydystrophy do not exhibit symptoms until 2-4 years of age. Specific symptoms and rate of progression may vary from case to case although the disorder is often slowly progressive.

Initial symptoms may include stiffness of the hands and shoulders. In some cases, claw-like deformities of the hands may occur. These symptoms may progress to cause difficulty with specific tasks (*e.g.*, getting dressed) . Eventually, carpal tunnel syndrome may develop. Carpal tunnel syndrome is a neurological disorder characterized by compression of the median nerve, which passes through the carpal tunnel inside the wrist (peripheral nerve entrapment). Symptoms of this disorder affect the hand and wrist and may include pain, numbness, loss of feeling in the fingertips, and/or unusual sensation such as burning or "pins and needles."

Additional symptoms associated with pseudo-Hurler polydystrophy may include scoliosis, degeneration of the hip, joints that are permanently fixed in a bent or flexed position (contractures), and short stature. Progressive degeneration of the hip and joint contractures may cause difficulty walking or force affected individuals to walk with a characteristic waddling gait. Affected children may also develop corneal opacity, mild retinopathy, and irregular curvature of the cornea (hyperopic astigmatism). Although many children with pseudo-Hurler polydystrophy have normal intelligence, some may develop mild mental retardation or learning disabilities. In some cases, affected children develop aortic insufficiency.

In certain embodiments the prophylactic and/or therapeutic methods described herein involve ameliorating one or more of the above symptoms (*e.g.*, one or more symptoms selected from the group consisting of selected from the group consisting of joint stiffness, scoliosis, skeletal deformities of the hands (*e.g*., claw-hands), growth delays, deterioration of the hip joints, clouding of the corneas of the eyes, mild mental retardation, easy fatigability, carpal tunnel syndrome, and heart disease), and/or delaying the onset, slowing, stopping, or reversing the progression of one or more of these symptoms.

### Niemann-Pick disease type C2 and C1 (NPCl mutation)

Niemann-Pick type C has a wide clinical spectrum. Affected individuals may have enlargement of the spleen (splenomegaly) and liver (hepatomegaly), or enlarged spleen or liver combined (hepatosplenomegaly), but this finding may be absent in later onset cases. Prolonged jaundice or elevated bilirubin can present at birth. In some cases, enlargement of the spleen or liver does not occur for months or years, or not at all. Enlargement of the spleen or liver frequently becomes less apparent with time, in contrast to the progression of other lysosomal storage diseases such as Niemann-Pick disease.

Progressive neurological disease is the hallmark of Niemann-Pick type C disease, and is responsible for disability and premature death in most cases beyond early childhood. Classically, children with NPC may initially present with delays in reaching normal developmental milestones skills before manifesting cognitive decline (dementia). Neurological signs and symptoms include cerebellar ataxia (unsteady walking with uncoordinated limb movements), dysarthria (slurred speech), dysphagia (difficulty in swallowing), tremor, epilepsy (both partial and generalized), vertical supranuclear palsy (upgaze palsy, downgaze palsy, saccadic palsy or paralysis), sleep inversion, gelastic cataplexy (sudden loss of muscle tone or drop attacks), dystonia (abnormal movements or postures caused by contraction of agonist and antagonist muscles across joints), most commonly begins with in turning of one foot when walking (action dystonia) and may spread to become generalized, spasticity (velocity dependent increase in muscle tone), hypotonia, ptosis (drooping of the upper eyelid), microcephaly (abnormally small head), psychosis, progressive dementia, progressive hearing loss, bipolar disorder, major and psychotic depression that can include hallucinations, delusions, mutism, or stupor. Typically, in the terminal stages of Niemann-Pick type C disease, the subject is bedridden, with complete ophthalmoplegia, loss of volitional movement and severe dementia.

In certain embodiments the prophylactic and/or therapeutic methods described herein involve ameliorating one or more of the above symptoms (*e.g.*, one or more symptoms selected from the group consisting of splenomegaly, hepatomegaly, hepatosplenomegaly, jaundice, cognitive impairment, cerebellar ataxia (unsteady walking with uncoordinated limb movements), dysarthria (slurred speech), dysphagia (difficulty in swallowing), tremor, epilepsy (both partial and generalized), vertical supranuclear palsy (upgaze palsy, downgaze palsy, saccadic palsy or paralysis), sleep inversion, gelastic cataplexy (sudden loss of muscle tone or drop attacks), dystonia (abnormal movements or postures caused by contraction of agonist and antagonist muscles across joints), spasticity (velocity dependent increase in muscle tone), hypotonia, ptosis (drooping of the upper eyelid), psychosis, progressive dementia, progressive hearing loss, bipolar disorder, major and psychotic depression, loss of volitional movement, and severe dementia), and/or delaying the onset, slowing, stopping, or reversing the progression of one or more of these symptoms.

### Danon disease

Danon disease is a condition typically characterized by cardiomyopathy, weakening of the muscles used for movement, called skeletal muscles (myopathy), and intellectual disability. Signs and symptoms begin in childhood or adolescence in most affected males and in early adulthood in most affected females.

Cardiomyopathy is the most common symptom of Danon disease and occurs in all males with the condition. Most affected men have hypertrophic cardiomyopathy. Other affected males have dilated cardiomyopathy, which is a condition that weakens and enlarges the heart, preventing it from pumping blood efficiently. Some affected men with hypertrophic cardiomyopathy later develop dilated cardiomyopathy. Either type of cardiomyopathy can lead to heart failure and premature death. Most women with Danon disease also develop cardiomyopathy.

Skeletal myopathy occurs in most men with Danon disease and about half of affected women. The weakness typically occurs in the muscles of the upper arms, shoulders, neck, and upper thighs. Many males with Danon disease have elevated levels creatine kinase in their blood, which is often a biomarker that indicates muscle disease.

In certain embodiments the prophylactic and/or therapeutic methods described herein involve ameliorating one or more of the above symptoms (*e.g*., one or more symptoms selected from the group consisting of cardiomyopathy, skeletal muscle myopathy, and cognitive impairment), and/or delaying the onset, slowing, stopping, or reversing the progression of one or more of these symptoms.

### Free sialic acid storage disorder

Sialic acid storage disease is an inherited disorder that primarily affects the nervous system. People with sialic acid storage disease have signs and symptoms that may vary widely in severity. This disorder is generally classified into one of three forms: infantile free sialic acid storage disease, Salla disease, and intermediate severe Salla disease.

Infantile free sialic acid storage disease (ISSD) is the most severe form of this disorder. B abies with this condition have severe developmental delay, weak muscle tone (hypotonia), and failure to gain weight and grow at the expected rate (failure to thrive). They may have unusual facial features that are often described as "coarse," seizures, bone malformations, an enlarged liver and spleen (hepatosplenomegaly), and an enlarged heart (cardiomegaly). The abdomen may be swollen due to the enlarged organs and an abnormal buildup of fluid in the abdominal cavity (ascites). Affected infants may have a condition called hydrops fetalis in which excess fluid accumulates in the body before birth. Children with this severe form of the condition usually live only into early childhood.

Salla disease is a less severe form of sialic acid storage disease. Babies with Salla disease usually begin exhibiting hypotonia during the first year of life and go on to experience progressive neurological problems. Signs and symptoms of Salla disease include intellectual disability and developmental delay, seizures, problems with movement and balance (ataxia), abnormal tensing of the muscles (spasticity), and involuntary slow, sinuous movements of the limbs (athetosis). Individuals with Salla disease usually survive into adulthood. People with intermediate severe Salla disease have signs and symptoms that fall between those of ISSD and Salla disease in severity.

In certain embodiments the prophylactic and/or therapeutic methods described herein involve ameliorating one or more of the above symptoms (e.g., one or more symptoms selected from the group consisting of cardiomyopathy, skeletal muscle myopathy, and cognitive impairment), and/or delaying the onset, slowing, stopping, or reversing the progression of one or more of these symptoms.

### Mucolipidosis IV

Mucolipidosis type IV is an inherited disorder characterized by delayed development and vision impairment that worsens over time. The severe form of the disorder is called typical mucolipidosis type IV, and the mild form is called atypical mucolipidosis type IV.

Approximately 95 percent of individuals with this condition have the severe form. People with typical mucolipidosis type IV typically have delayed development of mental and motor skills (psychomotor delay). Motor skills include sitting, standing, walking, grasping objects, and writing. Psychomotor delay is moderate to severe and usually becomes apparent during the first year of life. Affected individuals have intellectual disability, limited or absent speech, difficulty chewing and swallowing, weak muscle tone (hypotonia) that gradually turns into abnormal muscle stiffness (spasticity), and problems controlling hand movements. Most people with typical mucolipidosis type IV are unable to walk independently. In about 15 percent of affected individuals, the psychomotor problems worsen over time.

Vision may be normal at birth in people with typical mucolipidosis type IV, however, it typically becomes increasingly impaired during the first decade of life. Individuals with this condition typically develop clouding cornea and progressive breakdown of the retina. By their early teens, affected individuals often have severe vision loss or blindness.

People with typical mucolipidosis type IV also typically have impaired production of stomach acid (achlorhydria). Achlorhydria does not cause any symptoms in these individuals, but it does result in unusually high levels of gastrin in the blood. Individuals with mucolipidosis type IV may not have enough iron in their blood, which can lead to anemia. People with the severe form of this disorder usually survive to adulthood, however, they may have a shortened lifespan.

About 5 percent of affected individuals have atypical mucolipidosis type IV. These individuals usually have mild psychomotor delay and may develop the ability to walk . People with atypical mucolipidosis type IV tend to have milder eye abnormalities than those with the severe form of the disorder. Achlorhydria also may be present in mildly affected individuals.

In certain embodiments the prophylactic and/or therapeutic methods described herein involve ameliorating one or more of the above symptoms (e.g., one or more symptoms selected from the group consisting of delayed development, vision impairment, psychomotor delay, cognitive impairment, limited or absent speech, difficulty chewing and swallowing, weak muscle tone (hypotonia), abnormal muscle stiffness (spasticity), locomotor impairment, clouding of the cornea, and impaired production of stomach acid (achlorhydria)), and/or delaying the onset, slowing, stopping, or reversing the progression of one or more of these symptoms.

### Multiple sulfatase deficiency (MSD).

Multiple sulfatase deficiency is a condition that mainly affects the brain, skin, and skeleton. Because the signs and symptoms of multiple sulfatase deficiency vary widely, the condition has been "split" into three types: neonatal, late-infantile, and juvenile.

The neonatal type is the most severe form, with signs and symptoms appearing soon after birth. Affected individuals typically have deterioration of tissue in the nervous system (leukodystrophy), that can contribute to movement problems, seizures, developmental delay, and slow growth. Skeletal abnormalities can include scoliosis, joint stiffness, and dysostosis multiplex. Affected individuals may also have hearing loss, heart malformations, and an enlarged liver and spleen(hepatosplenomegaly). Many of the signs and symptoms of neonatal multiple sulfatase deficiency worsen over time.

The late-infantile type is the most common form of multiple sulfatase deficiency. It is typically characterized by normal cognitive development in early childhood followed by a progressive loss of mental abilities and movement (psychomotor regression) due to leukodystrophy or other brain abnormalities. Individuals with this form of the condition do not have as many features as those with the neonatal type, but they often have ichthyosis, skeletal abnormalities, and coarse facial features.

The juvenile type is the rarest form of multiple sulfatase deficiency. Signs and symptoms of the juvenile type typically appear in mid- to late childhood. Affected individuals have normal early cognitive development but then experience psychomotor regression, however, the regression in the juvenile type usually occurs at a slower rate than in the late-infantile type. Ichthyosis is also common in the juvenile type of multiple sulfatase deficiency.

Life expectancy is shortened in individuals with all types of multiple sulfatase deficiency. Typically, affected individuals survive only a few years after the signs and symptoms of the condition appear, but life expectancy varies depending on the severity of the condition and how quickly the neurological problems worsen.

In certain embodiments the prophylactic and/or therapeutic methods described herein involve ameliorating one or more of the above symptoms (*e.g*., one or more symptoms selected from the group consisting of leukodystrophy, scoliosis, hepatosplenomegaly, psychomotor regression), and ichthyosis), and/or delaying the onset, slowing, stopping, or reversing the progression of one or more of these symptoms.

The foregoing lysosomal storage diseases are illustrative and non-limiting. Using the teachings provided herein, one of skill in the art can readily utilize molecular tweezers in the treatment and/or prophylaxis of numerous other LSDs.

### Molecular tweezers for treatment of lipofuscin-related disorders.

In various embodiments the compounds described herein (*e.g.*, molecular tweezers) (*see, e.g.,* Compounds 2-19 in Figures 5 and 6) are administered to a subject (*e.g.,* to a mammal in need thereof) for the treatment or prophylaxis of a lipofuscin-related disorder. In certain embodiments the lipofuscin-related disorder comprises an ocular disease that is a lipofuscin-related disorder. In certain embodiments the lipofuscin-related disorder comprises a lipofuscinosis.

In certain embodiments the lipofuscin-related disorder comprises a disorder such as a lipofuscin-related disorder associated with an eye disease, a neuronal ceroid lipofuscinosis (*e.g*. Batten disease), acromegaly, amyotrophic lateral sclerosis, denervation atrophy, lipid myopathy, chronic obstructive pulmonary disease, centronuclear myopathy, melanosis coli, and the like. In certain embodiments the lipofuscin-related disorder is a lipofuscin-related disorder associated with an eye disease (*e.g.*, age-related macular degeneration, vitelliform macular degeneration (Best's macular dystrophy), retinal pigment epitheliopathy associated with choroidal melanoma, severe ocular trauma, and the like).

### Age-Related Macular Degeneration.

Age-related macular degeneration (AMD) is the most common cause of irreversible central vision loss in elderly patients. Dilated funduscopic findings are diagnostic; color photographs, fluorescein angiography, and optical coherence tomography assist in confirming the diagnosis and in directing treatment. AMD is the leading cause of permanent, irreversible vision loss in the elderly. Age related macular degeneration occurs in two forms: 1) Dry (nonexudative or atrophic); and 2) Wet (exudative or neovascular).

Dry AMD causes changes of the retinal pigment epithelium, typically visible as dark pinpoint areas. The retinal pigment epithelium plays a critical role in keeping the cones and rods healthy and functioning well. Accumulation of waste products from the rods and cones can result in drusen, which appear as yellow spots. Areas of chorioretinal atrophy (referred to as geographic atrophy) occur in more advanced cases of dry AMD. There is no elevated macular scar (disciform scar), edema, hemorrhage, or exudation.

Wet AMD occurs when new abnormal blood vessels develop under the retina in a process called choroidal neovascularization (abnormal new vessel formation). Localized macular edema or hemorrhage may elevate an area of the macula or cause a localized retinal pigment epithelial detachment. Eventually, untreated neovascularization causes a disciform scar under the macula.

In Dry AMD, the loss of central vision occurs over years and is painless, and most patients retain enough vision to read and drive. Central blind spots (scotomas) usually occur late in the disease and can sometimes become severe. Symptoms are usually bilateral. Funduscopic changes include, but are not limited to changes in the retinal pigment epithelium, Drusen, and areas of chorioretinal atrophy.

In wet AMD rapid vision loss occurs usually over days to week. The first symptom is usually visual distortion, such as a central blind spot (scotoma) or curving of straight lines (metamorphopsia). P eripheral vision and color vision are generally unaffected; however, the patient may become legally blind (< 20/200 vision) in the affected eye, particularly if AMD is not treated. W et AMD usually affects one eye at a time; thus, symptoms of wet AMD are often unilateral. Funduscopic changes include, but are not limited to subretinal fluid appearing as localized retinal elevation, retinal edema, gray-green discoloration under the macula, exudates in or around the macula, detachment of retinal pigment epithelium (visible as an area of retinal elevation), subretinal hemorrhage in or around the macula, and the like.

In certain embodiments the prophylactic and/or therapeutic methods described herein involve ameliorating one or more of the above symptoms (*e.g*., one or more symptoms selected from the group consisting of drusen or waste deposits on the surface of the retina, changes in color (pigment) of the macula, blurred or fuzzy vision, the illusion that straight lines are wavy; the illusion that some objects are smaller than they really are, the appearance of a gray, dark or empty area in the center of the visual field, and fading of color vision), and/or delaying the onset, slowing, stopping, or reversing the progression of one or more of these symptoms.

### Vitelliform macular degeneration

Vitelliform macular degeneration (a.k.a., vitelliform macular dystrophy) is a genetic eye disorder that can cause progressive vision loss. This disorder affects the retina. Specifically, vitelliform macular dystrophy disrupts cells in a small area near the center of the retina called the macula. Vitelliform macular dystrophy causes lipofuscin to build up in cells underlying the macula. Over time, the abnormal accumulation of this substance can damage cells that are critical for clear central vision. As a result, people with this disorder often lose their central vision, and their eyesight may become blurry or distorted. Vitelliform macular dystrophy typically does not affect side (peripheral) vision or the ability to see at night.

Two forms of vitelliform macular dystrophy have been described with similar features. The early-onset form (known as Best disease) usually appears in childhood; the onset of symptoms and the severity of vision loss vary widely. The adultonset form begins later, usually in mid-adulthood, and tends to cause vision loss that worsens slowly over time. The two forms of vitelliform macular dystrophy each have characteristic changes in the macula that can be detected during an eye examination.

In certain embodiments the prophylactic and/or therapeutic methods described herein involve ameliorating one or more of the above symptoms (*e.g*., one or more symptoms selected from the group consisting of central vision loss, blurry eyesight, distorted eyesight, macular cell death), and/or delaying the onset, slowing, stopping, or reversing the progression of one or more of these symptoms.

### Stargardt disease

Stargardt disease is the most common inherited retinal disease. It usually has an autosomal recessive inheritance caused by mutations in the *ABCA4* gene. Rarely it has an autosomal dominant inheritance due to defects with *ELOVL4* or *PROMI* genes. It is characterized by macular degeneration that begins in childhood, adolescence or adulthood, resulting in progressive loss of vision.

In STGD1, the genetic defect causes malfunction of the ATP-binding cassette transporter (ABCA4) protein of the visual phototransduction cycle. Defective ABCA4 leads to improper shuttling of vitamin A throughout the retina, and accelerated formation of toxic vitamin A dimers (also known as bisretinoids), and associated degradation byproducts. Vitamin A dimers and other byproducts are widely accepted as the cause of STGD1. In STGD4, a butterfly pattern of dystrophy is caused by mutations in a gene that encodes a membrane bound protein that is involved in the elongation of very long chain fatty acids.

In certain embodiments the prophylactic and/or therapeutic methods described herein involve ameliorating one or more of the above symptoms (*e.g*., one or more symptoms selected from the group consisting of high levels of vitamin dimers and byproducts thereof, blurry or distorted vision, inability to see in low lighting, difficulty recognizing familiar faces, and loss of color vision), and/or delaying the onset, slowing, stopping, or reversing the progression of one or more of these symptoms.

### Neuronal ceroid lipofuscinoses

In certain embodiments the molecular tweezers described herein are used in the treatment and/or prophylaxis of a neuronal ceroid lipofuscinosis. Illustrative, but non-limiting examples of neuronal ceroid lipofuscinoses include, but are not limited to, infantile NCL (Santavuori-Haltia disease), late infantile NCL (Jansky-Bielschowsky disease, Juvenile NCL (CLN1, Batten disease), adult NCL (Kufs disease), Finnish Late Infantile NCL, Variant Late Infantile NCL, CLN7 NCL, CLN8 NCL (Northern Epilepsy, progressive epilepsy with mental retardation (EPMR)), Turkish Late Infantile Variant NCL, and CLN10 NCL (Congenital, Cathepsin D Deficiency).

Neuronal ceroid lipofuscinoses (NCLs) are typically characterized by the progressive, permanent loss of motor and psychological ability with a severe intracellular accumulation of lipofuscins,. There are four classic diagnoses that have received the most attention from researchers and the medical field, differentiated from one another by age of symptomatic onset, duration, early-onset manifestations such as blindness or seizures, and the forms which lipofuscin accumulation takes.

In the early infantile variant of NCL (also called INCL or Santavuori-Haltia), probands appear normal at birth, but early visual loss leading to complete retinal blindness by the age of 2 years is the first indicator of the disease; by 3 years of age a vegetative state is reached and by 4 years isoelectric encephalograms confirm brain death. Late infantile variant usually manifests between 2 and 4 years of age with seizures and deterioration of vision. The maximum age before death for late infantile variant is 10-12 years. Juvenile NCL (JNCL, Batten Disease, or Spielmeyer-Vogt), with a prevalence of 1 in 100,000, usually arises between 4 and 10 years of age; the first symptoms include considerable vision loss due to retinal dystrophy, with seizures, psychological degeneration, and eventual death in the mid- to late-20s or 30s ensuing. Adult variant NCL (ANCL or Kuf's Disease) is less understood and generally manifests milder symptoms; however, while symptoms typically appear around 30 years of age, death usually occurs ten years later.

In certain embodiments the prophylactic and/or therapeutic methods described herein involve ameliorating one or more of the above symptoms (*e.g.*, one or more symptoms selected from the group consisting of an amelioration of one or more symptoms selected from the group consisting of cognitive dysfunction, movement/locomotor dysfunction, and vision loss), and/or delaying the onset, slowing, stopping, or reversing the progression of one or more of these symptoms.

The foregoing lipofuscin-related disorders are illustrative and non-limiting. Using the teachings provided herein, one of skill in the art can readily utilize molecular tweezers in the treatment and/or prophylaxis of numerous other lipofuscin-related disorders.

The foregoing methods of using the compounds described herein for inhibiting a virus are illustrative and non-limiting. Using the teachings provided therein the compounds can be used for inhibiting other viruses and/or in other contexts as will be recognized by one of skill in the art.

### EXAMPLES

The following examples are offered to illustrate, but not to limit the claimed invention. Compounds 1, 12-17 are not according to the invention.

### Example 1: Synthesis of Symmetrical Dialkyl Diphosphates 1-11.

These molecules are prepared from the published diacetoxy tweezer precursor (Scheme 1): deacetylation with lithium aluminum hydride, followed by phosphorylation with phosphorus oxychloride and subsequent aqueous hydrolysis leads to the parent tweezer, CLR01 (Talbiersky et al. (2008) J. Am. Chem. Soc. 130(30): 9824-9828; Sinha et al. (2011) J. Am. Chem. Soc. 133(42): 16958-16969), as the diphosphoric acid. Treatment of this key intermediate with a large excess of trichloroacetonitrile (>10 equivs.), followed by an equimolar amount of the aliphatic alcohol (also >10 equivs.), and subsequent heating to 90°C for 1 day gives complete conversion to the bis(monoesters) in high yields (80-96%). This reaction only works in the presence of excess pyridine, best used as the solvent. An optimized workup procedure removes unreacted reagents, so that after chromatographic separation, the dialkyl diphosphates are isolated in analytically pure form. Remarkably, no second esterification occurs on each phosphate group, rendering this process highly specific for monoalkylation on both sides. A large variety of primary and secondary aliphatic alcohols can be subjected to this P-esterification, even with carbon tails as long as C₁₈. Double and triple bonds are accepted and are not rearranged.

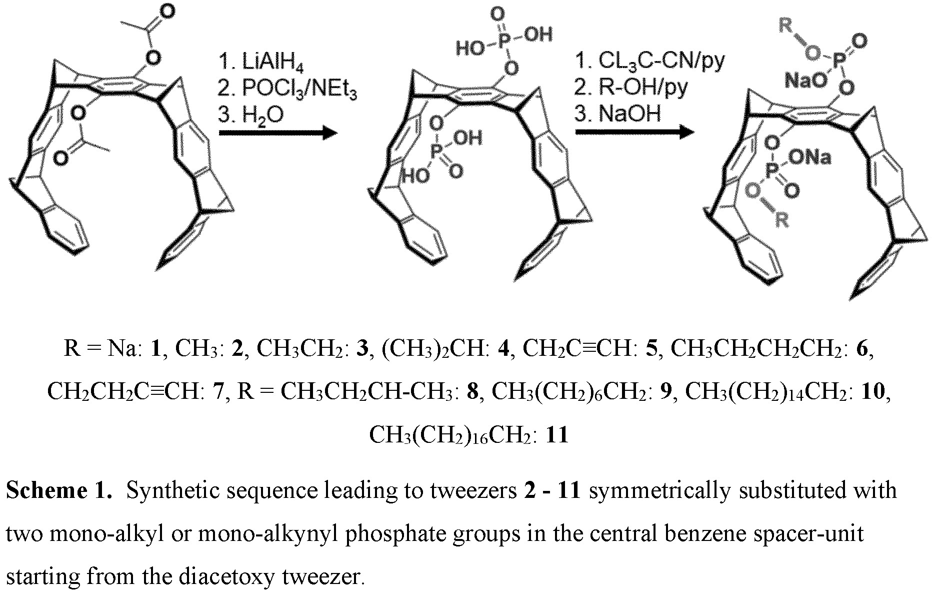

### Example 2: Unsymmetrical Diphosphate Monoester 12.

For the introduction of only one additional recognition unit a more elaborate synthesis is necessary, which again starts from the diacetoxy tweezer as a precursor **(Scheme 2)**. Selective hydrolysis of one acetyl group leading to the tweezer substituted by one hydroxyl and one acetoxy group in the central benzene spacer-unit is followed by mono-phosphorylation with POCl₃. After aqueous work-up, the free phosphoric acid is activated by excess trichloroacetonitrile and esterified with equimolar amounts of the primary or secondary alcohol. Subsequent hydrolysis of the remaining acetyl group is followed by a second phosphorylation step with POCl₃. Final neutralization affords the water-soluble unsymmetrically substituted diphosphate tweezers (12) with only one alkyl arm in the central benzene spacer-unit. The second phosphorylation must be carried out under rigorous exclusion of humidity and with more than 3 equivs. of triethylamine base. The resulting product is purified over silica gel (reverse-phase) and stored at -18 °C. Final neutralization with NaOH affords water-soluble material as the trianion (pH 7).

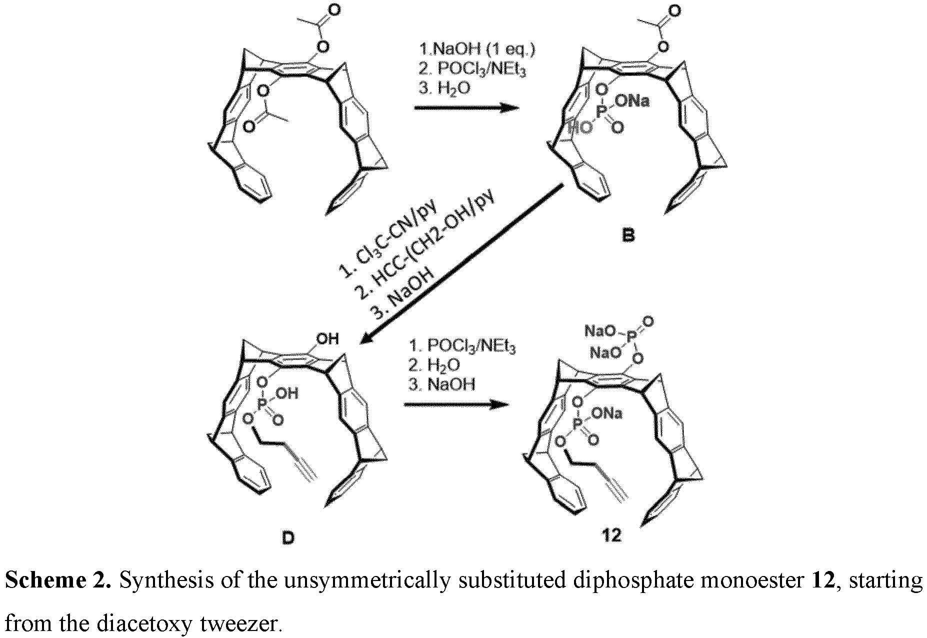

### Example 3: The Phosphoramidite Method

A totally different approach to unsymmetrical diphosphate monoesters was discovered when phosphate units were introduced as phosphoramidites (P-III), which could be later oxidized to the P-V oxidation state. This method starts with the diacetoxy tweezer (**Scheme 3**): Partial hydrolysis followed by phosphorylation with POCl₃ and final esterification with an excess of methanol leads to the precursor **E.** This can be treated with diisopropylamino-chloro-methyl-phosphoramidite and furnishes the mixed P-V/P-III species **F** as a key intermediate. Subsequent addition of an equimolar mixture of tetrazole and butynol activates the phosphoramidite and smoothly introduces the butynyl ester group. The resulting phosphite can be quantitatively oxidized to the phosphate (**G**) by t-butyl hydroperoxide.

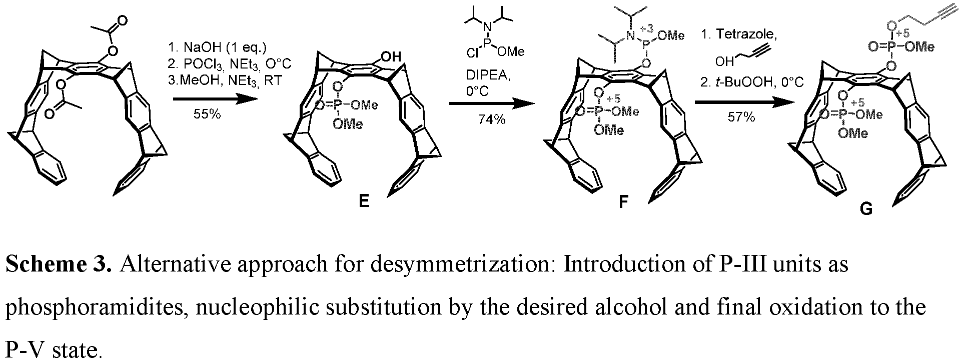

Even better is the use of cyanoethyl esters which can be finally cleaved very mildly (**Scheme 4**). The diacetoxy tweezer is thus reduced by lithium aluminum hydride leading to the tweezer substituted with two hydroxyl groups in the central benzene spacer-unit. Reaction of this tweezer with diisopropylamino-chloro-cyanoethyl phosphoramidite gives rise to a 1:1 mixture of mono- and diphosphorylated phosphoramidite **H** and **I**. Treatment of **I** alone or of the **H/I** mixture with 1:1 butynol/tetrazole affords the corresponding phosphite, which is best oxidized with hydrogen peroxide to give the mixed butynyl/cyanoethyl phosphate ester **J**. After complete deprotonation of the phenolic OH in **J** with BuLi, the second phosphorylation can be initiated with equimolar amounts of dimethylamino-dichloro-phosphoramidate. Smooth transition occurs to a mixed P-V substituted molecular tweezer (**K**). Final deprotection only requires heating to 60 °C for 1.5 h and a short treatment with aqueous ammonia. It furnishes pure monosubstituted molecular tweezer **7** as free phosphoric acid, which can be neutralized with 3 equiv. of NaOH to yield the corresponding water-soluble sodium phosphate. Mild hydrolysis can also be effected on **J**, leading to the respective monobutynyl monophosphate tweezer **M.**

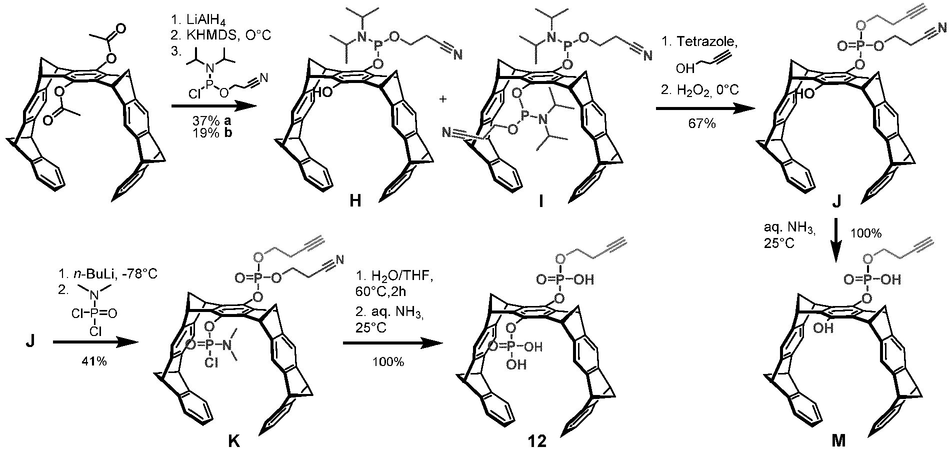

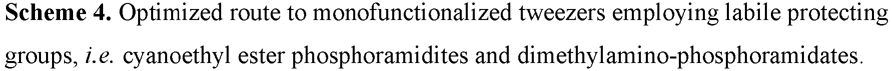

### Example 4: Aqueous solubility and Affinity of the new tweezers 2 - 13 for lysine and arginine.

All new tweezer derivatives are well soluble in aqueous buffers; only dialkyl derivatives with C₈ esters or longer drop below 1 mM solubility.

Fluorescence titrations of tweezers **5, 7,** and **12** (c = 25 µM) substituted by two or one alkynyl phosphate groups with basic amino acid derivatives furnish dissociation constants in the low micromolar regime, equal to or better than those determined for the phosphate tweezer **1.** Surprisingly, the octyl ester **9** furnished the highest lysine and arginine affinity ever observed for a molecular tweezer (*K*_{d} ~ 1-5 µM); these values were independently confirmed by ITC titrations (confirmed K*_{d}* ~ 13 µM-ITC: c = 0.1 mM). Further investigations were conducted with representative bioactive short peptides and furnished high affinities, with dioctyl diester, again one order of magnitude higher than the parent tweezer **1** (**Table 3**).

**Table 3. Dissociation constants K_{d} [µM] of the mono- and dialkyl tweezer diphosphates with basic amino acids and peptides determined by fluorometric titration in aqueous buffer at neutral pH**

| **No.** | **R** | **Yield [%]¹** | **Lys** | **Arg** | **KAA** | **KLVFF** | **RGD** | **ITC²** |
|---|---|---|---|---|---|---|---|---|
| **1** | Na³ | **95** | **9** | **20** | **30** | **20** | **86** | **15** |
| **2** | Methyl | 90 | 52 | 60 | 160 | 59 | 73 | 155 |
| **5** | Propargyl | 60 | 6 | 16 | 13 | 10 | 72 | 24 |
| **7** | Butynyl | 89 | 6 | 8 | 6 | n. d. | 19 | 50 |
| **9** | Octyl | 85 | **1** | **5** | **1** | n.d. | **1** | **13** |
| **12** | Monobutynyl | 30 | 10 | 44 | 20 | 14 | 60 | 61 |
| ¹ from diacetoxy tweezer; ²Ac-Lys-OMe; ³ in aqueous buffer the phosphate groups in tweezer **1** are partially protonated. | | | | | | | | |

### Example 5: Stability against hydrolysis by acid, base or enzymes.

The new dialkyl tweezers do not suffer from hydrolytic cleavage by pH changes or enzymatic degradation in the course of *in-vitro* experiments. In an ¹H-NMR experiment, 0.1 mM samples of the diethyl tweezer **3** were treated with 1M NaOD (pH 14, 3h) or 10% aq. DCl (pH ~0, 16h), and ¹H NMR spectra were measured at regular time intervals during a full day. Even after 24 h, there was no decrease of the ester alcohol signal intensity nor did any new signal appear in the ¹H or ³¹P NMR spectrum. Likewise, a standard phosphatase assay (alkaline phosphatase, borate buffer pH 9.8, 37 °C) did not produce any trace of cleavage product (Her et al. (2015) J. Chem. Ed. 92(11): 1943-1948). Similarly, incubation of **3** with mouse plasma, mouse liver microsomes, or simulated gastric fluid for 1 h with analysis of the mixture by LC-MS/MS every 15 min showed no evidence of hydrolysis, whereas the positive control, diclofenac, was hydrolyzed quantitatively under the same conditions.

### Example 6: Pharmacological characteristics.

For high bioavailability, drugs must cross cell membranes and if the target is in the central nervous system, they must also penetrate through the blood brain barrier (BBB). For cell membrane permeability the hydrophobicity of a drug is very important, often approximated by the octanol/water partition coefficient, logP(o/w). Established parameters for estimated BBB passage are logBB (the concentration of drug in the brain divided by concentration in the blood) and log PS (permeability surface-area product). Calculated values for both logP(o/w) and logBB of the new tweezer derivatives are given in **Table 4.** From all logP(o/w) values, tweezers with extended and branched alkyl esters are expected to penetrate cell membranes much more easily than the parent compound CLR01. It was shown that molecules with logBB > 0.3 cross the BBB readily while molecules with logBB < -1 are poorly distributed to the brain. Remarkably, short and branched alkyl esters on molecular tweezers are optimal for BBB permeability, whereas the polar CLR01, but also the strongly hydrophobic hexa- and octadecyl esters are expected to have much lower BBB penetration, most likely due to their sheer size.

**Table 4. LogP(o/w) and logBB values of CLR01 and the new dialkyl diphosphate tweezers calculated with the Schrödinger software Qikprop. (Schrödinger Release 2017-3: QikProp, Schrödinger, LLC, New York, NY, 2017.)**

| **Compound No.** | **R** | **logP (o/w)** | **logBB** |
|---|---|---|---|
| **1** | Na^{g} | 7.31 | -1.71 |
| **2** | Methyl | 7.84 | -1.27 |
| **3** | Ethyl | 8.75 | **-0.83** |
| **4** | *i*-Propyl | 9.43 | **-0.77** |
| **5** | Propargyl | 8.77 | **-0.99** |
| **6** | Butyl | 9.73 | -1.6 |
| **7** | Butynyl | 9.68 | -1.38 |
| **8** | *sec-Butyl* | 10.02 | -1.14 |
| **9** | Octyl | 12.96 | -1.41 |
| **10** | Hexadecyl | **19.06** | -3.79 |
| **11** | Octadecyl | **20.47** | -3.22 |
| **12** | Monobutynyl | 8.70 | -2.07 |

### Example 7: Unsymmetrical Monoalkoxy-monophosphates 13-17.

Monophosphate tweezers have a lower molecular weight and decreased overall polarity, advantageous for uptake and BBB passage. They became accessible via direct alkylation of the monoacetoxy tweezer. Thus, monomethylation with methyl iodide, followed by phosphorylation with POCl₃, hydrolysis and neutralization affords the short methyl ether derivative **14** (**Scheme 5**) (Dutt et al. (2013) Eur. J. Org. Chem. 2013(34): 7705-7714). A related sequence is currently used for selective ethylation (**15**) as well as trifluoroethylation (**16**). A different protocol for trifluoromethylation leads to the short and lipophilic trifluoromethoxy derivative **17,** using the procedure published by Liu et al in the first step (Liu et al. (2015) Angew. Chem. Int. Ed. Engl. 54(40): 11839-11842).

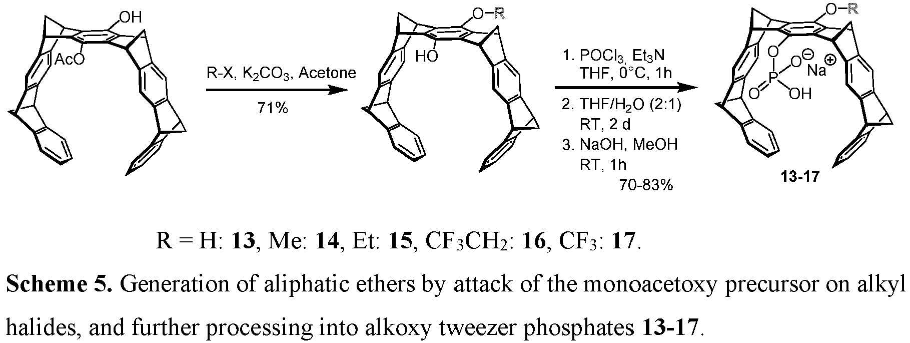

### Example 8: Synthesis of Tetraesters 18-19 (Prodrugs).

The attachment of a total of four alkyl substituents, two on both phosphates, renders the tweezers much more lipophilic, with the expectation that they will pass more easily through membranes and thus favors BBB penetration. Despite their apparent stability against alkaline phosphatase, they were surprisingly found to be cleaved *in vivo* (in mice) and release the active compound **1** (CLR01), which can be detected by LC-MS/MS. The tetraesters are difficult to make because even the dichlorophosphoryl intermediate obtained from POCl₃ treatment is sterically so well shielded, that only moderate yields are furnished by direct alcoholysis (**Scheme 6,** 5-10%). A number of alternative routes with 2 or more steps failed to produce the desired tetraesters.

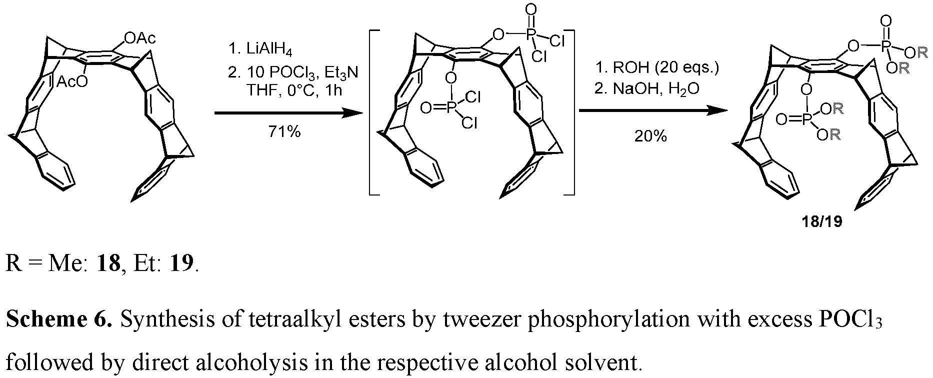

### Example 9: Evaluation of the toxicity of new molecular tweezers in cell culture and their ability to protect cultured cells against the toxicity of amyloid β-protein.

The new derivatives included in the experiment described below were **13** and **14,** in which one phosphate group was replaced by a hydroxyl or a methoxy, respectively; the symmetric diesters, **3-9,** in which each phosphate group was protected by ethyl, isopropyl, propargyl, n-butyl, butinyl, sec-butyl, or octyl, groups, respectively; and two tetraesters, **18** and **19,** in which both anionic oxygens of each phosphate group were protected by methyl or ethyl groups, respectively.

Derivatives **3-9, 13** and **14** were evaluated first for toxicity in differentiated rat pheochromocytoma (PC-12) cells using the MTT and/or LDH assays, as described previously (Sinha et al. (2011) J. Am. Chem. Soc. 133(42): 16958-16969; Fradinger et al. (2008) Proc. Natl. Acad. Sci. USA, 105(37): 14175-14180). Tetraesters **18** and **19** were not tested because they were expected to act as pro-drugs only and not have activity themselves. The highest concentration tested was 100 µM. Non-toxic compounds then were tested for their ability to inhibit the toxicity of 10 µM Aβ42 in the same assay. The parent compound **1** was used as a control in both cases. The results are summarized in **Table** 5. The data suggest that esters containing ≥4 carbons tend to be too toxic. Surprisingly, three compounds, **3, 4,** and **13** had higher protective effect against Aβ42-induced toxicity compared to **1.**

**Table 5. Characterization of new tweezers in cell culture.**

| **Compound** | **Self-toxicity** | **Inhibition of Aβ42 toxicity** |
|---|---|---|
| **1** | Non-toxic | IC₅₀ = 69 µM (MTT) |
| **3** | Non-toxic | IC₅₀ = 42 µM (LDH), IC₅₀ = 13 µM (MTT) |
| **4** | Non-toxic | IC₅₀ = 28 µM (LDH) |
| **5** | Non-toxic | - |
| **6** | Toxic at 100 µM | Inactive at 30 µM |
| **7** | Toxic at 30 µM | Not tested |
| **8** | Toxic at 30 µM | Not tested |
| **9** | Toxic at 30 µM | Not tested |
| **13** | Non-toxic | IC₅₀ = 16 µM (LDH) |
| **14** | Toxic at 100 µM | - |

In view of these results, compounds **3, 4, 13, 14, 18,** and **19** were screened for PK behavior in three routes of administration and two time points in 8-10-weeks old, male, wild-type mice. Each compound was administered to the mice at 2 mg/kg intravenously (I.V.), 2 mg/kg subcutaneously (S.C.), or 10 mg/kg orally (P.O.). Forty minutes or four hours post-administration, the mice were sacrificed, blood and brain were collected and homogenized, and concentration of the active compound **1** in the case of prodrugs **3, 4, 18,** or **19,** or of the administered compounds themselves in the case of **14** and **15** was measured using HPLC-MS/MS.

The results of the PK experiments are summarized in **Tables** 6-8. Encouragingly, compound **14** had substantially increased the oral bioavailability compared to **1.** Compound **14** also had substantially increased BBB penetration when administered I.V.

Pro-drug **3** had increased BBB penetration in all three routes of administration compared to the parent compound **1.** Pro-drug **4** also showed improvement relative to **1.** Pro-drug **19 led** to an even stronger increase in BBB penetration.

**Table 6. PK screening of new molecular tweezers derivatives administered I.V.**

| **Compound** | **Blood** | | **Brain** | | | |
|---|---|---|---|---|---|---|
| | **Concentration (ng/mL)¹** | | **Concentration (ng/g)** | | **BBB penetration (%)²** | |
| | 40 min | 4 h | 40 min | 4h | 40 min | 4 h |
| **1** | 1712 | 298 | 0.57 | 0.48 | 0.0 | 0.2 |
| **3** | 4.2 | 0 | 0.69 | 0.39 | 16.4 | >0 |
| **4** | 25.6 | 0 | 0.00 | 0.14 | 0.0 | >0 |
| **13** | 55 | 53 | 3.58 | 1.28 | 6.5 | 2.4 |
| **14** | 243 | 34 | 24.3 | 22.4 | 10.0 | 65.9 |
| **18** | 0 | 0 | 0.13 | 0.00 | 0.0 | 0.0 |
| **19** | 0 | 6.2 | 0.11 | 2.71 | 0.0 | 43.7 |
| ¹Represents the parent compound in the case of 13 and 14, and compound 1 in all other cases. ²Calculated as the percent ratio between the brain concentration and blood concentration. | | | | | | |

**Table 7. PK screening of new molecular tweezers derivatives administered S.C.**

| **Compound** | **Blood** | | | | **Brain** | | | |
|---|---|---|---|---|---|---|---|---|
| | **Concentration (ng/mL)¹** | | **Bioavailability (%)³** | | **Concentration (ng/g)** | | **BBB penetration (%)²** | |
| | **40 min** | **4 h** | **40 min** | **4 h** | **40 min** | **4 h** | **40 min** | **4 h** |
| **1** | 1727 | 258 | 100.9 | 86.6 | 0.72 | 0.00 | 0.0 | 0.0 |
| **3** | 0.8 | 0 | 20.0 | 0.0 | 0.35 | 0.22 | 41.7 | >0 |
| **4** | 15.7 | 0 | 61.2 | 0.0 | 0.00 | 0.00 | 0.0 | 0.0 |
| **13** | 98 | 50 | 178.2 | 94.3 | 1.19 | 1.69 | 1.2 | 3.4 |
| **14** | 683 | 99 | 281.1 | 291.2 | 0.37 | 0.41 | 0.1 | 0.4 |
| **18** | 0 | 0 | 0.0 | 0.0 | 0.34 | 0.00 | >0 | 0.0 |
| **19** | 0 | 1.6 | 0.0 | 26.5 | 0.00 | 2.82 | >>00 | 172.0 |
| ¹Represents the parent compound in the case of 13 and 14, and compound 1 in all other cases. ²Calculated as the percent ratio between the brain concentration and blood concentration. ³Calculated as the percent ratio of the I.V. blood concentration | | | | | | | | |

**Table 8. PK screening of new molecular tweezers derivatives administered P.O.**

| **Compound** | **Blood** | | | | **Brain** | | | |
|---|---|---|---|---|---|---|---|---|
| | **Concentration (ng/mL)¹** | | **Bioavailability (%)³** | | **Concentration (ng/g)** | | **BBB penetration (%)²** | |
| | **40 min** | **4 h** | **40 min** | **4 h** | **40 min** | **4 h** | **40 min** | **4 h** |
| **1** | 7.8 | 0.0 | 0.5 | 0.0 | 0.00 | 0.00 | 0.0 | 0.0 |
| **3** | 0.0 | 0.0 | 0.0 | 0.0 | 0.36 | 0.31 | >0 | >0 |
| **4** | 0.0 | 0.0 | 0.0 | 0.0 | 0.07 | 0.48 | >0 | >0 |
| **13** | 0.0 | 0.3 | 0.0 | 0.5 | 1.79 | 0.26 | 0.0 | 100 |
| **14** | 9.5 | 6.9 | 3.9 | 20.3 | 0.58 | 0.18 | 6.1 | 2.6 |
| **18** | 0.0 | 0.0 | 0.0 | 0.0 | 0.25 | 0.00 | >0 | 0.0 |
| **19** | NA | NA | NA | NA | NA | NA | NA | NA |
| ¹Represents the parent compound in the case of 13 and 14, and compound 1 in all other cases. ²Calculated as the percent ratio between the brain concentration and blood concentration. ³Calculated as the percent ratio of the I.V. blood concentration | | | | | | | | |

Based on the 2-time-point screen results, a full 24-h PK profile has been obtained for compounds **13** and **14.** In these experiments, the blood and brain levels were measured at 7 time points - 0.3, 0.6, 1, 2, 4, 8, and 24 h. The results are summarized in Tables 9-11. Data for **1** are given for reference.

**Table 9. PK analysis of compounds 13 and 14 administered I.V.**

| **Compound** | **Blood** | | **Brain** | | |
|---|---|---|---|---|---|
| | **T_{1/2} (h)** | **AUC** | **T_{1/2} (h)** | **AUC** | **LogBB** |
| **1** | 1.8 | 6,804 | 4.0 | 1.63 | -3.62 |
| **13** | 1.2 | 423 | 1.5 | 3.71 | -2.06 |
| **14** | 1.7 | 1880 | 0.8 | 73.2 | -1.41 |

**Table 10. PK analysis of compounds 13 and 14 administered S.C.**

| **Compound** | **Blood** | | | **Brain** | | |
|---|---|---|---|---|---|---|
| | **T_{1/2} (h)** | **AUC** | **Bioavailability (%)** | **T_{1/2} (h)** | **AUC** | **LogBB** |
| **1** | 1.05 | 4744 | 69.7 | 0.9 | 1.3 | 0.34 |
| **13** | 11.1 | 1043 | 246.7 | 1.1 | 16 | 3.31 |
| **14** | 14.8 | 1147 | 61.0 | NA | 4.4 | 0 |

**Table 11. PK analysis of compounds 13 and 14 administered P.O.**

| | **Blood** | | | **Brain** | | |
|---|---|---|---|---|---|---|
| **Compound** | **T_{1/2} (h)** | **AUC** | **Bioavailability (%)** | **T_{1/2} (h)** | **AUC** | **LogBB** |
| **1** | 1.3 | 8.94 | 0.1 | NA | 0 | NA |
| **13** | 16 | 91.2 | 21.6 | 3.1 | 1.33 | -1.84 |
| **14** | 4.4 | 16.1 | 0.9 | 6.5 | 13.7 | -0.07 |

The data demonstrate clearly that both compounds **13** and **14** have improved oral bioavailability and BBB penetration relative to compound **1.**

### Example 10: Evaluation of the antiviral properties of the new tweezer derivatives.

Recently, it was discovered that the molecular tweezer CLR01 (1) has antiviral properties (Lump et al. (2015) eLife, 4: e05397; Röcker et al. (2018) Antiviral Res. 152: 26-35). The concentrations of **1** required to inhibit enveloped viruses such as HIV-1 and HSV-2 are in the median micromolar range (Lump et al. (2015) eLife, 4: e05397). The antiviral activity against HIV-1 and likely other enveloped viruses is mediated by interacting with the head groups of lipids that are enriched in the viral membrane as compared to the cellular membrane. This interaction causes mechanical stress and results in a loss of viral membrane integrity, which is essential for viral infectivity. Attachment of hydrophobic chains to the phosphate groups of **1** was predicted to enhance membrane insertion and hence viral inactivation.

The new derivatives included in the study of viral activation were **2-7** and **9-11.**

### Cell viability assays

To determine the maximally tolerated concentrations of these derivatives in cell culture and to exclude cytotoxic effects in viral infectivity assays, a CellTiter-Glo^{®} assay (Promega) was performed. This assay quantifies the amount of intracellular ATP. The compounds were directly added to TZM-bl cells (a HeLa derived HIV-1 reporter cell line TZM-bl; 20,000 cells/well, FIG. 1, panel A) or Vero E6 cells (a Zika virus (= ZIKV) permissive cell line; 12,000 cells/well, FIG. 1, panel B). Cell viability was quantified two days later by measuring ATP levels. Half-maximal toxicity values (CC₅₀) from these data (in case of **1:** including additional data) were determined using GraphPad Prism software (see Table 7).

### Infectivity assays

### Zika virus (ZIKV)

The prototypic ZIKV strain MR766 at a MOI (multiplicity of infection) of 2 was incubated with 0.2-150 µM of the different compounds **1-7** and **9-11** or PBS for 10 min at 37 °C, thereafter these mixtures were used to infect 6,000 Vero E6 cells seeded the day before in 96-well plates. After 3-4 days, infection rates were determined via a colorimetric MTT assay that quantitatively determines the ZIKV-induced cytopathic effect (Talbiersky et al. (2008) J. Am. Chem. Soc. 130(30): 9824-9828; Sinha et al. (2011) J. Am. Chem. Soc. 133(42): 16958-16969). 20-µl of MTT solution (5 mg/ml in PBS) were added to 200 µl cells. Following a 3-h incubation at 37°C, cell-free supernatants were discarded and formazan crystals were dissolved in 100 µl of a 1:2 mixture of dimethyl sulfoxide and ethanol. Absorption was measured at 490 nm and baseline corrected at 650 nm using a VMax Kinetic ELISA microplate reader (Molecular Devices). To determine infection rates, sample values were subtracted from untreated control set to 100%. Error bars are standard deviations of replicates (FIG. 2, panels A and B). For compounds **1, 5, 7** and **9,** experiment was performed 3 times in triplicates (FIG. 2, panel A) whereas the other compounds have so far only been tested once in triplicates (FIG. 2, panel B). Using these data, half-maximal inhibitory concentrations (IC₅₀ values), shown in Table 12, were calculated. For compounds **2, 3, 4, 6, 10** and **11,** the mean of the three IC₅₀ values obtained in 3 experiments is displayed. The selectivity index (SI) (Table 12) is calculated for each compound by dividing its CC₅₀ value by the IC₅₀ value.

### HIV-1

A CCR5-tropic HIV-1 strain (20 ng/ml p24 antigen) was incubated with 0.2-150 µM of the different compounds or PBS for 10 min at 37 °C before it was added to 10,000 TZM-bl cells in 180-µl medium seeded in 96-well flat-bottom plates the day before infection. Infection rates were determined 3 days post infection by detecting β-galactosidase activity in cellular lysates using the Tropix Gal-Screen kit (Applied Biosystems) and an Orion microplate luminometer (Berthold). Values represent % infection (mean) compared to buffer control (FIG. 3, panels A and B). For compounds **1, 5, 7** and **9,** experiment was performed 3 times in triplicates (FIG. 3, panel A) whereas the other compounds have so far only been tested once in triplicates (FIG. 3, panel B). Half-maximal inhibitory concentrations (IC₅₀ values), were calculated. For compounds **2, 3, 4, 6, 10** and **11,** the mean of the three IC₅₀ values obtained in 3 experiments is displayed. The selectivity index (SI) is calculated for each compound by dividing its CC₅₀ value by its IC₅₀ value (Table 12).

**Table 12. Half-maximal concentrations for virus inhibition (IC₅₀), half-maximal cytotoxic concentrations (CC₅₀) and selectivity indices (SI) of compounds on Vero E6 cells/ ZIKV infection and TZM-bl cells/ HIV-1 infection. All concentrations are given in µM; n.a., not analyzed.**

| **No.** | **IC₅₀ ZIKV** | **CC₅₀ Vero E6** | **SI (Vero E6/ ZIKV)** | **IC₅₀ HIV-1** | **CC₅₀ TZM-bl** | **SI (TZM-bl/ HIV-1)** |
|---|---|---|---|---|---|---|
| **1** | 46 | 584 | 13 | 37 | 964 | 26 |
| **2** | 13 | n.a. | - | 17 | 367 | 22 |
| **3** | 40 | n.a. | - | 17 | >500 | >29 |
| **4** | 55 | n.a. | - | 17 | 369 | 22 |
| **5** | 33 | 355 | 11 | 23 | 362 | 16 |
| **6** | 22 | n.a. | - | 6 | 347 | 58 |
| **7** | 9 | 330 | 37 | 7 | 357 | 51 |
| **9** | 20 | 316 | 16 | 31 | >500 | >16 |
| **10** | 81 | n.a. | - | 23 | >500 | >22 |
| **11** | 16 | n.a. | - | 50 | 10 | 0.2 |

### Antiviral activity in the presence of serum

The antiviral activity of **1** is inhibited in the presence of serum (Röcker et al. (2018) Antiviral Res. 152: 26-35), limiting its use to non-systemic (*e.g.,* topical) applications. Therefore, the new derivatives were tested to see if they also were so was also inhibited or if some of them can maintain their activity in the presence of serum. The activity of the compounds was investigated in presence of 2.5% human serum. CCR5-tropic HIV-1 NL4-3 strain (20 ng/ml p24 antigen) was incubated with 150 µM of the compounds and a final concentration of 2.5% of human serum for 10 min at 37 °C before the mixtures were added to TZM-bl cells in triplicates and infection rates were determined as described above. Unpaired t-tests were used to compare the buffer control to the 150 µM condition of the different compounds (* denotes p < 0.01 ; ** p < 0.001; *** p < 0.0001 ). Compounds 6 and 7 were more active than 1 and blocked HIV-1 infectivity by around 66% (FIG. 4).

The data demonstrate that some of the new derivatives have a stronger activity and better SI than **1.** In particular compound 7 has an anti-ZIKV SI value of 37, which is ~3-times higher than that of compound **1** (SI = 13). Both compounds **6** and **7** have anti-HIV SI values that are ~2-times higher than that of **1.** These two compounds also maintain their activity to a higher degree than **1** in the presence of serum, which together with their higher activity suggests that they may be useful as systemic anti-viral drugs.

## Claims

1. A compound of formula I or a pharmaceutically acceptable salt thereof: 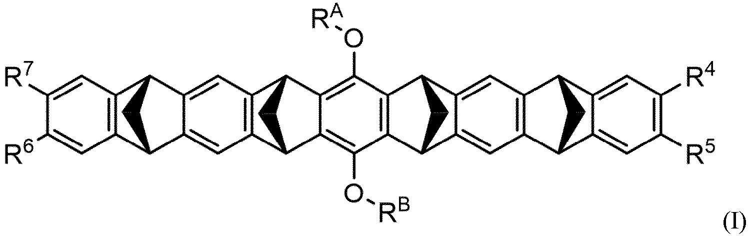 wherein:
R^{A} is 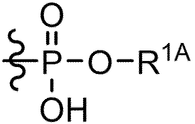 and;
R^{B} is 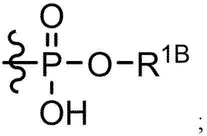 or
R^{A} is 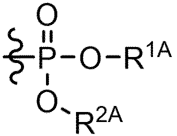 and
R^{B} is 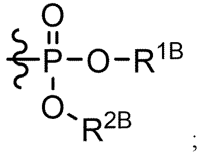 and
R^{1A}, R^{1B}, R^{2A}, R^{2B} are selected from unsubstitued or substituted alkyl, alkenyl, or alkynyl, whereby the substituents are selected from halogen, hydroxyl, carbonyl, thiocarbonyl, alkoxy, phosphoryl, phosphate, phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, aromatic or heteroaromatic moieties which themselves can be substituted;
R⁴ and R⁵ are independently selected from H, halo, alkyl, alkenyl, alkynyl, amino, amide, carboxyl, ester, or nitro; or R⁴ and R⁵, together with the atoms that separate them, complete a cycloalkyl, aryl, or heteroaryl;
R⁶ and R⁷ are independently selected from H, halo, alkyl, alkenyl, alkynyl, amino, amide, carboxyl, ester, or nitro; or R⁶ and R⁷, together with the atoms that separate them, complete a cycloalkyl, aryl, or heteroaryl.

2. The compound of claim 1, having the structure: 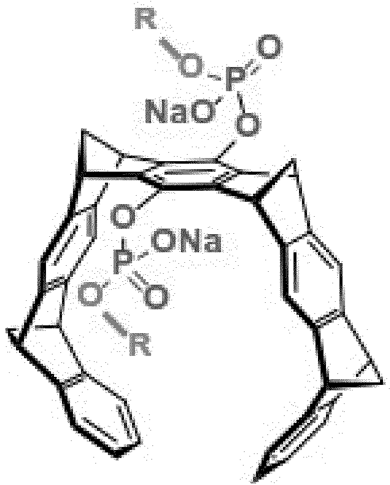 wherein R is CH₃, CH₃CH₂, (CH₃)₂CH, CH₂C≡CH, CH₃CH₂CH₂CH₂, CH₂CH₂C≡CH, CH₃CH₂CH-CH₃, CH₃(CH₂)₆CH₂, CH₃(CH₂)₁₄CH₂ or CH₃(CH₂)₁₆CH₂.

3. A pharmaceutical composition comprising:
a compound of any one of the preceding claims; and
a pharmaceutically acceptable carrier.

4. Composition according to Claim 3 or the compound of any of the claims 1 or 2 for use in disrupting protein aggregation, comprising contacting a protein or a protein aggregate with a compound of Claim 1 or 2

5. Composition according to Claim 3 or the compound of any of the claims 1 or 2 for use in the treatment of a subject having a spinal cord injury or traumatic brain injury, said use comprising administering to said subject a compound of any of the claims 1 or 2 in an amount sufficient to reduce aggregation and/or cytotoxicity of said amyloidogenic protein.

6. Composition according to Claim 3 or the compound of any of the claims 1 or 2 for use in inhibiting the growth, and/or the proliferation, and/or the infectivity, of an enveloped virus, said use comprising contacting said virus with an effective amount of a compound according to any of the claims 1 to 2.

## Patentansprüche

1. Verbindung der Formel I oder ein pharmazeutisch verträgliches Salz davon: 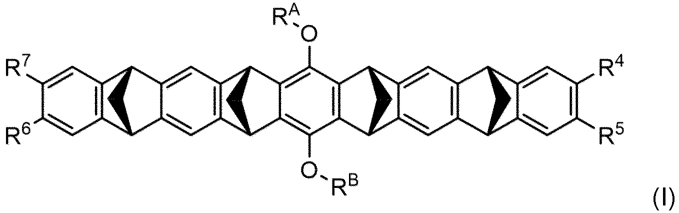 wobei:
R^{A} 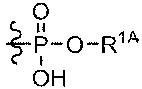 ist, und
R^{B} 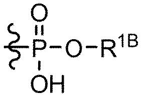 ist, oder
R^{A} 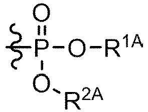 ist, und
R^{1A}, R^{1B}, R^{2A}, R^{2B} ausgewählt sind aus unsubstituiertem oder substituiertem Alkyl, Alkenyl oder Alkinyl, wobei die Substituenten ausgewählt sind aus einem Halogen, Hydroxyl, Carbonyl, Thiocarbonyl, Alkoxy, Phosphoryl, Phosphat, Phosphonat, Phosphinat, Amino, Amido, Amidin, Imin, Cyano, Nitro, Azido, Sulfhydryl, Alkylthio, Sulfat, Sulfonat, Sulfamoyl, Sulfonamido, Sulfonyl, Heterocyclyl, Aralkyl, aromatischen oder heteroaromatischen Resten, die selbst substituiert sein können,
R⁴ und R⁵ unabhängig voneinander ausgewählt sind aus H, Halogen, Alkyl, Alkenyl, Alkinyl, Amino, Amid, Carboxyl, Ester oder Nitro, oder R⁴ und R⁵ zusammen mit den sie trennenden Atomen ein Cycloalkyl, Aryl oder Heteroaryl vervollständigen, und
R⁶ und R⁷ unabhängig voneinander ausgewählt sind aus H, Halogen, Alkyl, Alkenyl, Alkinyl, Amino, Amid, Carboxyl, Ester oder Nitro, oder R⁶ und R⁷ zusammen mit den sie trennenden Atomen ein Cycloalkyl, Aryl oder Heteroaryl vervollständigen.

2. Verbindung nach Anspruch 1, mit der Struktur: 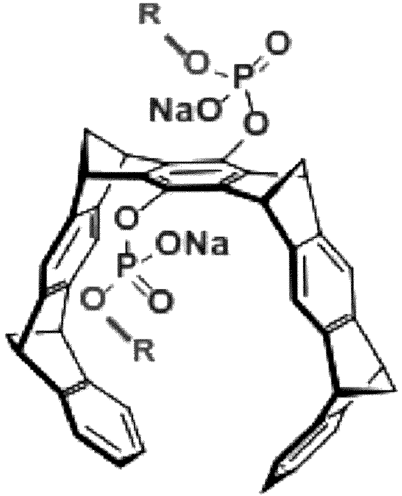 wobei R CH₃, CH₃CH₂, (CH₃)₂CH, CH₂C≡CH, CH₃CH₂CH₂CH₂, CH₂CH₂C≡CH, CH₃CH₂CH-CH₃, CH₃(CH₂)₆CH₂, CH₃(CH₂)₁₄CH₂ oder CH₃(CH₂)₁₆CH₂ ist

3. Pharmazeutische Zusammensetzung, aufweisend:
eine Verbindung nach einem der vorhergehenden Ansprüche; und
einen pharmazeutisch verträglichen Träger.

4. Zusammensetzung nach Anspruch 3 oder Verbindung nach Anspruch 1 oder 2 zur Verwendung bei der Unterbrechung einer Proteinaggregation, aufweisend Inkontaktbringen eines Proteins oder eines Proteinaggregats mit einer Verbindung nach Anspruch 1 oder 2.

5. Zusammensetzung nach Anspruch 3 oder Verbindung nach Anspruch 1 oder 2 zur Verwendung bei der Behandlung eines Patienten mit einer Rückenmarksverletzung oder einer traumatischen Hirnverletzung, wobei die Verwendung das Verabreichen einer Verbindung nach Anspruch 1 oder 2 in einer Menge an den Patienten aufweist, die ausreicht, um die Aggregation und/oder Zytotoxizität des amyloidogenen Proteins zu vermindern.

6. Zusammensetzung nach Anspruch 3 oder Verbindung nach Anspruch 1 oder 2 zur Verwendung zum Hemmen des Wachstums und/oder der Vermehrung und/oder der Infektiosität eines umhüllten Virus, wobei die Verwendung das Inkontaktbringen des Virus mit einer wirksamen Menge einer Verbindung nach Anspruch 1 oder 2 aufweist.

## Revendications

1. Composé de formule I ou sel pharmaceutiquement acceptable de celui-ci : 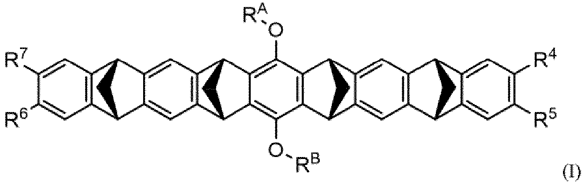 dans lequel :
R^{A} est 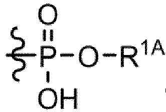 et ;
R^{B} est 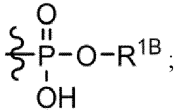 ou
R^{A} est 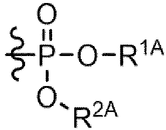 et
R^{B} est 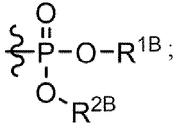 et
R^{1A}, R^{1B}, R^{2A}, R^{2B} sont sélectionnés parmi un alkyle, alcényle, ou alcynyle, substitué ou non substitué, dont les substituants sont sélectionnés parmi un halogène, hydroxyle, carbonyle, thiocarbonyle, alcoxy, phosphoryle, phosphate, phosphonate, phosphinate, amino, amido, amidine, imine, cyano, nitro, azido, sulfhydryle, alkylthio, sulfate, sulfonate, sulfamoyle, sulfonamido, sufonyle, hétérocyclyle, aralkyle, fragments aromatiques ou hétéroaromatiques pouvant être eux-mêmes substitués ;
R⁴ et R⁵ sont indépendamment sélectionnés parmi un hydrogène, halogène, alkyle, alcényle, alcynyle, amino, amide, carboxyle, ester ou nitro ; ou R⁴ et R⁵, conjointement avec les atomes qui les séparent, complètent un cycloalkyle, aryle, ou hétéroaryle ;
R⁶ et R⁷ sont indépendamment sélectionnés parmi un hydrogène, halogène, alkyle, alcényle, alcynyle, amino, amide, carboxyle, ester ou nitro ; ou R⁶ et R⁷, conjointement avec les atomes qui les séparent, complètent un cycloalkyle, aryle, ou hétéroaryle.

2. Composé selon la revendication 1, ayant la structure : 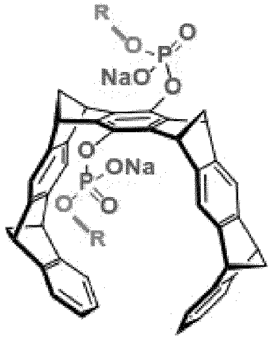 dans lequel R est CH₃, CH₃CH₂, (CH₃)₂CH, CH₂C≡CH, CH₃CH₂CH₂CH₂, CH₂CH₂C≡CH, CH₃CH₂CH-CH₃, CH₃(CH₂)₆CH₂, CH₃(CH₂)₁₄CH₂ ou CH₃(CH₂)₁₆CH₂.

3. Composition pharmaceutique comprenant :
un composé selon l'une quelconque des revendications précédentes ; et
un vecteur pharmaceutiquement acceptable.

4. Composition selon la revendication 3 ou composé selon l'une quelconque des revendications 1 ou 2, destiné à être utilisé pour perturber l'agrégation protéique, comprenant la mise en contact d'une protéine ou d'un agrégat protéique avec un composé selon la revendication 1 ou la revendication 2.

5. Composition selon la revendication 3 ou composé selon l'une quelconque des revendications 1 ou 2, pour une utilisation dans le traitement d'un sujet souffrant d'une lésion de la moelle épinière ou d'un traumatisme crânien, ladite utilisation comprenant l'administration audit sujet d'un composé selon l'une quelconque des revendications 1 ou 2, en quantité suffisante pour réduire l'agrégation et/ou la cytotoxicité de ladite protéine amyloïdogène.

6. Composition selon la revendication 3 ou composé selon l'une quelconque des revendications 1 ou 2, pour une utilisation dans l'inhibition de la croissance, et/ou de la prolifération, et/ou de l'infectiosité d'un virus à enveloppe, ladite utilisation comprenant la mise en contact dudit virus avec une quantité efficace d'un composé selon l'une quelconque des revendications 1 à 2.
